# EUROPEAN PATENT APPLICATION

(11) **EP 3 546 946 A1**
(43) Date of publication of application: **02.10.2019**
(21) Application number: 18164958.3
(22) Date of filing: 29.03.2018
(51) Int. Cl.: G01N 33/68

(54) **METHOD OF DIAGNOSING HEART MUSCLE DAMAGE**

(71) Applicant: Lange, Rüdiger, 81925 München (DE); Krane, Markus, 80469 München (DE)
(72) Inventor: Lange, Rüdiger, 81925 München (DE); Krane, Markus, 80469 München (DE)
(74) Representative: Viering, Jentschura & Partner mbB Patent- und Rechtsanwälte

(57) **Abstract**

The present invention relates to methods of diagnosing heart muscle tissue damage, determining the extent of heart muscle tissue damage and differentiating between atrial heart muscle damage and ventricular heart muscle damage in a patient. Furthermore, the invention relates to a kit and a marker panel for use in these methods.

SEQUENCE LISTING

<110>  Lange, Rüdiger; Krane, Markus; Dreßen, Martina; Lahm, Harald;
Doppler, Stefanie

<120>  Method of diagnosing heart muscle damage

<130>  P69450

<160>  70

<170>  PatentIn version 3.5

<210>  1
<211>  1372
<212>  DNA
<213>  Homo sapiens

<400>  1
accagcagtg aaaatctaga gctggagctc agcctctggg aaggcccagc atggaggcag      60

ccacagctcc ggaggtggcc gcaggatcca agctgaaggt gaaagaagcc agcccagcgg     120

atgctgaacc accccaggct tcacctggac agggggctgg cagccccact ccccagctcc     180

tgccccctat agaagagcac cccaagatct ggctacctcg ggccctgagg cagacctaca     240

tccggaaggt tggggacaca gtgaacctac taatcccatt ccagggcaag cccaaacctc     300

aagccatctg gacacatgat ggctgtgcct tggacaccag gcgtgtgagt gtgcggaatg     360

gggagcaaga ctccatcctc ttcatccgag aagcccaacg tgctgactca ggtcgctacc     420

aactccgcgt gcagctgggt gggctggagg ccaccgccac cattgacatc ctggtgattg     480

agaggccagg ccctcctcag agtattaagc tggtggacgt ttggggcttc agcgctacac     540

tggaatggac accgccccaa gatacgggga atacagcact tctgggatac acggtgcaga     600

aggctgacac aaaatccggg ctgtggttca cggtgctgga gcactatcac cgcaccagct     660

gcatcgtctc tgacctcatc atcggcaact cctatgcctt ccgtgtcttt gctgaaaacc     720

agtgcggact cagtgaaaca gcccccatca ctacggacct cgcccacatc cagaaagcag     780

ctactgttta caagaccaag gggtttgccc aacgagactt ctctgaagcc ccaaagttta     840

cccagcctct ggccgactgc actacagtca ccggctataa tacccagctc ttctgctgtg     900

tccgcgcctc tccccggccc aagatcatct ggctgaagaa caagatggat atccaaggca     960

accctaagta cagagccctg actcacctgg gaatctgctc cctagagatc cgcaagccgg    1020

gtccctttga tggaggcatc tatacctgca aggcggtgaa ccccctaggg gaggcatctg    1080

tggactgtcg ggtggatgtg aaagttccta attgaggaca cctaagaagg catggtactc    1140

tgacaaagcc agtgcccagc aatgaagtga attcatggca caggagccag ggaccgtgac    1200

aacggtgccc tcagcagcct ggtgtcctct ggccatttgc tgtcatggct acaaagtgct    1260

ccctcttttc agtgcctcca ggaagcccgc cagtggtgag actgtgtcct caaggagctt    1320

cggcaccatg tctggtttgg attcatctaa ataaatatgt ggttccccag tg 1372

<210> 2
<211> 1065
<212> DNA
<213> Homo sapiens

<400> 2
atggaggcag ccacagctcc ggaggtggcc gcaggatcca agctgaaggt gaaagaagcc 60

agcccagcgg atgctgaacc accccaggct tcacctggac agggggctgg cagccccact 120

ccccagctcc tgccccctat agaagagcac cccaagatct ggctacctcg ggccctgagg 180

cagacctaca tccggaaggt tggggacaca gtgaacctac taatcccatt ccagggcaag 240

cccaaacctc aagccatctg gacacatgat ggctgtgcct tggacaccag gcgtgtgagt 300

gtgcggaatg gggagcaaga ctccatcctc ttcatccgag aagcccaacg tgctgactca 360

ggtcgctacc aactccgcgt gcagctgggt gggctggagg ccaccgccac cattgacatc 420

ctggtgattg agaggccagg ccctcctcag agtattaagc tggtggacgt ttggggcttc 480

agcgctacac tggaatggac accgccccaa gatacgggga atacagcact tctgggatac 540

acggtgcaga aggctgacac aaaatccggg ctgtggttca cggtgctgga gcactatcac 600

cgcaccagct gcatcgtctc tgacctcatc atcggcaact cctatgcctt ccgtgtcttt 660

gctgaaaacc agtgcggact cagtgaaaca gcccccatca ctacggacct cgcccacatc 720

cagaaagcag ctactgttta caagaccaag gggtttgccc aacgagactt ctctgaagcc 780

ccaaagttta cccagcctct ggccgactgc actacagtca ccggctataa tacccagctc 840

ttctgctgtg tccgcgcctc tccccggccc aagatcatct ggctgaagaa caagatggat 900

atccaaggca accctaagta cagagccctg actcacctgg gaatctgctc cctagagatc 960

cgcaagccgg gtccctttga tggaggcatc tatacctgca aggcggtgaa ccccctaggg 1020

gaggcatctg tggactgtcg ggtggatgtg aaagttccta attga 1065

<210> 3
<211> 354
<212> PRT
<213> Homo sapiens

<400> 3

Met Glu Ala Ala Thr Ala Pro Glu Val Ala Ala Gly Ser Lys Leu Lys
1               5                   10                  15

Val Lys Glu Ala Ser Pro Ala Asp Ala Glu Pro Pro Gln Ala Ser Pro
            20                  25                  30

Gly Gln Gly Ala Gly Ser Pro Thr Pro Gln Leu Leu Pro Pro Ile Glu
        35                  40                  45

Glu His Pro Lys Ile Trp Leu Pro Arg Ala Leu Arg Gln Thr Tyr Ile
    50                  55                  60

Arg Lys Val Gly Asp Thr Val Asn Leu Leu Ile Pro Phe Gln Gly Lys
65                  70                  75                  80

Pro Lys Pro Gln Ala Ile Trp Thr His Asp Gly Cys Ala Leu Asp Thr
                85                  90                  95

Arg Arg Val Ser Val Arg Asn Gly Glu Gln Asp Ser Ile Leu Phe Ile
            100                 105                 110

Arg Glu Ala Gln Arg Ala Asp Ser Gly Arg Tyr Gln Leu Arg Val Gln
        115                 120                 125

Leu Gly Gly Leu Glu Ala Thr Ala Thr Ile Asp Ile Leu Val Ile Glu
    130                 135                 140

Arg Pro Gly Pro Pro Gln Ser Ile Lys Leu Val Asp Val Trp Gly Phe
145                 150                 155                 160

Ser Ala Thr Leu Glu Trp Thr Pro Pro Gln Asp Thr Gly Asn Thr Ala
                165                 170                 175

Leu Leu Gly Tyr Thr Val Gln Lys Ala Asp Thr Lys Ser Gly Leu Trp
            180                 185                 190

Phe Thr Val Leu Glu His Tyr His Arg Thr Ser Cys Ile Val Ser Asp
        195                 200                 205

Leu Ile Ile Gly Asn Ser Tyr Ala Phe Arg Val Phe Ala Glu Asn Gln
    210                 215                 220

Cys Gly Leu Ser Glu Thr Ala Pro Ile Thr Thr Asp Leu Ala His Ile
225                 230                 235                 240

Gln Lys Ala Ala Thr Val Tyr Lys Thr Lys Gly Phe Ala Gln Arg Asp
                245                 250                 255

Phe Ser Glu Ala Pro Lys Phe Thr Gln Pro Leu Ala Asp Cys Thr Thr
            260                 265                 270

Val Thr Gly Tyr Asn Thr Gln Leu Phe Cys Cys Val Arg Ala Ser Pro
        275                 280                 285

Arg Pro Lys Ile Ile Trp Leu Lys Asn Lys Met Asp Ile Gln Gly Asn

290                 295                 300

Pro Lys Tyr Arg Ala Leu Thr His Leu Gly Ile Cys Ser Leu Glu Ile
305                 310                 315                 320

Arg Lys Pro Gly Pro Phe Asp Gly Gly Ile Tyr Thr Cys Lys Ala Val
                325                 330                 335

Asn Pro Leu Gly Glu Ala Ser Val Asp Cys Arg Val Asp Val Lys Val
            340                 345                 350

Pro Asn

<210>  4
<211>  1303
<212>  DNA
<213>  Homo sapiens

<400>  4
accagcagtg aaaatctaga gctggagctc agcctctggg aaggcccagc atggaggcag      60

ccacagctcc ggaggtggcc gcaggatcca agctgaaggt gaaagaagcc agcccagcgg     120

atgctgaacc accccaggct tcacctggac agggggctgg cagccccact ccccagctcc     180

tgccccctat agaagagcac cccaagatct ggctacctcg ggccctgagg cagacctaca     240

tccggaaggt tggggacaca gtgaacctac taatcccatt ccagggcaag cccaaacctc     300

aagccatctg gacacatgat ggctgtgcct tggacaccag gcgtgtgagt gtgcggaatg     360

gggagcaaga ctccatcctc ttcatccgag aagcccaacg tgctgactca gagaggccag     420

gccctcctca gagtattaag ctggtggacg tttggggctt cagcgctaca ctggaatgga     480

caccgcccca agatacgggg aatacagcac ttctgggata cacggtgcag aaggctgaca     540

caaaatccgg gctgtggttc acggtgctgg agcactatca ccgcaccagc tgcatcgtct     600

ctgacctcat catcggcaac tcctatgcct tccgtgtctt tgctgaaaac cagtgcggac     660

tcagtgaaac agcccccatc actacggacc tcgcccacat ccagaaagca gctactgttt     720

acaagaccaa ggggtttgcc caacgagact tctctgaagc cccaaagttt acccagcctc     780

tggccgactg cactacagtc accggctata atacccagct cttctgctgt gtccgcgcct     840

ctccccggcc caagatcatc tggctgaaga acaagatgga tatccaaggc aaccctaagt     900

acagagccct gactcacctg ggaatctgct ccctagagat ccgcaagccg ggtccctttg     960

atggaggcat ctatacctgc aaggcggtga acccctaggg ggaggcatct gtggactgtc    1020

gggtggatgt gaaagttcct aattgaggac acctaagaag gcatggtact ctgacaaagc    1080

cagtgcccag caatgaagtg aattcatggc acaggagcca gggaccgtga caacggtgcc    1140

ctcagcagcc tggtgtcctc tggccatttg ctgtcatggc tacaaagtgc tccctctttt 1200

cagtgcctcc aggaagcccg ccagtggtga gactgtgtcc tcaaggagct tcggcaccat 1260

gtctggtttg gattcatcta aataaatatg tggttcccca gtg 1303

<210> 5
<211> 996
<212> DNA
<213> Homo sapiens

<400> 5
atggaggcag ccacagctcc ggaggtggcc gcaggatcca agctgaaggt gaaagaagcc 60

agcccagcgg atgctgaacc accccaggct tcacctggac agggggctgg cagccccact 120

ccccagctcc tgccccctat agaagagcac cccaagatct ggctacctcg ggccctgagg 180

cagacctaca tccggaaggt tggggacaca gtgaacctac taatcccatt ccagggcaag 240

cccaaacctc aagccatctg gacacatgat ggctgtgcct tggacaccag gcgtgtgagt 300

gtgcggaatg gggagcaaga ctccatcctc ttcatccgag aagcccaacg tgctgactca 360

gagaggccag gccctcctca gagtattaag ctggtggacg tttggggctt cagcgctaca 420

ctggaatgga caccgcccca agatacgggg aatacagcac ttctgggata cacggtgcag 480

aaggctgaca caaaatccgg gctgtggttc acggtgctgg agcactatca ccgcaccagc 540

tgcatcgtct ctgacctcat catcggcaac tcctatgcct tccgtgtctt tgctgaaaac 600

cagtgcggac tcagtgaaac agcccccatc actacggacc tcgcccacat ccagaaagca 660

gctactgttt acaagaccaa ggggtttgcc caacgagact tctctgaagc cccaaagttt 720

acccagcctc tggccgactg cactacagtc accggctata atacccagct cttctgctgt 780

gtccgcgcct ctccccggcc caagatcatc tggctgaaga acaagatgga tatccaaggc 840

aaccctaagt acagagccct gactcacctg ggaatctgct ccctagagat ccgcaagccg 900

ggtccctttg atggaggcat ctatacctgc aaggcggtga accccctagg ggaggcatct 960

gtggactgtc gggtggatgt gaaagttcct aattga 996

<210> 6
<211> 331
<212> PRT
<213> Homo sapiens

<400> 6

Met Glu Ala Ala Thr Ala Pro Glu Val Ala Ala Gly Ser Lys Leu Lys
1               5                   10                  15

Val Lys Glu Ala Ser Pro Ala Asp Ala Glu Pro Pro Gln Ala Ser Pro
            20                  25                  30

Gly Gln Gly Ala Gly Ser Pro Thr Pro Gln Leu Leu Pro Pro Ile Glu
        35                  40                  45

Glu His Pro Lys Ile Trp Leu Pro Arg Ala Leu Arg Gln Thr Tyr Ile
    50                  55                  60

Arg Lys Val Gly Asp Thr Val Asn Leu Leu Ile Pro Phe Gln Gly Lys
65                  70                  75                  80

Pro Lys Pro Gln Ala Ile Trp Thr His Asp Gly Cys Ala Leu Asp Thr
                85                  90                  95

Arg Arg Val Ser Val Arg Asn Gly Glu Gln Asp Ser Ile Leu Phe Ile
            100                 105                 110

Arg Glu Ala Gln Arg Ala Asp Ser Glu Arg Pro Gly Pro Pro Gln Ser
        115                 120                 125

Ile Lys Leu Val Asp Val Trp Gly Phe Ser Ala Thr Leu Glu Trp Thr
    130                 135                 140

Pro Pro Gln Asp Thr Gly Asn Thr Ala Leu Leu Gly Tyr Thr Val Gln
145                 150                 155                 160

Lys Ala Asp Thr Lys Ser Gly Leu Trp Phe Thr Val Leu Glu His Tyr
                165                 170                 175

His Arg Thr Ser Cys Ile Val Ser Asp Leu Ile Ile Gly Asn Ser Tyr
            180                 185                 190

Ala Phe Arg Val Phe Ala Glu Asn Gln Cys Gly Leu Ser Glu Thr Ala
        195                 200                 205

Pro Ile Thr Thr Asp Leu Ala His Ile Gln Lys Ala Ala Thr Val Tyr
    210                 215                 220

Lys Thr Lys Gly Phe Ala Gln Arg Asp Phe Ser Glu Ala Pro Lys Phe
225                 230                 235                 240

Thr Gln Pro Leu Ala Asp Cys Thr Thr Val Thr Gly Tyr Asn Thr Gln
                245                 250                 255

Leu Phe Cys Cys Val Arg Ala Ser Pro Arg Pro Lys Ile Ile Trp Leu
            260                 265                 270

Lys Asn Lys Met Asp Ile Gln Gly Asn Pro Lys Tyr Arg Ala Leu Thr
        275                 280                 285

His Leu Gly Ile Cys Ser Leu Glu Ile Arg Lys Pro Gly Pro Phe Asp
    290                 295                 300

Gly Gly Ile Tyr Thr Cys Lys Ala Val Asn Pro Leu Gly Glu Ala Ser
305                 310                 315                 320

Val Asp Cys Arg Val Asp Val Lys Val Pro Asn
                325                 330

<210>  7
<211>  477
<212>  PRT
<213>  Homo sapiens

<400>  7

Met Met Glu Lys Asn Thr Ser Glu Gly Pro Ala Cys Ser Pro Glu Glu
1               5                   10                  15

Thr Ala Ser Glu Ser Ala Lys Val Pro Thr Ala Glu Pro Pro Gly Glu
            20                  25                  30

Val Ala Val Ser Glu Ser Thr Arg Glu Glu Gln Val Pro Lys Pro Gln
        35                  40                  45

Ala Pro Ala Pro Gln Ala Pro Thr Ala Ser Thr Ala Thr Lys Pro Ala
    50                  55                  60

Pro Pro Ser Glu Asp Val Pro Ser Ala Pro Leu Leu Leu Thr Leu Asp
65                  70                  75                  80

Asp Val Ser Ser Ser Ser Val Thr Val Ser Trp Glu Pro Pro Glu Arg
                85                  90                  95

Leu Gly Arg Leu Gly Leu Gln Gly Tyr Val Leu Glu Leu Cys Arg Glu
            100                 105                 110

Gly Ala Ser Glu Trp Val Pro Val Ser Ala Arg Pro Met Met Val Thr
        115                 120                 125

Gln Gln Thr Val Arg Asn Leu Ala Leu Gly Asp Lys Phe Leu Leu Arg
    130                 135                 140

Val Ser Ala Val Ser Ser Ala Gly Ala Gly Pro Pro Ala Met Leu Asp
145                 150                 155                 160

Gln Pro Ile His Ile Arg Glu Asn Ile Glu Ala Pro Lys Ile Arg Val
                165                 170                 175

Pro Arg His Leu Arg Gln Thr Tyr Ile Arg Gln Val Gly Glu Thr Val
            180                 185                 190

Asn Leu Gln Ile Pro Phe Gln Gly Lys Pro Lys Pro Gln Ala Thr Trp
        195                 200                 205

Thr His Asn Gly His Ala Leu Asp Ser Gln Arg Val Ser Met Arg Thr
    210                 215                 220

Gly Asp Gln Asp Ser Ile Leu Phe Ile Arg Ser Ala Gln Arg Ser Asp
225                 230                 235                 240

Ser Gly Arg Tyr Glu Leu Thr Val Arg Val Glu Asp Leu Glu Ala Lys
                245                 250                 255

Ala Val Ile Asp Ile Leu Val Ile Glu Lys Pro Gly Pro Pro Ser Ser
            260                 265                 270

Ile Arg Leu Leu Asp Val Trp Gly Cys Asn Ala Ala Leu Gln Trp Thr
        275                 280                 285

Pro Pro Gln Asp Thr Gly Asn Thr Glu Leu Leu Gly Tyr Met Val Gln
    290                 295                 300

Lys Ala Asp Lys Lys Thr Gly Gln Trp Phe Thr Val Leu Glu Arg Tyr
305                 310                 315                 320

His Pro Thr Thr Cys Thr Ile Ser Asp Leu Ile Ile Gly Asn Ser Tyr
                325                 330                 335

Ser Phe Arg Val Phe Ser Glu Asn Leu Cys Gly Leu Ser Thr Ser Ala
            340                 345                 350

Thr Val Thr Lys Glu Leu Ala His Ile Gln Lys Ala Asp Ile Ala Ala
        355                 360                 365

Lys Pro Lys Gly Phe Ile Glu Arg Asp Phe Ser Glu Ala Pro Ser Phe
    370                 375                 380

Thr Gln Pro Leu Ala Asp His Thr Ser Thr Pro Gly Tyr Ser Thr Gln
385                 390                 395                 400

Leu Phe Cys Ser Val Arg Ala Ser Pro Lys Pro Lys Ile Ile Trp Met
                405                 410                 415

Lys Asn Lys Met Glu Ile Gln Gly Asn Pro Lys Tyr Arg Ala Leu Ser
            420                 425                 430

Glu Gln Gly Val Cys Thr Leu Glu Ile Arg Lys Pro Ser Pro Phe Asp
        435                 440                 445

Ser Gly Val Tyr Thr Cys Lys Ala Ile Asn Val Leu Gly Glu Ala Ser
    450                 455                 460

Val Asp Cys Arg Leu Glu Val Lys Ala Ser Ala Ala His
465                 470                 475

<210>  8
<211>  1877
<212>  PRT
<213>  Homo sapiens

<400>  8

Met Leu Gly Ser Ser Val Lys Ser Val Gln Pro Glu Val Glu Leu Ser
1               5                   10                  15

Ser Gly Gly Gly Asp Glu Gly Ala Asp Glu Pro Arg Gly Ala Gly Arg
            20                  25                  30

Lys Ala Ala Ala Ala Asp Gly Arg Gly Met Leu Pro Lys Arg Ala Lys
        35                  40                  45

Ala Pro Gly Gly Gly Gly Gly Met Ala Lys Ala Ser Ala Ala Glu Leu
    50                  55                  60

Lys Val Phe Lys Ser Gly Ser Val Asp Ser Arg Val Pro Gly Gly Pro
65                  70                  75                  80

Pro Ala Ser Asn Leu Arg Lys Gln Lys Ser Leu Thr Asn Leu Ser Phe
                85                  90                  95

Leu Thr Asp Ser Glu Lys Lys Leu Gln Leu Tyr Glu Pro Glu Trp Ser
            100                 105                 110

Asp Asp Met Ala Lys Ala Pro Lys Gly Leu Gly Lys Val Gly Ser Lys
        115                 120                 125

Gly Arg Glu Ala Pro Leu Met Ser Lys Thr Leu Ser Lys Ser Glu His
    130                 135                 140

Ser Leu Phe Gln Ala Lys Gly Ser Pro Ala Gly Gly Ala Lys Thr Pro
145                 150                 155                 160

Leu Ala Pro Leu Ala Pro Asn Leu Gly Lys Pro Ser Arg Ile Pro Arg
                165                 170                 175

Gly Pro Tyr Ala Glu Val Lys Pro Leu Ser Lys Ala Pro Glu Ala Ala
            180                 185                 190

Val Ser Glu Asp Gly Lys Ser Asp Asp Glu Leu Leu Ser Ser Lys Ala
        195                 200                 205

Lys Ala Gln Lys Ser Ser Gly Pro Val Pro Ser Ala Lys Gly Gln Glu
    210                 215                 220

Glu Arg Ala Phe Leu Lys Val Asp Pro Glu Leu Val Val Thr Val Leu
225                 230                 235                 240

Gly Asp Leu Glu Gln Leu Leu Phe Ser Gln Met Leu Asp Pro Glu Ser
                245                 250                 255

Gln Arg Lys Arg Thr Val Gln Asn Val Leu Asp Leu Arg Gln Asn Leu
            260                 265                 270

Glu Glu Thr Met Ser Ser Leu Arg Gly Ser Gln Val Thr His Ser Ser
        275                 280                 285

Leu Glu Met Thr Cys Tyr Asp Ser Asp Asp Ala Asn Pro Arg Ser Val
    290                 295                 300

Ser Ser Leu Ser Asn Arg Ser Ser Pro Leu Ser Trp Arg Tyr Gly Gln
305                 310                 315                 320

Ser Ser Pro Arg Leu Gln Ala Gly Asp Ala Pro Ser Val Gly Gly Ser
                325                 330                 335

Cys Arg Ser Glu Gly Thr Pro Ala Trp Tyr Met His Gly Glu Arg Ala
            340                 345                 350

His Tyr Ser His Thr Met Pro Met Arg Ser Pro Ser Lys Leu Ser His
        355                 360                 365

Ile Ser Arg Leu Glu Leu Val Glu Ser Leu Asp Ser Asp Glu Val Asp
    370                 375                 380

Leu Lys Ser Gly Tyr Met Ser Asp Ser Asp Leu Met Gly Lys Thr Met
385                 390                 395                 400

Thr Glu Asp Asp Asp Ile Thr Thr Gly Trp Asp Glu Ser Ser Ser Ile
                405                 410                 415

Ser Ser Gly Leu Ser Asp Ala Ser Asp Asn Leu Ser Ser Glu Glu Phe

            420                 425                 430

Asn Ala Ser Ser Ser Leu Asn Ser Leu Pro Ser Thr Pro Thr Ala Ser
        435                 440                 445

Arg Arg Asn Ser Thr Ile Val Leu Arg Thr Asp Ser Glu Lys Arg Ser
    450                 455                 460

Leu Ala Glu Ser Gly Leu Ser Trp Phe Ser Glu Ser Glu Glu Lys Ala
465                 470                 475                 480

Pro Lys Lys Leu Glu Tyr Asp Ser Gly Ser Leu Lys Met Glu Pro Gly
                485                 490                 495

Thr Ser Lys Trp Arg Arg Glu Arg Pro Glu Ser Cys Asp Asp Ser Ser
            500                 505                 510

Lys Gly Gly Glu Leu Lys Lys Pro Ile Ser Leu Gly His Pro Gly Ser
        515                 520                 525

Leu Lys Lys Gly Lys Thr Pro Pro Val Ala Val Thr Ser Pro Ile Thr
    530                 535                 540

His Thr Ala Gln Ser Ala Leu Lys Val Ala Gly Lys Pro Glu Gly Lys
545                 550                 555                 560

Ala Thr Asp Lys Gly Lys Leu Ala Val Lys Asn Thr Gly Leu Gln Arg
                565                 570                 575

Ser Ser Ser Asp Ala Gly Arg Asp Arg Leu Ser Asp Ala Lys Lys Pro
            580                 585                 590

Pro Ser Gly Ile Ala Arg Pro Ser Thr Ser Gly Ser Phe Gly Tyr Lys
        595                 600                 605

Lys Pro Pro Pro Ala Thr Gly Thr Ala Thr Val Met Gln Thr Gly Gly
    610                 615                 620

Ser Ala Thr Leu Ser Lys Ile Gln Lys Ser Ser Gly Ile Pro Val Lys
625                 630                 635                 640

Pro Val Asn Gly Arg Lys Thr Ser Leu Asp Val Ser Asn Ser Ala Glu
                645                 650                 655

Pro Gly Phe Leu Ala Pro Gly Ala Arg Ser Asn Ile Gln Tyr Arg Ser
            660                 665                 670

Leu Pro Arg Pro Ala Lys Ser Ser Ser Met Ser Val Thr Gly Gly Arg
        675                 680                 685

Gly Gly Pro Arg Pro Val Ser Ser Ser Ile Asp Pro Ser Leu Leu Ser
    690                 695                 700

Thr Lys Gln Gly Gly Leu Thr Pro Ser Arg Leu Lys Glu Pro Thr Lys
705                 710                 715                 720

Val Ala Ser Gly Arg Thr Thr Pro Ala Pro Val Asn Gln Thr Asp Arg
                725                 730                 735

Glu Lys Glu Lys Ala Lys Ala Lys Ala Val Ala Leu Asp Ser Asp Asn
            740                 745                 750

Ile Ser Leu Lys Ser Ile Gly Ser Pro Glu Ser Thr Pro Lys Asn Gln
        755                 760                 765

Ala Ser His Pro Thr Ala Thr Lys Leu Ala Glu Leu Pro Pro Thr Pro
    770                 775                 780

Leu Arg Ala Thr Ala Lys Ser Phe Val Lys Pro Pro Ser Leu Ala Asn
785                 790                 795                 800

Leu Asp Lys Val Asn Ser Asn Ser Leu Asp Leu Pro Ser Ser Ser Asp
                805                 810                 815

Thr Thr His Ala Ser Lys Val Pro Asp Leu His Ala Thr Ser Ser Ala
            820                 825                 830

Ser Gly Gly Pro Leu Pro Ser Cys Phe Thr Pro Ser Pro Ala Pro Ile
        835                 840                 845

Leu Asn Ile Asn Ser Ala Ser Phe Ser Gln Gly Leu Glu Leu Met Ser
    850                 855                 860

Gly Phe Ser Val Pro Lys Glu Thr Arg Met Tyr Pro Lys Leu Ser Gly
865                 870                 875                 880

Leu His Arg Ser Met Glu Ser Leu Gln Met Pro Met Ser Leu Pro Ser
                885                 890                 895

Ala Phe Pro Ser Ser Thr Pro Val Pro Thr Pro Pro Ala Pro Pro Ala
            900                 905                 910

Ala Pro Thr Glu Glu Glu Thr Glu Glu Leu Thr Trp Ser Gly Ser Pro
        915                 920                 925

Arg Ala Gly Gln Leu Asp Ser Asn Gln Arg Asp Arg Asn Thr Leu Pro
    930                 935                 940

Lys Lys Gly Leu Arg Tyr Gln Leu Gln Ser Gln Glu Glu Thr Lys Glu
945                 950                 955                 960

Arg Arg His Ser His Thr Ile Gly Gly Leu Pro Glu Ser Asp Asp Gln
                965                 970                 975

Ser Glu Leu Pro Ser Pro Pro Ala Leu Pro Met Ser Leu Ser Ala Lys
            980                 985                 990

Gly Gln Leu Thr Asn Ile Val Ser  Pro Thr Ala Ala Thr  Thr Pro Arg
        995                 1000                 1005

Ile Thr  Arg Ser Asn Ser Ile  Pro Thr His Glu Ala  Ala Phe Glu
    1010                 1015                 1020

Leu Tyr  Ser Gly Ser Gln Met  Gly Ser Thr Leu Ser  Leu Ala Glu
    1025                 1030                 1035

Arg Pro  Lys Gly Met Ile Arg  Ser Gly Ser Phe Arg  Asp Pro Thr
    1040                 1045                 1050

Asp Asp  Val His Gly Ser Val  Leu Ser Leu Ala Ser  Ser Ala Ser
    1055                 1060                 1065

Ser Thr  Tyr Ser Ser Ala Glu  Glu Arg Met Gln Ser  Glu Gln Ile
    1070                 1075                 1080

Arg Lys  Leu Arg Arg Glu Leu  Glu Ser Ser Gln Glu  Lys Val Ala
    1085                 1090                 1095

Thr Leu  Thr Ser Gln Leu Ser  Ala Asn Ala Asn Leu  Val Ala Ala
    1100                 1105                 1110

Phe Glu  Gln Ser Leu Val Asn  Met Thr Ser Arg Leu  Arg His Leu
    1115                 1120                 1125

Ala Glu  Thr Ala Glu Glu Lys  Asp Thr Glu Leu Leu  Asp Leu Arg
    1130                 1135                 1140

Glu Thr  Ile Asp Phe Leu Lys  Lys Lys Asn Ser Glu  Ala Gln Ala
    1145                 1150                 1155

Val Ile  Gln Gly Ala Leu Asn  Ala Ser Glu Thr Thr  Pro Lys Glu
    1160                 1165                 1170

Leu Arg Ile Lys Arg Gln Asn Ser Ser Asp Ser Ile Ser Ser Leu
    1175                1180                1185

Asn Ser Ile Thr Ser His Ser Ser Ile Gly Ser Ser Lys Asp Ala
    1190                1195                1200

Asp Ala Lys Lys Lys Lys Lys Lys Ser Trp Val Tyr Glu Leu Arg
    1205                1210                1215

Ser Ser Phe Asn Lys Ala Phe Ser Ile Lys Lys Gly Pro Lys Ser
    1220                1225                1230

Ala Ser Ser Tyr Ser Asp Ile Glu Glu Ile Ala Thr Pro Asp Ser
    1235                1240                1245

Ser Ala Pro Ser Ser Pro Lys Leu Gln His Gly Ser Thr Glu Thr
    1250                1255                1260

Ala Ser Pro Ser Ile Lys Ser Ser Thr Ser Ser Ser Val Gly Thr
    1265                1270                1275

Asp Val Thr Glu Gly Pro Ala His Pro Ala Pro His Thr Arg Leu
    1280                1285                1290

Phe His Ala Asn Glu Glu Glu Glu Pro Glu Lys Lys Glu Val Ser
    1295                1300                1305

Glu Leu Arg Ser Glu Leu Trp Glu Lys Glu Met Lys Leu Thr Asp
    1310                1315                1320

Ile Arg Leu Glu Ala Leu Asn Ser Ala His Gln Leu Asp Gln Leu
    1325                1330                1335

Arg Glu Thr Met His Asn Met Gln Leu Glu Val Asp Leu Leu Lys
    1340                1345                1350

Ala Glu Asn Asp Arg Leu Lys Val Ala Pro Gly Pro Ser Ser Gly
    1355                1360                1365

Ser Thr Pro Gly Gln Val Pro Gly Ser Ser Ala Leu Ser Ser Pro
    1370                1375                1380

Arg Arg Ser Leu Gly Leu Ala Leu Thr His Ser Phe Gly Pro Ser
    1385                1390                1395

Leu Ala Asp Thr Asp Leu Ser Pro Met Asp Gly Ile Ser Thr Cys

1400                 1405                 1410

Gly Pro Lys Glu Glu Val Thr Leu Arg Val Val Val Arg Met Pro
    1415                 1420                 1425

Pro Gln His Ile Ile Lys Gly Asp Leu Lys Gln Gln Glu Phe Phe
    1430                 1435                 1440

Leu Gly Cys Ser Lys Val Ser Gly Lys Val Asp Trp Lys Met Leu
    1445                 1450                 1455

Asp Glu Ala Val Phe Gln Val Phe Lys Asp Tyr Ile Ser Lys Met
    1460                 1465                 1470

Asp Pro Ala Ser Thr Leu Gly Leu Ser Thr Glu Ser Ile His Gly
    1475                 1480                 1485

Tyr Ser Ile Ser His Val Lys Arg Val Leu Asp Ala Glu Pro Pro
    1490                 1495                 1500

Glu Met Pro Pro Cys Arg Arg Gly Val Asn Asn Ile Ser Val Ser
    1505                 1510                 1515

Leu Lys Gly Leu Lys Glu Lys Cys Val Asp Ser Leu Val Phe Glu
    1520                 1525                 1530

Thr Leu Ile Pro Lys Pro Met Met Gln His Tyr Ile Ser Leu Leu
    1535                 1540                 1545

Leu Lys His Arg Arg Leu Val Leu Ser Gly Pro Ser Gly Thr Gly
    1550                 1555                 1560

Lys Thr Tyr Leu Thr Asn Arg Leu Ala Glu Tyr Leu Val Glu Arg
    1565                 1570                 1575

Ser Gly Arg Glu Val Thr Glu Gly Ile Val Ser Thr Phe Asn Met
    1580                 1585                 1590

His Gln Gln Ser Cys Lys Asp Leu Gln Leu Tyr Leu Ser Asn Leu
    1595                 1600                 1605

Ala Asn Gln Ile Asp Arg Glu Thr Gly Ile Gly Asp Val Pro Leu
    1610                 1615                 1620

Val Ile Leu Leu Asp Asp Leu Ser Glu Ala Gly Ser Ile Ser Glu
    1625                 1630                 1635

Leu Val Asn Gly Ala Leu Thr Cys Lys Tyr His Lys Cys Pro Tyr
    1640                1645                1650

Ile Ile Gly Thr Thr Asn Gln Pro Val Lys Met Thr Pro Asn His
    1655                1660                1665

Gly Leu His Leu Ser Phe Arg Met Leu Thr Phe Ser Asn Asn Val
    1670                1675                1680

Glu Pro Ala Asn Gly Phe Leu Val Arg Tyr Leu Arg Arg Lys Leu
    1685                1690                1695

Val Glu Ser Asp Ser Asp Ile Asn Ala Asn Lys Glu Glu Leu Leu
    1700                1705                1710

Arg Val Leu Asp Trp Val Pro Lys Leu Trp Tyr His Leu His Thr
    1715                1720                1725

Phe Leu Glu Lys His Ser Thr Ser Asp Phe Leu Ile Gly Pro Cys
    1730                1735                1740

Phe Phe Leu Ser Cys Pro Ile Gly Ile Glu Asp Phe Arg Thr Trp
    1745                1750                1755

Phe Ile Asp Leu Trp Asn Asn Ser Ile Ile Pro Tyr Leu Gln Glu
    1760                1765                1770

Gly Ala Lys Asp Gly Ile Lys Val His Gly Gln Lys Ala Ala Trp
    1775                1780                1785

Glu Asp Pro Val Glu Trp Val Arg Asp Thr Leu Pro Trp Pro Ser
    1790                1795                1800

Ala Gln Gln Asp Gln Ser Lys Leu Tyr His Leu Pro Pro Pro Thr
    1805                1810                1815

Val Gly Pro His Ser Ile Ala Ser Pro Pro Glu Asp Arg Thr Val
    1820                1825                1830

Lys Asp Ser Thr Pro Ser Ser Leu Asp Ser Asp Pro Leu Met Ala
    1835                1840                1845

Met Leu Leu Lys Leu Gln Glu Ala Ala Asn Tyr Ile Glu Ser Pro
    1850                1855                1860

Asp Arg Glu Thr Ile Leu Asp Pro Asn Leu Gln Ala Thr Leu
    1865                1870                1875

<210>  9
<211>  498
<212>  PRT
<213>  Homo sapiens

<400>  9

Met Phe Asn Tyr Glu Arg Pro Lys His Phe Ile Gln Ser Gln Asn Pro
1               5                   10                  15

Cys Gly Ser Arg Leu Gln Pro Pro Gly Pro Glu Thr Ser Ser Phe Ser
            20                  25                  30

Ser Gln Thr Lys Gln Ser Ser Ile Ile Ile Gln Pro Arg Gln Cys Thr
        35                  40                  45

Glu Gln Arg Phe Ser Ala Ser Ser Thr Leu Ser Ser His Ile Thr Met
    50                  55                  60

Ser Ser Ser Ala Phe Pro Ala Ser Pro Lys Gln His Ala Gly Ser Asn
65                  70                  75                  80

Pro Gly Gln Arg Val Thr Thr Thr Tyr Asn Gln Ser Pro Ala Ser Phe
                85                  90                  95

Leu Ser Ser Ile Leu Pro Ser Gln Pro Asp Tyr Asn Ser Ser Lys Ile
            100                 105                 110

Pro Ser Ala Met Asp Ser Asn Tyr Gln Gln Ser Ser Ala Gly Gln Pro
        115                 120                 125

Ile Asn Ala Lys Pro Ser Gln Thr Ala Asn Ala Lys Pro Ile Pro Arg
    130                 135                 140

Thr Pro Asp His Glu Ile Gln Gly Ser Lys Glu Ala Leu Ile Gln Asp
145                 150                 155                 160

Leu Glu Arg Lys Leu Lys Cys Lys Asp Thr Leu Leu His Asn Gly Asn
                165                 170                 175

Gln Arg Leu Thr Tyr Glu Glu Lys Met Ala Arg Arg Leu Leu Gly Pro
            180                 185                 190

Gln Asn Ala Ala Ala Val Phe Gln Ala Gln Asp Asp Ser Gly Ala Gln
        195                 200                 205

Asp Ser Gln Gln His Asn Ser Glu His Ala Arg Leu Gln Val Pro Thr
    210                 215                 220

Ser Gln Val Arg Ser Arg Ser Thr Ser Arg Gly Asp Val Asn Asp Gln
225                 230                 235                 240

Asp Ala Ile Gln Glu Lys Phe Tyr Pro Pro Arg Phe Ile Gln Val Pro
                245                 250                 255

Glu Asn Met Ser Ile Asp Glu Gly Arg Phe Cys Arg Met Asp Phe Lys
            260                 265                 270

Val Ser Gly Leu Pro Ala Pro Asp Val Ser Trp Tyr Leu Asn Gly Arg
        275                 280                 285

Thr Val Gln Ser Asp Asp Leu His Lys Met Ile Val Ser Glu Lys Gly
    290                 295                 300

Leu His Ser Leu Ile Phe Glu Val Val Arg Ala Ser Asp Ala Gly Ala
305                 310                 315                 320

Tyr Ala Cys Val Ala Lys Asn Arg Ala Gly Glu Ala Thr Phe Thr Val
                325                 330                 335

Gln Leu Asp Val Leu Ala Lys Glu His Lys Arg Ala Pro Met Phe Ile
            340                 345                 350

Tyr Lys Pro Gln Ser Lys Lys Val Leu Glu Gly Asp Ser Val Lys Leu
        355                 360                 365

Glu Cys Gln Ile Ser Ala Ile Pro Pro Pro Lys Leu Phe Trp Lys Arg
    370                 375                 380

Asn Asn Glu Met Val Gln Phe Asn Thr Asp Arg Ile Ser Leu Tyr Gln
385                 390                 395                 400

Asp Asn Thr Gly Arg Val Thr Leu Leu Ile Lys Asp Val Asn Lys Lys
                405                 410                 415

Asp Ala Gly Trp Tyr Thr Val Ser Ala Val Asn Glu Ala Gly Val Thr
            420                 425                 430

Thr Cys Asn Thr Arg Leu Asp Val Thr Ala Arg Pro Asn Gln Thr Leu
        435                 440                 445

Pro Ala Pro Lys Gln Leu Arg Val Arg Pro Thr Phe Ser Lys Tyr Leu
    450                 455                 460

Ala Leu Asn Gly Lys Gly Leu Asn Val Lys Gln Ala Phe Asn Pro Glu
465                 470                 475                 480

Gly Glu Phe Gln Arg Leu Ala Ala Gln Ser Gly Leu Tyr Glu Ser Glu
                485                 490                 495

Glu Leu

<210>  10
<211>  643
<212>  PRT
<213>  Homo sapiens

<400>  10

Met Cys Gly Ile Cys Cys Ser Val Asn Phe Ser Ala Glu His Phe Ser
1               5                   10                  15

Gln Asp Leu Lys Glu Asp Leu Leu Tyr Asn Leu Lys Gln Arg Gly Pro
            20                  25                  30

Asn Ser Ser Lys Gln Leu Leu Lys Ser Asp Val Asn Tyr Gln Cys Leu
        35                  40                  45

Phe Ser Ala His Val Leu His Leu Arg Gly Val Leu Thr Thr Gln Pro
    50                  55                  60

Val Glu Asp Glu Arg Gly Asn Val Phe Leu Trp Asn Gly Glu Ile Phe
65                  70                  75                  80

Ser Gly Ile Lys Val Glu Ala Glu Glu Asn Asp Thr Gln Ile Leu Phe
                85                  90                  95

Asn Tyr Leu Ser Ser Cys Lys Asn Glu Ser Glu Ile Leu Ser Leu Phe
            100                 105                 110

Ser Glu Val Gln Gly Pro Trp Ser Phe Ile Tyr Tyr Gln Ala Ser Ser
        115                 120                 125

His Tyr Leu Trp Phe Gly Arg Asp Phe Phe Gly Arg Arg Ser Leu Leu
    130                 135                 140

Trp His Phe Ser Asn Leu Gly Lys Ser Phe Cys Leu Ser Ser Val Gly
145                 150                 155                 160

Thr Gln Thr Ser Gly Leu Ala Asn Gln Trp Gln Glu Val Pro Ala Ser
                165                 170                 175

Gly Leu Phe Arg Ile Asp Leu Lys Ser Thr Val Ile Ser Gly Cys Ile
            180                 185                 190

Ile Leu Gln Leu Tyr Pro Trp Lys Tyr Ile Ser Arg Glu Asn Ile Ile
        195                 200                 205

Glu Glu Asn Val Asn Ser Leu Ser Gln Ile Ser Ala Asp Leu Pro Ala
    210                 215                 220

Phe Val Ser Val Val Ala Asn Glu Ala Lys Leu Tyr Leu Glu Lys Pro
225                 230                 235                 240

Val Val Pro Leu Asn Met Met Leu Pro Gln Ala Ala Leu Glu Thr His
                245                 250                 255

Cys Ser Asn Ile Ser Asn Val Pro Pro Thr Arg Glu Ile Leu Gln Val
            260                 265                 270

Phe Leu Thr Asp Val His Met Lys Glu Val Ile Gln Gln Phe Ile Asp
        275                 280                 285

Val Leu Ser Val Ala Val Lys Lys Arg Val Leu Cys Leu Pro Arg Asp
    290                 295                 300

Glu Asn Leu Thr Ala Asn Glu Val Leu Lys Thr Cys Asp Arg Lys Ala
305                 310                 315                 320

Asn Val Ala Ile Leu Phe Ser Gly Gly Ile Asp Ser Met Val Ile Ala
                325                 330                 335

Thr Leu Ala Asp Arg His Ile Pro Leu Asp Glu Pro Ile Asp Leu Leu
            340                 345                 350

Asn Val Ala Phe Ile Ala Glu Glu Lys Thr Met Pro Thr Thr Phe Asn
        355                 360                 365

Arg Glu Gly Asn Lys Gln Lys Asn Lys Cys Glu Ile Pro Ser Glu Glu
    370                 375                 380

Phe Ser Lys Asp Val Ala Ala Ala Ala Ala Asp Ser Pro Asn Lys His
385                 390                 395                 400

Val Ser Val Pro Asp Arg Ile Thr Gly Arg Ala Gly Leu Lys Glu Leu
                405                 410                 415

Gln Ala Val Ser Pro Ser Arg Ile Trp Asn Phe Val Glu Ile Asn Val
            420                 425                 430

Ser Met Glu Glu Leu Gln Lys Leu Arg Arg Thr Arg Ile Cys His Leu

435                 440                 445

Ile Arg Pro Leu Asp Thr Val Leu Asp Asp Ser Ile Gly Cys Ala Val
    450                 455                 460

Trp Phe Ala Ser Arg Gly Ile Gly Trp Leu Val Ala Gln Glu Gly Val
465                 470                 475                 480

Lys Ser Tyr Gln Ser Asn Ala Lys Val Val Leu Thr Gly Ile Gly Ala
                485                 490                 495

Asp Glu Gln Leu Ala Gly Tyr Ser Arg His Arg Val Arg Phe Gln Ser
            500                 505                 510

His Gly Leu Glu Gly Leu Asn Lys Glu Ile Met Met Glu Leu Gly Arg
        515                 520                 525

Ile Ser Ser Arg Asn Leu Gly Arg Asp Asp Arg Val Ile Gly Asp His
    530                 535                 540

Gly Lys Glu Ala Arg Phe Pro Phe Leu Asp Glu Asn Val Val Ser Phe
545                 550                 555                 560

Leu Asn Ser Leu Pro Ile Trp Glu Lys Ala Asn Leu Thr Leu Pro Arg
                565                 570                 575

Gly Ile Gly Glu Lys Leu Leu Leu Arg Leu Ala Ala Val Glu Leu Gly
            580                 585                 590

Leu Thr Ala Ser Ala Leu Leu Pro Lys Arg Ala Met Gln Phe Gly Ser
        595                 600                 605

Arg Ile Ala Lys Met Glu Lys Ile Asn Glu Lys Ala Ser Asp Lys Cys
    610                 615                 620

Gly Arg Leu Gln Ile Met Ser Leu Glu Asn Leu Ser Ile Glu Lys Glu
625                 630                 635                 640

Thr Lys Leu

<210>  11
<211>  664
<212>  PRT
<213>  Homo sapiens

<400>  11

Met Glu Arg Pro Glu Glu Gly Lys Gln Ser Pro Pro Pro Gln Pro Trp

1               5                   10                  15

Gly Arg Leu Leu Arg Leu Gly Ala Glu Glu Gly Glu Pro His Val Leu
            20                  25                  30

Leu Arg Lys Arg Glu Trp Thr Ile Gly Arg Arg Arg Gly Cys Asp Leu
        35                  40                  45

Ser Phe Pro Ser Asn Lys Leu Val Ser Gly Asp His Cys Arg Ile Val
    50                  55                  60

Val Asp Glu Lys Ser Gly Gln Val Thr Leu Glu Asp Thr Ser Thr Ser
65                  70                  75                  80

Gly Thr Val Ile Asn Lys Leu Lys Val Val Lys Lys Gln Thr Cys Pro
                85                  90                  95

Leu Gln Thr Gly Asp Val Ile Tyr Leu Val Tyr Arg Lys Asn Glu Pro
            100                 105                 110

Glu His Asn Val Ala Tyr Leu Tyr Glu Ser Leu Ser Glu Lys Gln Gly
        115                 120                 125

Met Thr Gln Glu Ser Phe Glu Ala Asn Lys Glu Asn Val Phe His Gly
    130                 135                 140

Thr Lys Asp Thr Ser Gly Ala Gly Ala Gly Arg Gly Ala Asp Pro Arg
145                 150                 155                 160

Val Pro Pro Ser Ser Pro Ala Thr Gln Val Cys Phe Glu Glu Pro Gln
                165                 170                 175

Pro Ser Thr Ser Thr Ser Asp Leu Phe Pro Thr Ala Ser Ala Ser Ser
            180                 185                 190

Thr Glu Pro Ser Pro Ala Gly Arg Glu Arg Ser Ser Ser Cys Gly Ser
        195                 200                 205

Gly Gly Gly Gly Ile Ser Pro Lys Gly Ser Gly Pro Ser Val Ala Ser
    210                 215                 220

Asp Glu Val Ser Ser Phe Ala Ser Ala Leu Pro Asp Arg Lys Thr Ala
225                 230                 235                 240

Ser Phe Ser Ser Leu Glu Pro Gln Asp Gln Glu Asp Leu Glu Pro Val
                245                 250                 255

Lys Lys Lys Met Arg Gly Asp Gly Asp Leu Asp Leu Asn Gly Gln Leu
            260                 265                 270

Leu Val Ala Gln Pro Arg Arg Asn Ala Gln Thr Val His Glu Asp Val
        275                 280                 285

Arg Ala Ala Ala Gly Lys Pro Asp Lys Met Glu Glu Thr Leu Thr Cys
    290                 295                 300

Ile Ile Cys Gln Asp Leu Leu His Asp Cys Val Ser Leu Gln Pro Cys
305                 310                 315                 320

Met His Thr Phe Cys Ala Ala Cys Tyr Ser Gly Trp Met Glu Arg Ser
                325                 330                 335

Ser Leu Cys Pro Thr Cys Arg Cys Pro Val Glu Arg Ile Cys Lys Asn
            340                 345                 350

His Ile Leu Asn Asn Leu Val Glu Ala Tyr Leu Ile Gln His Pro Asp
        355                 360                 365

Lys Ser Arg Ser Glu Glu Asp Val Gln Ser Met Asp Ala Arg Asn Lys
    370                 375                 380

Ile Thr Gln Asp Met Leu Gln Pro Lys Val Arg Arg Ser Phe Ser Asp
385                 390                 395                 400

Glu Glu Gly Ser Ser Glu Asp Leu Leu Glu Leu Ser Asp Val Asp Ser
                405                 410                 415

Glu Ser Ser Asp Ile Ser Gln Pro Tyr Val Val Cys Arg Gln Cys Pro
            420                 425                 430

Glu Tyr Arg Arg Gln Ala Ala Gln Pro Pro His Cys Pro Ala Pro Glu
        435                 440                 445

Gly Glu Pro Gly Ala Pro Gln Ala Leu Gly Asp Ala Pro Ser Thr Ser
    450                 455                 460

Val Ser Leu Thr Thr Ala Val Gln Asp Tyr Val Cys Pro Leu Gln Gly
465                 470                 475                 480

Ser His Ala Leu Cys Thr Cys Cys Phe Gln Pro Met Pro Asp Arg Arg
                485                 490                 495

Ala Glu Arg Glu Gln Asp Pro Arg Val Ala Pro Gln Gln Cys Ala Val
            500                 505                 510

Cys Leu Gln Pro Phe Cys His Leu Tyr Trp Gly Cys Thr Arg Thr Gly
        515                 520                 525

Cys Tyr Gly Cys Leu Ala Pro Phe Cys Glu Leu Asn Leu Gly Asp Lys
    530                 535                 540

Cys Leu Asp Gly Val Leu Asn Asn Asn Ser Tyr Glu Ser Asp Ile Leu
545                 550                 555                 560

Lys Asn Tyr Leu Ala Thr Arg Gly Leu Thr Trp Lys Asn Met Leu Thr
                565                 570                 575

Glu Ser Leu Val Ala Leu Gln Arg Gly Val Phe Leu Leu Ser Asp Tyr
            580                 585                 590

Arg Val Thr Gly Asp Thr Val Leu Cys Tyr Cys Cys Gly Leu Arg Ser
        595                 600                 605

Phe Arg Glu Leu Thr Tyr Gln Tyr Arg Gln Asn Ile Pro Ala Ser Glu
    610                 615                 620

Leu Pro Val Ala Val Thr Ser Arg Pro Asp Cys Tyr Trp Gly Arg Asn
625                 630                 635                 640

Cys Arg Thr Gln Val Lys Ala His His Ala Met Lys Phe Asn His Ile
                645                 650                 655

Cys Glu Gln Thr Arg Phe Lys Asn
            660

<210>  12
<211>  604
<212>  PRT
<213>  Homo sapiens

<400>  12

Met Met Arg Ala Val Trp Glu Ala Leu Ala Ala Leu Ala Ala Val Ala
1               5                   10                  15

Cys Leu Val Gly Ala Val Arg Gly Gly Pro Gly Leu Ser Met Phe Ala
            20                  25                  30

Gly Gln Ala Ala Gln Pro Asp Pro Cys Ser Asp Glu Asn Gly His Pro
        35                  40                  45

Arg Arg Cys Ile Pro Asp Phe Val Asn Ala Ala Phe Gly Lys Asp Val
    50                  55                  60

Arg Val Ser Ser Thr Cys Gly Arg Pro Pro Ala Arg Tyr Cys Val Val
65                  70                  75                  80

Ser Glu Arg Gly Glu Glu Arg Leu Arg Ser Cys His Leu Cys Asn Ala
                85                  90                  95

Ser Asp Pro Lys Lys Ala His Pro Pro Ala Phe Leu Thr Asp Leu Asn
            100                 105                 110

Asn Pro His Asn Leu Thr Cys Trp Gln Ser Glu Asn Tyr Leu Gln Phe
        115                 120                 125

Pro His Asn Val Thr Leu Thr Leu Ser Leu Gly Lys Lys Phe Glu Val
    130                 135                 140

Thr Tyr Val Ser Leu Gln Phe Cys Ser Pro Arg Pro Glu Ser Met Ala
145                 150                 155                 160

Ile Tyr Lys Ser Met Asp Tyr Gly Arg Thr Trp Val Pro Phe Gln Phe
                165                 170                 175

Tyr Ser Thr Gln Cys Arg Lys Met Tyr Asn Arg Pro His Arg Ala Pro
            180                 185                 190

Ile Thr Lys Gln Asn Glu Gln Glu Ala Val Cys Thr Asp Ser His Thr
        195                 200                 205

Asp Met Arg Pro Leu Ser Gly Gly Leu Ile Ala Phe Ser Thr Leu Asp
    210                 215                 220

Gly Arg Pro Ser Ala His Asp Phe Asp Asn Ser Pro Val Leu Gln Asp
225                 230                 235                 240

Trp Val Thr Ala Thr Asp Ile Arg Val Ala Phe Ser Arg Leu His Thr
                245                 250                 255

Phe Gly Asp Glu Asn Glu Asp Asp Ser Glu Leu Ala Arg Asp Ser Tyr
            260                 265                 270

Phe Tyr Ala Val Ser Asp Leu Gln Val Gly Gly Arg Cys Lys Cys Asn
        275                 280                 285

Gly His Ala Ala Arg Cys Val Arg Asp Arg Asp Asp Ser Leu Val Cys
    290                 295                 300

Asp Cys Arg His Asn Thr Ala Gly Pro Glu Cys Asp Arg Cys Lys Pro
305                 310                 315                 320

Phe His Tyr Asp Arg Pro Trp Gln Arg Ala Thr Ala Arg Glu Ala Asn
                325                 330                 335

Glu Cys Val Ala Cys Asn Cys Asn Leu His Ala Arg Arg Cys Arg Phe
            340                 345                 350

Asn Met Glu Leu Tyr Lys Leu Ser Gly Arg Lys Ser Gly Gly Val Cys
        355                 360                 365

Leu Asn Cys Arg His Asn Thr Ala Gly Arg His Cys His Tyr Cys Lys
    370                 375                 380

Glu Gly Tyr Tyr Arg Asp Met Gly Lys Pro Ile Thr His Arg Lys Ala
385                 390                 395                 400

Cys Lys Ala Cys Asp Cys His Pro Val Gly Ala Ala Gly Lys Thr Cys
                405                 410                 415

Asn Gln Thr Thr Gly Gln Cys Pro Cys Lys Asp Gly Val Thr Gly Ile
            420                 425                 430

Thr Cys Asn Arg Cys Ala Lys Gly Tyr Gln Gln Ser Arg Ser Pro Ile
        435                 440                 445

Ala Pro Cys Ile Lys Ile Pro Val Ala Pro Pro Thr Thr Ala Ala Ser
    450                 455                 460

Ser Val Glu Glu Pro Glu Asp Cys Asp Ser Tyr Cys Lys Ala Ser Lys
465                 470                 475                 480

Gly Lys Leu Lys Ile Asn Met Lys Lys Tyr Cys Lys Lys Asp Tyr Ala
                485                 490                 495

Val Gln Ile His Ile Leu Lys Ala Asp Lys Ala Gly Asp Trp Trp Lys
            500                 505                 510

Phe Thr Val Asn Ile Ile Ser Val Tyr Lys Gln Gly Thr Ser Arg Ile
        515                 520                 525

Arg Arg Gly Asp Gln Ser Leu Trp Ile Arg Ser Arg Asp Ile Ala Cys
    530                 535                 540

Lys Cys Pro Lys Ile Lys Pro Leu Lys Lys Tyr Leu Leu Leu Gly Asn
545                 550                 555                 560

Ala Glu Asp Ser Pro Asp Gln Ser Gly Ile Val Ala Asp Lys Ser Ser

565                 570                 575

Leu Val Ile Gln Trp Arg Asp Thr Trp Ala Arg Arg Leu Arg Lys Phe
            580                 585                 590

Gln Gln Arg Glu Lys Lys Gly Lys Cys Lys Lys Ala
        595                 600

<210>  13
<211>  354
<212>  PRT
<213>  Homo sapiens

<400>  13

Met Ser Leu Trp Val Asp Lys Tyr Arg Pro Lys Ser Leu Asn Ala Leu
1               5                   10                  15

Ser His Asn Glu Glu Leu Thr Asn Phe Leu Lys Ser Leu Ser Asp Gln
            20                  25                  30

Pro Arg Asp Leu Pro His Leu Leu Leu Tyr Gly Pro Asn Gly Thr Gly
        35                  40                  45

Lys Lys Thr Arg Cys Met Ala Leu Leu Glu Ser Ile Phe Gly Pro Gly
    50                  55                  60

Val Tyr Arg Leu Lys Ile Asp Val Arg Gln Phe Val Thr Ala Ser Asn
65                  70                  75                  80

Arg Lys Leu Glu Leu Asn Val Val Ser Ser Pro Tyr His Leu Glu Ile
                85                  90                  95

Thr Pro Ser Asp Met Gly Asn Asn Asp Arg Ile Val Ile Gln Glu Leu
            100                 105                 110

Leu Lys Glu Val Ala Gln Met Glu Gln Val Asp Phe Gln Asp Ser Lys
        115                 120                 125

Asp Gly Leu Ala His Arg Tyr Lys Cys Val Ile Ile Asn Glu Ala Asn
    130                 135                 140

Ser Leu Thr Lys Asp Ala Gln Ala Ala Leu Arg Arg Thr Met Glu Lys
145                 150                 155                 160

Tyr Ser Lys Asn Ile Arg Leu Ile Met Val Cys Asp Ser Met Ser Pro
                165                 170                 175

Ile Ile Ala Pro Ile Lys Ser Arg Cys Leu Leu Ile Arg Cys Pro Ala

180                 185                 190

Pro Ser Asp Ser Glu Ile Ser Thr Ile Leu Ser Asp Val Val Thr Asn
        195                 200                 205

Glu Arg Ile Gln Leu Glu Thr Lys Asp Ile Leu Lys Arg Ile Ala Gln
    210                 215                 220

Ala Ser Asn Gly Asn Leu Arg Val Ser Leu Leu Met Leu Glu Ser Met
225                 230                 235                 240

Ala Leu Asn Asn Glu Leu Ala Leu Lys Ser Ser Ser Pro Ile Ile Lys
                245                 250                 255

Pro Asp Trp Ile Ile Val Ile His Lys Leu Thr Arg Lys Ile Val Lys
            260                 265                 270

Glu Arg Ser Val Asn Ser Leu Ile Glu Cys Arg Ala Val Leu Tyr Asp
        275                 280                 285

Leu Leu Ala His Cys Ile Pro Ala Asn Ile Ile Leu Lys Glu Leu Thr
    290                 295                 300

Phe Ser Leu Leu Asp Val Glu Thr Leu Asn Thr Thr Asn Lys Ser Ser
305                 310                 315                 320

Ile Ile Glu Tyr Ser Ser Val Phe Asp Glu Arg Leu Ser Leu Gly Asn
                325                 330                 335

Lys Ala Ile Phe His Leu Glu Gly Phe Ile Ala Lys Val Met Cys Cys
            340                 345                 350

Leu Asp

<210>  14
<211>  740
<212>  PRT
<213>  Homo sapiens

<400>  14

Met Glu Lys Leu Gly Val Glu Pro Glu Glu Glu Gly Gly Gly Asp Asp
1               5                   10                  15

Asp Glu Glu Asp Ala Glu Ala Trp Ala Met Glu Leu Ala Asp Val Gly
            20                  25                  30

Ala Ala Ala Ser Ser Gln Gly Val His Asp Gln Val Leu Pro Thr Pro

35                  40                  45

Asn Ala Ser Ser Arg Val Ile Val His Val Asp Leu Asp Cys Phe Tyr
    50                  55                  60

Ala Gln Val Glu Met Ile Ser Asn Pro Glu Leu Lys Asp Lys Pro Leu
65                  70                  75                  80

Gly Val Gln Gln Lys Tyr Leu Val Val Thr Cys Asn Tyr Glu Ala Arg
                85                  90                  95

Lys Leu Gly Val Lys Lys Leu Met Asn Val Arg Asp Ala Lys Glu Lys
            100                 105                 110

Cys Pro Gln Leu Val Leu Val Asn Gly Glu Asp Leu Thr Arg Tyr Arg
        115                 120                 125

Glu Met Ser Tyr Lys Val Thr Glu Leu Leu Glu Glu Phe Ser Pro Val
    130                 135                 140

Val Glu Arg Leu Gly Phe Asp Glu Asn Phe Val Asp Leu Thr Glu Met
145                 150                 155                 160

Val Glu Lys Arg Leu Gln Gln Leu Gln Ser Asp Glu Leu Ser Ala Val
                165                 170                 175

Thr Val Ser Gly His Val Tyr Asn Asn Gln Ser Ile Asn Leu Leu Asp
            180                 185                 190

Val Leu His Ile Arg Leu Leu Val Gly Ser Gln Ile Ala Ala Glu Met
        195                 200                 205

Arg Glu Ala Met Tyr Asn Gln Leu Gly Leu Thr Gly Cys Ala Gly Val
    210                 215                 220

Ala Ser Asn Lys Leu Leu Ala Lys Leu Val Ser Gly Val Phe Lys Pro
225                 230                 235                 240

Asn Gln Gln Thr Val Leu Leu Pro Glu Ser Cys Gln His Leu Ile His
                245                 250                 255

Ser Leu Asn His Ile Lys Glu Ile Pro Gly Ile Gly Tyr Lys Thr Ala
            260                 265                 270

Lys Cys Leu Glu Ala Leu Gly Ile Asn Ser Val Arg Asp Leu Gln Thr
        275                 280                 285

Phe Ser Pro Lys Ile Leu Glu Lys Glu Leu Gly Ile Ser Val Ala Gln
    290                 295                 300

Arg Ile Gln Lys Leu Ser Phe Gly Glu Asp Asn Ser Pro Val Ile Leu
305                 310                 315                 320

Ser Gly Pro Pro Gln Ser Phe Ser Glu Glu Asp Ser Phe Lys Lys Cys
                325                 330                 335

Ser Ser Glu Val Glu Ala Lys Asn Lys Ile Glu Glu Leu Leu Ala Ser
            340                 345                 350

Leu Leu Asn Arg Val Cys Gln Asp Gly Arg Lys Pro His Thr Val Arg
        355                 360                 365

Leu Ile Ile Arg Arg Tyr Ser Ser Glu Lys His Tyr Gly Arg Glu Ser
    370                 375                 380

Arg Gln Cys Pro Ile Pro Ser His Val Ile Gln Lys Leu Gly Thr Gly
385                 390                 395                 400

Asn Tyr Asp Val Met Thr Pro Met Val Asp Ile Leu Met Lys Leu Phe
                405                 410                 415

Arg Asn Met Val Asn Val Lys Met Pro Phe His Leu Thr Leu Leu Ser
            420                 425                 430

Val Cys Phe Cys Asn Leu Lys Ala Leu Asn Thr Ala Lys Lys Gly Leu
        435                 440                 445

Ile Asp Tyr Tyr Leu Met Pro Ser Leu Ser Thr Thr Ser Arg Ser Gly
    450                 455                 460

Lys His Ser Phe Lys Met Lys Asp Thr His Met Glu Asp Phe Pro Lys
465                 470                 475                 480

Asp Lys Glu Thr Asn Arg Asp Phe Leu Pro Ser Gly Arg Ile Glu Ser
                485                 490                 495

Thr Arg Thr Arg Glu Ser Pro Leu Asp Thr Thr Asn Phe Ser Lys Glu
            500                 505                 510

Lys Asp Ile Asn Glu Phe Pro Leu Cys Ser Leu Pro Glu Gly Val Asp
        515                 520                 525

Gln Glu Val Phe Lys Gln Leu Pro Val Asp Ile Gln Glu Glu Ile Leu
    530                 535                 540

Ser Gly Lys Ser Arg Glu Lys Phe Gln Gly Lys Gly Ser Val Ser Cys
545                 550                 555                 560

Pro Leu His Ala Ser Arg Gly Val Leu Ser Phe Phe Ser Lys Lys Gln
                565                 570                 575

Met Gln Asp Ile Pro Ile Asn Pro Arg Asp His Leu Ser Ser Ser Lys
            580                 585                 590

Gln Val Ser Ser Val Ser Pro Cys Glu Pro Gly Thr Ser Gly Phe Asn
        595                 600                 605

Ser Ser Ser Ser Ser Tyr Met Ser Ser Gln Lys Asp Tyr Ser Tyr Tyr
    610                 615                 620

Leu Asp Asn Arg Leu Lys Asp Glu Arg Ile Ser Gln Gly Pro Lys Glu
625                 630                 635                 640

Pro Gln Gly Phe His Phe Thr Asn Ser Asn Pro Ala Val Ser Ala Phe
                645                 650                 655

His Ser Phe Pro Asn Leu Gln Ser Glu Gln Leu Phe Ser Arg Asn His
            660                 665                 670

Thr Thr Asp Ser His Lys Gln Thr Val Ala Thr Asp Ser His Glu Gly
        675                 680                 685

Leu Thr Glu Asn Arg Glu Pro Asp Ser Val Asp Glu Lys Ile Thr Phe
    690                 695                 700

Pro Ser Asp Ile Asp Pro Gln Val Phe Tyr Glu Leu Pro Glu Ala Val
705                 710                 715                 720

Gln Lys Glu Leu Leu Ala Glu Trp Lys Arg Ala Gly Ser Asp Phe His
                725                 730                 735

Ile Gly His Lys
            740

<210>  15
<211>  757
<212>  PRT
<213>  Homo sapiens

<400>  15

<210>  16
<211>  589
<212>  PRT
<213>  Homo sapiens

<400>  16

Met Ser Ser Ser Tyr Trp Ser Glu Thr Ser Ser Ser Ser Cys Gly Thr
1               5                   10                  15

Gln Gln Leu Pro Glu Val Leu Gln Cys Gln Pro Gln His Tyr His Cys
            20                  25                  30

Tyr His Gln Ser Ser Gln Ala Gln Gln Pro Pro Glu Lys Asn Val Val
        35                  40                  45

Tyr Glu Arg Val Arg Thr Tyr Ser Gly Pro Met Asn Lys Val Val Gln
    50                  55                  60

Ala Leu Asp Pro Phe Asn Ser Arg Glu Val Leu Ser Pro Leu Lys Thr
65                  70                  75                  80

Thr Ser Ser Tyr Gln Asn Leu Val Trp Ser Asp His Ser Gln Glu Leu
                85                  90                  95

His Ser Pro Thr Leu Lys Ile Ser Thr Cys Ala Pro Ser Thr Leu His
            100                 105                 110

Ile Thr Gln Asn Thr Glu Gln Glu Leu His Ser Pro Thr Val Lys Leu
        115                 120                 125

Thr Thr Tyr Pro Gln Thr Thr Ile Arg Lys Tyr Val Val Gln Asn Pro
    130                 135                 140

Glu Gln Glu Pro Leu Ser Gln Phe Leu Arg Gly Ser His Phe Phe Pro
145                 150                 155                 160

Gly Asn Asn Val Ile Tyr Glu Lys Thr Ile Arg Lys Val Glu Lys Leu
                165                 170                 175

Asn Thr Asp Gln Gly Cys His Pro Gln Ala Gln Cys His His His Ile
            180                 185                 190

Ile Gln Gln Pro Gln Val Ile His Ser Ala His Trp Gln Gln Pro Asp
        195                 200                 205

Ser Ser Gln Gln Ile Gln Ala Ile Thr Gly Asn Asn Pro Ile Ser Thr
    210                 215                 220

His Ile Gly Asn Glu Leu Cys His Ser Gly Ser Ser Gln Ile Cys Glu
225                 230                 235                 240

Gln Val Ile Ile Gln Asp Asp Gly Pro Glu Lys Leu Asp Pro Arg Tyr
                245                 250                 255

Phe Gly Glu Leu Leu Ala Asp Leu Ser Arg Lys Asn Thr Asp Leu Tyr
            260                 265                 270

His Cys Leu Leu Glu His Leu Gln Arg Ile Gly Gly Ser Lys Gln Asp
        275                 280                 285

Phe Glu Ser Thr Asp Glu Ser Glu Asp Ile Glu Ser Leu Ile Pro Lys
    290                 295                 300

Gly Leu Ser Glu Phe Thr Lys Gln Gln Ile Arg Tyr Ile Leu Gln Met
305                 310                 315                 320

Arg Gly Met Ser Asp Lys Ser Leu Arg Leu Val Leu Ser Thr Phe Ser
                325                 330                 335

Asn Ile Arg Glu Glu Leu Gly His Leu Gln Asn Asp Met Thr Ser Leu
            340                 345                 350

Glu Asn Asp Lys Met Arg Leu Glu Lys Asp Leu Ser Phe Lys Asp Thr
        355                 360                 365

Gln Leu Lys Glu Tyr Glu Glu Leu Leu Ala Ser Val Arg Ala Asn Asn
    370                 375                 380

His Gln Gln Gln Gln Gly Leu Gln Asp Ser Ser Ser Lys Cys Gln Ala
385                 390                 395                 400

Leu Glu Glu Asn Asn Leu Ser Leu Arg His Thr Leu Ser Asp Met Glu
                405                 410                 415

Tyr Arg Leu Lys Glu Leu Glu Tyr Cys Lys Arg Asn Leu Glu Gln Glu
            420                 425                 430

Asn Gln Asn Leu Arg Met Gln Val Ser Glu Thr Cys Thr Gly Pro Met
        435                 440                 445

Leu Gln Ala Lys Met Asp Glu Ile Gly Asn His Tyr Thr Glu Met Val
    450                 455                 460

Lys Asn Leu Arg Met Glu Lys Asp Arg Glu Ile Cys Arg Leu Arg Ser
465                 470                 475                 480

Gln Leu Asn Gln Tyr His Lys Asp Val Ser Lys Arg Glu Gly Ser Cys
                485                 490                 495

Ser Asp Phe Gln Phe Lys Leu His Glu Leu Thr Ser Leu Leu Glu Glu
            500                 505                 510

Lys Asp Ser Leu Ile Lys Arg Gln Ser Glu Glu Leu Ser Lys Leu Arg
        515                 520                 525

Gln Glu Ile Tyr Ser Ser His Asn Gln Pro Ser Thr Gly Gly Arg Thr
    530                 535                 540

Thr Ile Thr Thr Lys Lys Tyr Arg Thr Gln Tyr Pro Ile Leu Gly Leu
545                 550                 555                 560

Leu Tyr Asp Asp Tyr Glu Tyr Ile Pro Pro Gly Ser Glu Thr Gln Thr
                565                 570                 575

Ile Val Ile Glu Lys Thr Glu Asp Lys Tyr Thr Cys Pro
            580                 585

<210>  17
<211>  144
<212>  PRT
<213>  Homo sapiens

<400>  17

Met Lys Thr Leu Leu Leu Leu Ala Val Ile Met Ile Phe Gly Leu Leu
1               5                   10                  15

Gln Ala His Gly Asn Leu Val Asn Phe His Arg Met Ile Lys Leu Thr
            20                  25                  30

Thr Gly Lys Glu Ala Ala Leu Ser Tyr Gly Phe Tyr Gly Cys His Cys
        35                  40                  45

Gly Val Gly Gly Arg Gly Ser Pro Lys Asp Ala Thr Asp Arg Cys Cys
    50                  55                  60

Val Thr His Asp Cys Cys Tyr Lys Arg Leu Glu Lys Arg Gly Cys Gly
65                  70                  75                  80

Thr Lys Phe Leu Ser Tyr Lys Phe Ser Asn Ser Gly Ser Arg Ile Thr
                85                  90                  95

Cys Ala Lys Gln Asp Ser Cys Arg Ser Gln Leu Cys Glu Cys Asp Lys
            100                 105                 110

Ala Ala Ala Thr Cys Phe Ala Arg Asn Lys Thr Thr Tyr Asn Lys Lys
        115                 120                 125

Tyr Gln Tyr Tyr Ser Asn Lys His Cys Arg Gly Ser Thr Pro Arg Cys
    130                 135                 140

<210>  18
<211>  669
<212>  PRT
<213>  Homo sapiens

<400>  18

Met Gly Thr Ala Leu Val Tyr His Glu Asp Met Thr Ala Thr Arg Leu
1               5                   10                  15

Leu Trp Asp Asp Pro Glu Cys Glu Ile Glu Arg Pro Glu Arg Leu Thr
            20                  25                  30

Ala Ala Leu Asp Arg Leu Arg Gln Arg Gly Leu Glu Gln Arg Cys Leu
        35                  40                  45

Arg Leu Ser Ala Arg Glu Ala Ser Glu Glu Glu Leu Gly Leu Val His
    50                  55                  60

Ser Pro Glu Tyr Val Ser Leu Val Arg Glu Thr Gln Val Leu Gly Lys
65                  70                  75                  80

Glu Glu Leu Gln Ala Leu Ser Gly Gln Phe Asp Ala Ile Tyr Phe His
                85                  90                  95

Pro Ser Thr Phe His Cys Ala Arg Leu Ala Ala Gly Ala Gly Leu Gln
            100                 105                 110

Leu Val Asp Ala Val Leu Thr Gly Ala Val Gln Asn Gly Leu Ala Leu
        115                 120                 125

Val Arg Pro Pro Gly His His Gly Gln Arg Ala Ala Ala Asn Gly Phe
    130                 135                 140

Cys Val Phe Asn Asn Val Ala Ile Ala Ala Ala His Ala Lys Gln Lys
145                 150                 155                 160

His Gly Leu His Arg Ile Leu Val Val Asp Trp Asp Val His His Gly
                165                 170                 175

Gln Gly Ile Gln Tyr Leu Phe Glu Asp Asp Pro Ser Val Leu Tyr Phe
            180                 185                 190

Ser Trp His Arg Tyr Glu His Gly Arg Phe Trp Pro Phe Leu Arg Glu
        195                 200                 205

Ser Asp Ala Asp Ala Val Gly Arg Gly Gln Gly Leu Gly Phe Thr Val
    210                 215                 220

Asn Leu Pro Trp Asn Gln Val Gly Met Gly Asn Ala Asp Tyr Val Ala
225                 230                 235                 240

Ala Phe Leu His Leu Leu Leu Pro Leu Ala Phe Glu Phe Asp Pro Glu
                245                 250                 255

Leu Val Leu Val Ser Ala Gly Phe Asp Ser Ala Ile Gly Asp Pro Glu
            260                 265                 270

Gly Gln Met Gln Ala Thr Pro Glu Cys Phe Ala His Leu Thr Gln Leu
        275                 280                 285

Leu Gln Val Leu Ala Gly Gly Arg Val Cys Ala Val Leu Glu Gly Gly
    290                 295                 300

Tyr His Leu Glu Ser Leu Ala Glu Ser Val Cys Met Thr Val Gln Thr
305                 310                 315                 320

Leu Leu Gly Asp Pro Ala Pro Pro Leu Ser Gly Pro Met Ala Pro Cys
                325                 330                 335

Gln Ser Ala Leu Glu Ser Ile Gln Ser Ala Arg Ala Ala Gln Ala Pro
            340                 345                 350

His Trp Lys Ser Leu Gln Gln Gln Asp Val Thr Ala Val Pro Met Ser
        355                 360                 365

Pro Ser Ser His Ser Pro Glu Gly Arg Pro Pro Pro Leu Leu Pro Gly
    370                 375                 380

Gly Pro Val Cys Lys Ala Ala Ala Ser Ala Pro Ser Ser Leu Leu Asp
385                 390                 395                 400

Gln Pro Cys Leu Cys Pro Ala Pro Ser Val Arg Thr Ala Val Ala Leu
                405                 410                 415

Thr Thr Pro Asp Ile Thr Leu Val Leu Pro Pro Asp Val Ile Gln Gln
            420                 425                 430

Glu Ala Ser Ala Leu Arg Glu Glu Thr Glu Ala Trp Ala Arg Pro His
        435                 440                 445

Glu Ser Leu Ala Arg Glu Glu Ala Leu Thr Ala Leu Gly Lys Leu Leu
    450                 455                 460

Tyr Leu Leu Asp Gly Met Leu Asp Gly Gln Val Asn Ser Gly Ile Ala
465                 470                 475                 480

Ala Thr Pro Ala Ser Ala Ala Ala Ala Thr Leu Asp Val Ala Val Arg
                485                 490                 495

Arg Gly Leu Ser His Gly Ala Gln Arg Leu Leu Cys Val Ala Leu Gly
            500                 505                 510

Gln Leu Asp Arg Pro Pro Asp Leu Ala His Asp Gly Arg Ser Leu Trp
        515                 520                 525

Leu Asn Ile Arg Gly Lys Glu Ala Ala Ala Leu Ser Met Phe His Val
    530                 535                 540

Ser Thr Pro Leu Pro Val Met Thr Gly Gly Phe Leu Ser Cys Ile Leu
545                 550                 555                 560

Gly Leu Val Leu Pro Leu Ala Tyr Gly Phe Gln Pro Asp Leu Val Leu
                565                 570                 575

Val Ala Leu Gly Pro Gly His Gly Leu Gln Gly Pro His Ala Ala Leu
            580                 585                 590

Leu Ala Ala Met Leu Arg Gly Leu Ala Gly Gly Arg Val Leu Ala Leu
        595                 600                 605

Leu Glu Glu Asn Ser Thr Pro Gln Leu Ala Gly Ile Leu Ala Arg Val
    610                 615                 620

Leu Asn Gly Glu Ala Pro Pro Ser Leu Gly Pro Ser Ser Val Ala Ser
625                 630                 635                 640

Pro Glu Asp Val Gln Ala Leu Met Tyr Leu Arg Gly Gln Leu Glu Pro
                645                 650                 655

Gln Trp Lys Met Leu Gln Cys His Pro His Leu Val Ala
            660                 665

<210>  19
<211>  573
<212>  PRT
<213>  Homo sapiens

<400>  19

Met Ala Arg Ala Val Gly Pro Glu Arg Arg Leu Leu Ala Val Tyr Thr
1               5                   10                  15

Gly Gly Thr Ile Gly Met Arg Ser Glu Leu Gly Val Leu Val Pro Gly
            20                  25                  30

Thr Gly Leu Ala Ala Ile Leu Arg Thr Leu Pro Met Phe His Asp Glu
        35                  40                  45

Glu His Ala Arg Ala Arg Gly Leu Ser Glu Asp Thr Leu Val Leu Pro
    50                  55                  60

Pro Ala Ser Arg Asn Gln Arg Ile Leu Tyr Thr Val Leu Glu Cys Gln
65                  70                  75                  80

Pro Leu Phe Asp Ser Ser Asp Met Thr Ile Ala Glu Trp Val Cys Leu
                85                  90                  95

Ala Gln Thr Ile Lys Arg His Tyr Glu Gln Tyr His Gly Phe Val Val
            100                 105                 110

Ile His Gly Thr Asp Thr Met Ala Phe Ala Ala Ser Met Leu Ser Phe
        115                 120                 125

Met Leu Glu Asn Leu Gln Lys Thr Val Ile Leu Thr Gly Ala Gln Val
    130                 135                 140

Pro Ile His Ala Leu Trp Ser Asp Gly Arg Glu Asn Leu Leu Gly Ala
145                 150                 155                 160

Leu Leu Met Ala Gly Gln Tyr Val Ile Pro Glu Val Cys Leu Phe Phe
                165                 170                 175

Gln Asn Gln Leu Phe Arg Gly Asn Arg Ala Thr Lys Val Asp Ala Arg
            180                 185                 190

Arg Phe Ala Ala Phe Cys Ser Pro Asn Leu Leu Pro Leu Ala Thr Val
        195                 200                 205

Gly Ala Asp Ile Thr Ile Asn Arg Glu Leu Val Arg Lys Val Asp Gly
    210                 215                 220

Lys Ala Gly Leu Val Val His Ser Ser Met Glu Gln Asp Val Gly Leu
225                 230                 235                 240

Leu Arg Leu Tyr Pro Gly Ile Pro Ala Ala Leu Val Arg Ala Phe Leu

245                 250                 255

Gln Pro Pro Leu Lys Gly Val Val Met Glu Thr Phe Gly Ser Gly Asn
            260                 265                 270

Gly Pro Thr Lys Pro Asp Leu Leu Gln Glu Leu Arg Val Ala Thr Glu
        275                 280                 285

Arg Gly Leu Val Ile Val Asn Cys Thr His Cys Leu Gln Gly Ala Val
    290                 295                 300

Thr Thr Asp Tyr Ala Ala Gly Met Ala Met Ala Gly Ala Gly Val Ile
305                 310                 315                 320

Ser Gly Phe Asp Met Thr Ser Glu Ala Ala Leu Ala Lys Leu Ser Tyr
                325                 330                 335

Val Leu Gly Gln Pro Gly Leu Ser Leu Asp Val Arg Lys Glu Leu Leu
            340                 345                 350

Thr Lys Asp Leu Arg Gly Glu Met Thr Pro Pro Ser Val Glu Glu Arg
        355                 360                 365

Arg Pro Ser Leu Gln Gly Asn Thr Leu Gly Gly Gly Val Ser Trp Leu
    370                 375                 380

Leu Ser Leu Ser Gly Ser Gln Glu Ala Asp Ala Leu Arg Asn Ala Leu
385                 390                 395                 400

Val Pro Ser Leu Ala Cys Ala Ala Ala His Ala Gly Asp Val Glu Ala
                405                 410                 415

Leu Gln Ala Leu Val Glu Leu Gly Ser Asp Leu Gly Leu Val Asp Phe
            420                 425                 430

Asn Gly Gln Thr Pro Leu His Ala Ala Ala Arg Gly Gly His Thr Glu
        435                 440                 445

Ala Val Thr Met Leu Leu Gln Arg Gly Val Asp Val Asn Thr Arg Asp
    450                 455                 460

Thr Asp Gly Phe Ser Pro Leu Leu Leu Ala Val Arg Gly Arg His Pro
465                 470                 475                 480

Gly Val Ile Gly Leu Leu Arg Glu Ala Gly Ala Ser Leu Ser Thr Gln
                485                 490                 495

Glu Leu Glu Glu Ala Gly Thr Glu Leu Cys Arg Leu Ala Tyr Arg Ala
        500                 505                 510

Asp Leu Glu Gly Leu Gln Val Trp Trp Gln Ala Gly Ala Asp Leu Gly
        515                 520                 525

Gln Pro Gly Tyr Asp Gly His Ser Ala Leu His Val Ala Glu Ala Ala
    530                 535                 540

Gly Asn Leu Ala Val Val Ala Phe Leu Gln Ser Leu Glu Gly Ala Val
545                 550                 555                 560

Gly Ala Gln Ala Pro Cys Pro Glu Val Leu Pro Gly Val
                565                 570

<210>  20
<211>  376
<212>  PRT
<213>  Homo sapiens

<400>  20

Met Gln Trp Thr Ser Leu Leu Leu Leu Ala Gly Leu Phe Ser Leu Ser
1               5                   10                  15

Gln Ala Gln Tyr Glu Asp Asp Pro His Trp Trp Phe His Tyr Leu Arg
            20                  25                  30

Ser Gln Gln Ser Thr Tyr Tyr Asp Pro Tyr Asp Pro Tyr Pro Tyr Glu
        35                  40                  45

Thr Tyr Glu Pro Tyr Pro Tyr Gly Val Asp Glu Gly Pro Ala Tyr Thr
    50                  55                  60

Tyr Gly Ser Pro Ser Pro Pro Asp Pro Arg Asp Cys Pro Gln Glu Cys
65                  70                  75                  80

Asp Cys Pro Pro Asn Phe Pro Thr Ala Met Tyr Cys Asp Asn Arg Asn
                85                  90                  95

Leu Lys Tyr Leu Pro Phe Val Pro Ser Arg Met Lys Tyr Val Tyr Phe
            100                 105                 110

Gln Asn Asn Gln Ile Thr Ser Ile Gln Glu Gly Val Phe Asp Asn Ala
        115                 120                 125

Thr Gly Leu Leu Trp Ile Ala Leu His Gly Asn Gln Ile Thr Ser Asp
    130                 135                 140

Lys Val Gly Arg Lys Val Phe Ser Lys Leu Arg His Leu Glu Arg Leu
145                 150                 155                 160

Tyr Leu Asp His Asn Asn Leu Thr Arg Met Pro Gly Pro Leu Pro Arg
                165                 170                 175

Ser Leu Arg Glu Leu His Leu Asp His Asn Gln Ile Ser Arg Val Pro
            180                 185                 190

Asn Asn Ala Leu Glu Gly Leu Glu Asn Leu Thr Ala Leu Tyr Leu Gln
        195                 200                 205

His Asn Glu Ile Gln Glu Val Gly Ser Ser Met Arg Gly Leu Arg Ser
    210                 215                 220

Leu Ile Leu Leu Asp Leu Ser Tyr Asn His Leu Arg Lys Val Pro Asp
225                 230                 235                 240

Gly Leu Pro Ser Ala Leu Glu Gln Leu Tyr Met Glu His Asn Asn Val
                245                 250                 255

Tyr Thr Val Pro Asp Ser Tyr Phe Arg Gly Ala Pro Lys Leu Leu Tyr
            260                 265                 270

Val Arg Leu Ser His Asn Ser Leu Thr Asn Asn Gly Leu Ala Ser Asn
        275                 280                 285

Thr Phe Asn Ser Ser Ser Leu Leu Glu Leu Asp Leu Ser Tyr Asn Gln
    290                 295                 300

Leu Gln Lys Ile Pro Pro Val Asn Thr Asn Leu Glu Asn Leu Tyr Leu
305                 310                 315                 320

Gln Gly Asn Arg Ile Asn Glu Phe Ser Ile Ser Ser Phe Cys Thr Val
                325                 330                 335

Val Asp Val Val Asn Phe Ser Lys Leu Gln Val Leu Arg Leu Asp Gly
            340                 345                 350

Asn Glu Ile Lys Arg Ser Ala Met Pro Ala Asp Ala Pro Leu Cys Leu
        355                 360                 365

Arg Leu Ala Ser Leu Ile Glu Ile
    370                 375

<210>  21
<211>  262
<212>  PRT

<213>  Homo sapiens

<400>  21

Met Ser Arg Leu Ser Trp Gly Tyr Arg Glu His Asn Gly Pro Ile His
1               5                   10                  15

Trp Lys Glu Phe Phe Pro Ile Ala Asp Gly Asp Gln Gln Ser Pro Ile
            20                  25                  30

Glu Ile Lys Thr Lys Glu Val Lys Tyr Asp Ser Ser Leu Arg Pro Leu
        35                  40                  45

Ser Ile Lys Tyr Asp Pro Ser Ser Ala Lys Ile Ile Ser Asn Ser Gly
    50                  55                  60

His Ser Phe Asn Val Asp Phe Asp Asp Thr Glu Asn Lys Ser Val Leu
65                  70                  75                  80

Arg Gly Gly Pro Leu Thr Gly Ser Tyr Arg Leu Arg Gln Val His Leu
                85                  90                  95

His Trp Gly Ser Ala Asp Asp His Gly Ser Glu His Ile Val Asp Gly
            100                 105                 110

Val Ser Tyr Ala Ala Glu Leu His Val Val His Trp Asn Ser Asp Lys
        115                 120                 125

Tyr Pro Ser Phe Val Glu Ala Ala His Glu Pro Asp Gly Leu Ala Val
    130                 135                 140

Leu Gly Val Phe Leu Gln Ile Gly Glu Pro Asn Ser Gln Leu Gln Lys
145                 150                 155                 160

Ile Thr Asp Thr Leu Asp Ser Ile Lys Glu Lys Gly Lys Gln Thr Arg
                165                 170                 175

Phe Thr Asn Phe Asp Leu Leu Ser Leu Leu Pro Pro Ser Trp Asp Tyr
            180                 185                 190

Trp Thr Tyr Pro Gly Ser Leu Thr Val Pro Pro Leu Leu Glu Ser Val
        195                 200                 205

Thr Trp Ile Val Leu Lys Gln Pro Ile Asn Ile Ser Ser Gln Gln Leu
    210                 215                 220

Ala Lys Phe Arg Ser Leu Leu Cys Thr Ala Glu Gly Glu Ala Ala Ala
225                 230                 235                 240

Phe Leu Val Ser Asn His Arg Pro Pro Gln Pro Leu Lys Gly Arg Lys
                245                 250                 255

Val Arg Ala Ser Phe His
            260

<210>  22
<211>  1150
<212>  PRT
<213>  Homo sapiens

<400>  22

Met Ala Val Pro Ala Arg Thr Cys Gly Ala Ser Arg Pro Gly Pro Ala
1               5                   10                  15

Arg Thr Ala Arg Pro Trp Pro Gly Cys Gly Pro His Pro Gly Pro Gly
            20                  25                  30

Thr Arg Arg Pro Thr Ser Gly Pro Pro Arg Pro Leu Trp Leu Leu Leu
        35                  40                  45

Pro Leu Leu Pro Leu Leu Ala Ala Pro Gly Ala Ser Ala Tyr Ser Phe
    50                  55                  60

Pro Gln Gln His Thr Met Gln His Trp Ala Arg Arg Leu Glu Gln Glu
65                  70                  75                  80

Val Asp Gly Val Met Arg Ile Phe Gly Gly Val Gln Gln Leu Arg Glu
                85                  90                  95

Ile Tyr Lys Asp Asn Arg Asn Leu Phe Glu Val Gln Glu Asn Glu Pro
            100                 105                 110

Gln Lys Leu Val Glu Lys Val Ala Gly Asp Ile Glu Ser Leu Leu Asp
        115                 120                 125

Arg Lys Val Gln Ala Leu Lys Arg Leu Ala Asp Ala Ala Glu Asn Phe
    130                 135                 140

Gln Lys Ala His Arg Trp Gln Asp Asn Ile Lys Glu Glu Asp Ile Val
145                 150                 155                 160

Tyr Tyr Asp Ala Lys Ala Asp Ala Glu Leu Asp Asp Pro Glu Ser Glu
                165                 170                 175

Asp Val Glu Arg Gly Ser Lys Ala Ser Thr Leu Arg Leu Asp Phe Ile
            180                 185                 190

Glu Asp Pro Asn Phe Lys Asn Lys Val Asn Tyr Ser Tyr Ala Ala Val
        195                 200                 205

Gln Ile Pro Thr Asp Ile Tyr Lys Gly Ser Thr Val Ile Leu Asn Glu
    210                 215                 220

Leu Asn Trp Thr Glu Ala Leu Glu Asn Val Phe Met Glu Asn Arg Arg
225                 230                 235                 240

Gln Asp Pro Thr Leu Leu Trp Gln Val Phe Gly Ser Ala Thr Gly Val
                245                 250                 255

Thr Arg Tyr Tyr Pro Ala Thr Pro Trp Arg Ala Pro Lys Lys Ile Asp
            260                 265                 270

Leu Tyr Asp Val Arg Arg Arg Pro Trp Tyr Ile Gln Gly Ala Ser Ser
        275                 280                 285

Pro Lys Asp Met Val Ile Ile Val Asp Val Ser Gly Ser Val Ser Gly
    290                 295                 300

Leu Thr Leu Lys Leu Met Lys Thr Ser Val Cys Glu Met Leu Asp Thr
305                 310                 315                 320

Leu Ser Asp Asp Asp Tyr Val Asn Val Ala Ser Phe Asn Glu Lys Ala
                325                 330                 335

Gln Pro Val Ser Cys Phe Thr His Leu Val Gln Ala Asn Val Arg Asn
            340                 345                 350

Lys Lys Val Phe Lys Glu Ala Val Gln Gly Met Val Ala Lys Gly Thr
        355                 360                 365

Thr Gly Tyr Lys Ala Gly Phe Glu Tyr Ala Phe Asp Gln Leu Gln Asn
    370                 375                 380

Ser Asn Ile Thr Arg Ala Asn Cys Asn Lys Met Ile Met Met Phe Thr
385                 390                 395                 400

Asp Gly Gly Glu Asp Arg Val Gln Asp Val Phe Glu Lys Tyr Asn Trp
                405                 410                 415

Pro Asn Arg Thr Val Arg Val Phe Thr Phe Ser Val Gly Gln His Asn
            420                 425                 430

Tyr Asp Val Thr Pro Leu Gln Trp Met Ala Cys Ala Asn Lys Gly Tyr
        435                 440                 445

Tyr Phe Glu Ile Pro Ser Ile Gly Ala Ile Arg Ile Asn Thr Gln Glu
    450                 455                 460

Tyr Leu Asp Val Leu Gly Arg Pro Met Val Leu Ala Gly Lys Glu Ala
465                 470                 475                 480

Lys Gln Val Gln Trp Thr Asn Val Tyr Glu Asp Ala Leu Gly Leu Gly
                485                 490                 495

Leu Val Val Thr Gly Thr Leu Pro Val Phe Asn Leu Thr Gln Asp Gly
            500                 505                 510

Pro Gly Glu Lys Lys Asn Gln Leu Ile Leu Gly Val Met Gly Ile Asp
        515                 520                 525

Val Ala Leu Asn Asp Ile Lys Arg Leu Thr Pro Asn Tyr Thr Leu Gly
    530                 535                 540

Ala Asn Gly Tyr Val Phe Ala Ile Asp Leu Asn Gly Tyr Val Leu Leu
545                 550                 555                 560

His Pro Asn Leu Lys Pro Gln Thr Thr Asn Phe Arg Glu Pro Val Thr
                565                 570                 575

Leu Asp Phe Leu Asp Ala Glu Leu Glu Asp Glu Asn Lys Glu Glu Ile
            580                 585                 590

Arg Arg Ser Met Ile Asp Gly Asn Lys Gly His Lys Gln Ile Arg Thr
        595                 600                 605

Leu Val Lys Ser Leu Asp Glu Arg Tyr Ile Asp Glu Val Thr Arg Asn
    610                 615                 620

Tyr Thr Trp Val Pro Ile Arg Ser Thr Asn Tyr Ser Leu Gly Leu Val
625                 630                 635                 640

Leu Pro Pro Tyr Ser Thr Phe Tyr Leu Gln Ala Asn Leu Ser Asp Gln
                645                 650                 655

Ile Leu Gln Val Lys Leu Pro Ile Ser Lys Leu Lys Asp Phe Glu Phe
            660                 665                 670

Leu Leu Pro Ser Ser Phe Glu Ser Glu Gly His Val Phe Ile Ala Pro
        675                 680                 685

Arg Glu Tyr Cys Lys Asp Leu Asn Ala Ser Asp Asn Asn Thr Glu Phe

690                 695                 700

Leu Lys Asn Phe Ile Glu Leu Met Glu Lys Val Thr Pro Asp Ser Lys
705                 710                 715                 720

Gln Cys Asn Asn Phe Leu Leu His Asn Leu Ile Leu Asp Thr Gly Ile
                725                 730                 735

Thr Gln Gln Leu Val Glu Arg Val Trp Arg Asp Gln Asp Leu Asn Thr
            740                 745                 750

Tyr Ser Leu Leu Ala Val Phe Ala Ala Thr Asp Gly Gly Ile Thr Arg
        755                 760                 765

Val Phe Pro Asn Lys Ala Ala Glu Asp Trp Thr Glu Asn Pro Glu Pro
    770                 775                 780

Phe Asn Ala Ser Phe Tyr Arg Arg Ser Leu Asp Asn His Gly Tyr Val
785                 790                 795                 800

Phe Lys Pro Pro His Gln Asp Ala Leu Leu Arg Pro Leu Glu Leu Glu
                805                 810                 815

Asn Asp Thr Val Gly Ile Leu Val Ser Thr Ala Val Glu Leu Ser Leu
            820                 825                 830

Gly Arg Arg Thr Leu Arg Pro Ala Val Val Gly Val Lys Leu Asp Leu
        835                 840                 845

Glu Ala Trp Ala Glu Lys Phe Lys Val Leu Ala Ser Asn Arg Thr His
    850                 855                 860

Gln Asp Gln Pro Gln Lys Cys Gly Pro Asn Ser His Cys Glu Met Asp
865                 870                 875                 880

Cys Glu Val Asn Asn Glu Asp Leu Leu Cys Val Leu Ile Asp Asp Gly
                885                 890                 895

Gly Phe Leu Val Leu Ser Asn Gln Asn His Gln Trp Asp Gln Val Gly
            900                 905                 910

Arg Phe Phe Ser Glu Val Asp Ala Asn Leu Met Leu Ala Leu Tyr Asn
        915                 920                 925

Asn Ser Phe Tyr Thr Arg Lys Glu Ser Tyr Asp Tyr Gln Ala Ala Cys
    930                 935                 940

Ala Pro Gln Pro Pro Gly Asn Leu Gly Ala Ala Pro Arg Gly Val Phe
945                 950                 955                 960

Val Pro Thr Val Ala Asp Phe Leu Asn Leu Ala Trp Trp Thr Ser Ala
                965                 970                 975

Ala Ala Trp Ser Leu Phe Gln Gln Leu Leu Tyr Gly Leu Ile Tyr His
            980                 985                 990

Ser Trp Phe Gln Ala Asp Pro Ala  Glu Ala Glu Gly Ser  Pro Glu Thr
        995                 1000                 1005

Arg Glu  Ser Ser Cys Val Met  Lys Gln Thr Gln Tyr  Tyr Phe Gly
    1010                 1015                 1020

Ser Val  Asn Ala Ser Tyr Asn  Ala Ile Ile Asp Cys  Gly Asn Cys
    1025                 1030                 1035

Ser Arg  Leu Phe His Ala Gln  Arg Leu Thr Asn Thr  Asn Leu Leu
    1040                 1045                 1050

Phe Val  Val Ala Glu Lys Pro  Leu Cys Ser Gln Cys  Glu Ala Gly
    1055                 1060                 1065

Arg Leu  Leu Gln Lys Glu Thr  His Ser Asp Gly Pro  Glu Gln Cys
    1070                 1075                 1080

Glu Leu  Val Gln Arg Pro Arg  Tyr Arg Arg Gly Pro  His Ile Cys
    1085                 1090                 1095

Phe Asp  Tyr Asn Ala Thr Glu  Asp Thr Ser Asp Cys  Gly Arg Gly
    1100                 1105                 1110

Ala Ser  Phe Pro Pro Ser Leu  Gly Val Leu Val Ser  Leu Gln Leu
    1115                 1120                 1125

Leu Leu  Leu Leu Gly Leu Pro  Pro Arg Pro Gln Pro  Gln Val Leu
    1130                 1135                 1140

Val His  Ala Ser Arg Arg Leu
    1145                 1150

<210>  23
<211>  582
<212>  PRT
<213>  Homo sapiens

<400>  23

Met Met Lys Leu Arg His Lys Asn Lys Lys Pro Gly Glu Gly Ser Lys
1               5                   10                  15

Gly His Lys Lys Ile Ser Trp Pro Tyr Pro Gln Pro Ala Lys Gln Asn
            20                  25                  30

Gly Lys Lys Ala Thr Ser Lys Val Pro Ser Ala Pro His Phe Val His
        35                  40                  45

Pro Asn Asp His Ala Asn Arg Glu Ala Glu Leu Lys Lys Lys Trp Val
    50                  55                  60

Glu Glu Met Arg Glu Lys Gln Gln Ala Ala Arg Glu Gln Glu Arg Gln
65                  70                  75                  80

Lys Arg Arg Thr Ile Glu Ser Tyr Cys Gln Asp Val Leu Arg Arg Gln
                85                  90                  95

Glu Glu Phe Glu His Lys Glu Glu Val Leu Gln Glu Leu Asn Met Phe
            100                 105                 110

Pro Gln Leu Asp Asp Glu Ala Thr Arg Lys Ala Tyr Tyr Lys Glu Phe
        115                 120                 125

Arg Lys Val Val Glu Tyr Ser Asp Val Ile Leu Glu Val Leu Asp Ala
    130                 135                 140

Arg Asp Pro Leu Gly Cys Arg Cys Phe Gln Met Glu Glu Ala Val Leu
145                 150                 155                 160

Arg Ala Gln Gly Asn Lys Lys Leu Val Leu Val Leu Asn Lys Ile Asp
                165                 170                 175

Leu Val Pro Lys Glu Val Val Glu Lys Trp Leu Asp Tyr Leu Arg Asn
            180                 185                 190

Glu Leu Pro Thr Val Ala Phe Lys Ala Ser Thr Gln His Gln Val Lys
        195                 200                 205

Asn Leu Asn Arg Cys Ser Val Pro Val Asp Gln Ala Ser Glu Ser Leu
    210                 215                 220

Leu Lys Ser Lys Ala Cys Phe Gly Ala Glu Asn Leu Met Arg Val Leu
225                 230                 235                 240

Gly Asn Tyr Cys Arg Leu Gly Glu Val Arg Thr His Ile Arg Val Gly
                245                 250                 255

Val Val Gly Leu Pro Asn Val Gly Lys Ser Ser Leu Ile Asn Ser Leu
            260                 265                 270

Lys Arg Ser Arg Ala Cys Ser Val Gly Ala Val Pro Gly Ile Thr Lys
        275                 280                 285

Phe Met Gln Glu Val Tyr Leu Asp Lys Phe Ile Arg Leu Leu Asp Ala
    290                 295                 300

Pro Gly Ile Val Pro Gly Pro Asn Ser Glu Val Gly Thr Ile Leu Arg
305                 310                 315                 320

Asn Cys Val His Val Gln Lys Leu Ala Asp Pro Val Thr Pro Val Glu
                325                 330                 335

Thr Ile Leu Gln Arg Cys Asn Leu Glu Glu Ile Ser Asn Tyr Tyr Gly
            340                 345                 350

Val Ser Gly Phe Gln Thr Thr Glu His Phe Leu Thr Ala Val Ala His
        355                 360                 365

Arg Leu Gly Lys Lys Lys Lys Gly Gly Leu Tyr Ser Gln Glu Gln Ala
    370                 375                 380

Ala Lys Ala Val Leu Ala Asp Trp Val Ser Gly Lys Ile Ser Phe Tyr
385                 390                 395                 400

Ile Pro Pro Pro Ala Thr His Thr Leu Pro Thr His Leu Ser Ala Glu
                405                 410                 415

Ile Val Lys Glu Met Thr Glu Val Phe Asp Ile Glu Asp Thr Glu Gln
            420                 425                 430

Ala Asn Glu Asp Thr Met Glu Cys Leu Ala Thr Gly Glu Ser Asp Glu
        435                 440                 445

Leu Leu Gly Asp Thr Asp Pro Leu Glu Met Glu Ile Lys Leu Leu His
    450                 455                 460

Ser Pro Met Thr Lys Ile Ala Asp Ala Ile Glu Asn Lys Thr Thr Val
465                 470                 475                 480

Tyr Lys Ile Gly Asp Leu Thr Gly Tyr Cys Thr Asn Pro Asn Arg His
                485                 490                 495

Gln Met Gly Trp Ala Lys Arg Asn Val Asp His Arg Pro Lys Ser Asn
            500                 505                 510

Ser Met Val Asp Val Cys Ser Val Asp Arg Arg Ser Val Leu Gln Arg
        515                 520                 525

Ile Met Glu Thr Asp Pro Leu Gln Gln Gly Gln Ala Leu Ala Ser Ala
    530                 535                 540

Leu Lys Asn Lys Lys Lys Met Gln Lys Arg Ala Asp Lys Ile Ala Ser
545                 550                 555                 560

Lys Leu Ser Asp Ser Met Met Ser Ala Leu Asp Leu Ser Gly Asn Ala
                565                 570                 575

Asp Asp Gly Val Gly Asp
            580

<210>  24
<211>  1487
<212>  PRT
<213>  Homo sapiens

<400>  24

<210>  25
<211>  330
<212>  PRT
<213>  Homo sapiens

<400>  25

Met Pro His Ser Val Thr Leu Arg Gly Pro Ser Pro Trp Gly Phe Arg
1               5                   10                  15

Leu Val Gly Gly Arg Asp Phe Ser Ala Pro Leu Thr Ile Ser Arg Val
            20                  25                  30

His Ala Gly Ser Lys Ala Ala Leu Ala Ala Leu Cys Pro Gly Asp Leu
        35                  40                  45

Ile Gln Ala Ile Asn Gly Glu Ser Thr Glu Leu Met Thr His Leu Glu
    50                  55                  60

Ala Gln Asn Arg Ile Lys Gly Cys His Asp His Leu Thr Leu Ser Val
65                  70                  75                  80

Ser Arg Pro Glu Gly Arg Ser Trp Pro Ser Ala Pro Asp Asp Ser Lys
                85                  90                  95

Ala Gln Ala His Arg Ile His Ile Asp Pro Glu Ile Gln Asp Gly Ser

            100                 105                 110

Pro Thr Thr Ser Arg Arg Pro Ser Gly Thr Gly Thr Gly Pro Glu Asp
        115                 120                 125

Gly Arg Pro Ser Leu Gly Ser Pro Tyr Gly Gln Pro Pro Arg Phe Pro
    130                 135                 140

Val Pro His Asn Gly Ser Ser Glu Ala Thr Leu Pro Ala Gln Met Ser
145                 150                 155                 160

Thr Leu His Val Ser Pro Pro Pro Ser Ala Asp Pro Ala Arg Gly Leu
                165                 170                 175

Pro Arg Ser Arg Asp Cys Arg Val Asp Leu Gly Ser Glu Val Tyr Arg
            180                 185                 190

Met Leu Arg Glu Pro Ala Glu Pro Val Ala Ala Glu Pro Lys Gln Ser
        195                 200                 205

Gly Ser Phe Arg Tyr Leu Gln Gly Met Leu Glu Ala Gly Glu Gly Gly
    210                 215                 220

Asp Trp Pro Gly Pro Gly Gly Pro Arg Asn Leu Lys Pro Thr Ala Ser
225                 230                 235                 240

Lys Leu Gly Ala Pro Leu Ser Gly Leu Gln Gly Leu Pro Glu Cys Thr
                245                 250                 255

Arg Cys Gly His Gly Ile Val Gly Thr Ile Val Lys Ala Arg Asp Lys
            260                 265                 270

Leu Tyr His Pro Glu Cys Phe Met Cys Ser Asp Cys Gly Leu Asn Leu
        275                 280                 285

Lys Gln Arg Gly Tyr Phe Phe Leu Asp Glu Arg Leu Tyr Cys Glu Ser
    290                 295                 300

His Ala Lys Ala Arg Val Lys Pro Pro Glu Gly Tyr Asp Val Val Ala
305                 310                 315                 320

Val Tyr Pro Asn Ala Lys Val Glu Leu Val
                325                 330

<210> 26
<211> 708
<212> PRT
<213> Homo sapiens

<400>  26

Met Arg Glu Arg Ile Trp Ala Pro Pro Leu Leu Leu Leu Leu Pro Leu
1               5                   10                  15

Leu Leu Pro Pro Pro Leu Trp Gly Gly Pro Pro Asp Ser Pro Arg Arg
            20                  25                  30

Glu Leu Glu Leu Glu Pro Gly Pro Leu Gln Pro Phe Asp Leu Leu Tyr
        35                  40                  45

Ala Ser Gly Ala Ala Ala Tyr Tyr Ser Gly Asp Tyr Glu Arg Ala Val
    50                  55                  60

Arg Asp Leu Glu Ala Ala Leu Arg Ser His Arg Arg Leu Arg Glu Ile
65                  70                  75                  80

Arg Thr Arg Cys Ala Arg His Cys Ala Ala Arg His Pro Leu Pro Pro
                85                  90                  95

Pro Pro Pro Gly Glu Gly Pro Gly Ala Glu Leu Pro Leu Phe Arg Ser
            100                 105                 110

Leu Leu Gly Arg Ala Arg Cys Tyr Arg Ser Cys Glu Thr Gln Arg Leu
        115                 120                 125

Gly Gly Pro Ala Ser Arg His Arg Val Ser Glu Asp Val Arg Ser Asp
    130                 135                 140

Phe Gln Arg Arg Val Pro Tyr Asn Tyr Leu Gln Arg Ala Tyr Ile Lys
145                 150                 155                 160

Leu Asn Gln Leu Glu Lys Ala Val Glu Ala Ala His Thr Phe Phe Val
                165                 170                 175

Ala Asn Pro Glu His Met Glu Met Gln Gln Asn Ile Glu Asn Tyr Arg
            180                 185                 190

Ala Thr Ala Gly Val Glu Ala Leu Gln Leu Val Asp Arg Glu Ala Lys
        195                 200                 205

Pro His Met Glu Ser Tyr Asn Ala Gly Val Lys His Tyr Glu Ala Asp
    210                 215                 220

Asp Phe Glu Met Ala Ile Arg His Phe Glu Gln Ala Leu Arg Glu Tyr
225                 230                 235                 240

Phe Val Glu Asp Thr Glu Cys Arg Thr Leu Cys Glu Gly Pro Gln Arg
                245                 250                 255

Phe Glu Glu Tyr Glu Tyr Leu Gly Tyr Lys Ala Gly Leu Tyr Glu Ala
            260                 265                 270

Ile Ala Asp His Tyr Met Gln Val Leu Val Cys Gln His Glu Cys Val
        275                 280                 285

Arg Glu Leu Ala Thr Arg Pro Gly Arg Leu Ser Pro Ile Glu Asn Phe
    290                 295                 300

Leu Pro Leu His Tyr Asp Tyr Leu Gln Phe Ala Tyr Tyr Arg Val Gly
305                 310                 315                 320

Glu Tyr Val Lys Ala Leu Glu Cys Ala Lys Ala Tyr Leu Leu Cys His
                325                 330                 335

Pro Asp Asp Glu Asp Val Leu Asp Asn Val Asp Tyr Tyr Glu Ser Leu
            340                 345                 350

Leu Asp Asp Ser Ile Asp Pro Ala Ser Ile Glu Ala Arg Glu Asp Leu
        355                 360                 365

Thr Met Phe Val Lys Arg His Lys Leu Glu Ser Glu Leu Ile Lys Ser
    370                 375                 380

Ala Ala Glu Gly Leu Gly Phe Ser Tyr Thr Glu Pro Asn Tyr Trp Ile
385                 390                 395                 400

Arg Tyr Gly Gly Arg Gln Asp Glu Asn Arg Val Pro Ser Gly Val Asn
                405                 410                 415

Val Glu Gly Ala Glu Val His Gly Phe Ser Met Gly Lys Lys Leu Ser
            420                 425                 430

Pro Lys Ile Asp Arg Asp Leu Arg Glu Gly Gly Pro Leu Leu Tyr Glu
        435                 440                 445

Asn Ile Thr Phe Val Tyr Asn Ser Glu Gln Leu Asn Gly Thr Gln Arg
    450                 455                 460

Val Leu Leu Asp Asn Val Leu Ser Glu Glu Gln Cys Arg Glu Leu His
465                 470                 475                 480

Ser Val Ala Ser Gly Ile Met Leu Val Gly Asp Gly Tyr Arg Gly Lys
                485                 490                 495

Thr Ser Pro His Thr Pro Asn Glu Lys Phe Glu Gly Ala Thr Val Leu
                500                 505                 510

Lys Ala Leu Lys Ser Gly Tyr Glu Gly Arg Val Pro Leu Lys Ser Ala
            515                 520                 525

Arg Leu Phe Tyr Asp Ile Ser Glu Lys Ala Arg Arg Ile Val Glu Ser
    530                 535                 540

Tyr Phe Met Leu Asn Ser Thr Leu Tyr Phe Ser Tyr Thr His Met Val
545                 550                 555                 560

Cys Arg Thr Ala Leu Ser Gly Gln Gln Asp Arg Arg Asn Asp Leu Ser
                565                 570                 575

His Pro Ile His Ala Asp Asn Cys Leu Leu Asp Pro Glu Ala Asn Glu
            580                 585                 590

Cys Trp Lys Glu Pro Pro Ala Tyr Thr Phe Arg Asp Tyr Ser Ala Leu
        595                 600                 605

Leu Tyr Met Asn Asp Asp Phe Glu Gly Gly Glu Phe Ile Phe Thr Glu
    610                 615                 620

Met Asp Ala Lys Thr Val Thr Ala Ser Ile Lys Pro Lys Cys Gly Arg
625                 630                 635                 640

Met Ile Ser Phe Ser Ser Gly Gly Glu Asn Pro His Gly Val Lys Ala
                645                 650                 655

Val Thr Lys Gly Lys Arg Cys Ala Val Ala Leu Trp Phe Thr Leu Asp
            660                 665                 670

Pro Leu Tyr Arg Glu Leu Glu Arg Ile Gln Ala Asp Glu Val Ile Ala
        675                 680                 685

Ile Leu Asp Gln Glu Gln Gln Gly Lys His Glu Leu Asn Ile Asn Pro
    690                 695                 700

Lys Asp Glu Leu
705

<210>  27
<211>  421
<212>  PRT
<213>  Homo sapiens

<400>  27

Met Gly Phe Leu Ser Pro Ile Tyr Val Ile Phe Phe Phe Phe Gly Val
1               5                   10                  15

Lys Val His Cys Gln Tyr Glu Thr Tyr Gln Trp Asp Glu Asp Tyr Asp
            20                  25                  30

Gln Glu Pro Asp Asp Asp Tyr Gln Thr Gly Phe Pro Phe Arg Gln Asn
        35                  40                  45

Val Asp Tyr Gly Val Pro Phe His Gln Tyr Thr Leu Gly Cys Val Ser
    50                  55                  60

Glu Cys Phe Cys Pro Thr Asn Phe Pro Ser Ser Met Tyr Cys Asp Asn
65                  70                  75                  80

Arg Lys Leu Lys Thr Ile Pro Asn Ile Pro Met His Ile Gln Gln Leu
                85                  90                  95

Tyr Leu Gln Phe Asn Glu Ile Glu Ala Val Thr Ala Asn Ser Phe Ile
            100                 105                 110

Asn Ala Thr His Leu Lys Glu Ile Asn Leu Ser His Asn Lys Ile Lys
        115                 120                 125

Ser Gln Lys Ile Asp Tyr Gly Val Phe Ala Lys Leu Pro Asn Leu Leu
    130                 135                 140

Gln Leu His Leu Glu His Asn Asn Leu Glu Glu Phe Pro Phe Pro Leu
145                 150                 155                 160

Pro Lys Ser Leu Glu Arg Leu Leu Leu Gly Tyr Asn Glu Ile Ser Lys
                165                 170                 175

Leu Gln Thr Asn Ala Met Asp Gly Leu Val Asn Leu Thr Met Leu Asp
            180                 185                 190

Leu Cys Tyr Asn Tyr Leu His Asp Ser Leu Leu Lys Asp Lys Ile Phe
        195                 200                 205

Ala Lys Met Glu Lys Leu Met Gln Leu Asn Leu Cys Ser Asn Arg Leu
    210                 215                 220

Glu Ser Met Pro Pro Gly Leu Pro Ser Ser Leu Met Tyr Leu Ser Leu
225                 230                 235                 240

Glu Asn Asn Ser Ile Ser Ser Ile Pro Glu Lys Tyr Phe Asp Lys Leu
                245                 250                 255

Pro Lys Leu His Thr Leu Arg Met Ser His Asn Lys Leu Gln Asp Ile
            260                 265                 270

Pro Tyr Asn Ile Phe Asn Leu Pro Asn Ile Val Glu Leu Ser Val Gly
        275                 280                 285

His Asn Lys Leu Lys Gln Ala Phe Tyr Ile Pro Arg Asn Leu Glu His
    290                 295                 300

Leu Tyr Leu Gln Asn Asn Glu Ile Glu Lys Met Asn Leu Thr Val Met
305                 310                 315                 320

Cys Pro Ser Ile Asp Pro Leu His Tyr His His Leu Thr Tyr Ile Arg
                325                 330                 335

Val Asp Gln Asn Lys Leu Lys Glu Pro Ile Ser Ser Tyr Ile Phe Phe
            340                 345                 350

Cys Phe Pro His Ile His Thr Ile Tyr Tyr Gly Glu Gln Arg Ser Thr
        355                 360                 365

Asn Gly Gln Thr Ile Gln Leu Lys Thr Gln Val Phe Arg Arg Phe Pro
    370                 375                 380

Asp Asp Asp Asp Glu Ser Glu Asp His Asp Asp Pro Asp Asn Ala His
385                 390                 395                 400

Glu Ser Pro Glu Gln Glu Gly Ala Glu Gly His Phe Asp Leu His Tyr
                405                 410                 415

Tyr Glu Asn Gln Glu
            420

<210> 28
<211> 1117
<212> PRT
<213> Homo sapiens

<400> 28

Met Glu Thr Phe Phe Arg Arg His Phe Arg Gly Lys Val Pro Gly Pro
1               5                   10                  15

Gly Glu Gly Gln Gln Arg Pro Ser Ser Val Gly Leu Pro Thr Gly Lys
            20                  25                  30

Ala Arg Arg Arg Ser Pro Ala Gly Gln Ala Ser Ser Ser Leu Ala Gln
        35                  40                  45

Arg Arg Arg Ser Ser Ala Gln Leu Gln Gly Cys Leu Leu Ser Cys Gly
    50                  55                  60

Val Arg Ala Gln Gly Ser Ser Arg Arg Arg Ser Ser Thr Val Pro Pro
65                  70                  75                  80

Ser Cys Asn Pro Arg Phe Ile Val Asp Lys Val Leu Thr Pro Gln Pro
                85                  90                  95

Thr Thr Val Gly Ala Gln Leu Leu Gly Ala Pro Leu Leu Leu Thr Gly
            100                 105                 110

Leu Val Gly Met Asn Glu Glu Glu Gly Val Gln Glu Asp Val Val Ala
        115                 120                 125

Glu Ala Ser Ser Ala Ile Gln Pro Gly Thr Lys Thr Pro Gly Pro Pro
    130                 135                 140

Pro Pro Arg Gly Ala Gln Pro Leu Leu Pro Leu Pro Arg Tyr Leu Arg
145                 150                 155                 160

Arg Ala Ser Ser His Leu Leu Pro Ala Asp Ala Val Tyr Asp His Ala
                165                 170                 175

Leu Trp Gly Leu His Gly Tyr Tyr Arg Arg Leu Ser Gln Arg Arg Pro
            180                 185                 190

Ser Gly Gln His Pro Gly Pro Gly Gly Arg Arg Ala Ser Gly Thr Thr
        195                 200                 205

Ala Gly Thr Met Leu Pro Thr Arg Val Arg Pro Leu Ser Arg Arg Arg
    210                 215                 220

Gln Val Ala Leu Arg Arg Lys Ala Ala Gly Pro Gln Ala Trp Ser Ala
225                 230                 235                 240

Leu Leu Ala Lys Ala Ile Thr Lys Ser Gly Leu Gln His Leu Ala Pro
                245                 250                 255

Pro Pro Pro Thr Pro Gly Ala Pro Cys Ser Glu Ser Glu Arg Gln Ile
            260                 265                 270

Arg Ser Thr Val Asp Trp Ser Glu Ser Ala Thr Tyr Gly Glu His Ile
        275                 280                 285

Trp Phe Glu Thr Asn Val Ser Gly Asp Phe Cys Tyr Val Gly Glu Gln

290                 295                 300

Tyr Cys Val Ala Arg Met Leu Lys Ser Val Ser Arg Arg Lys Cys Ala
305                 310                 315                 320

Ala Cys Lys Ile Val Val His Thr Pro Cys Ile Glu Gln Leu Glu Lys
                325                 330                 335

Ile Asn Phe Arg Cys Lys Pro Ser Phe Arg Glu Ser Gly Ser Arg Asn
            340                 345                 350

Val Arg Glu Pro Thr Phe Val Arg His His Trp Val His Arg Arg Arg
        355                 360                 365

Gln Asp Gly Lys Cys Arg His Cys Gly Lys Gly Phe Gln Gln Lys Phe
    370                 375                 380

Thr Phe His Ser Lys Glu Ile Val Ala Ile Ser Cys Ser Trp Cys Lys
385                 390                 395                 400

Gln Ala Tyr His Ser Lys Val Ser Cys Phe Met Leu Gln Gln Ile Glu
                405                 410                 415

Glu Pro Cys Ser Leu Gly Val His Ala Ala Val Val Ile Pro Pro Thr
            420                 425                 430

Trp Ile Leu Arg Ala Arg Arg Pro Gln Asn Thr Leu Lys Ala Ser Lys
        435                 440                 445

Lys Lys Lys Arg Ala Ser Phe Lys Arg Lys Ser Ser Lys Lys Gly Pro
    450                 455                 460

Glu Glu Gly Arg Trp Arg Pro Phe Ile Ile Arg Pro Thr Pro Ser Pro
465                 470                 475                 480

Leu Met Lys Pro Leu Leu Val Phe Val Asn Pro Lys Ser Gly Gly Asn
                485                 490                 495

Gln Gly Ala Lys Ile Ile Gln Ser Phe Leu Trp Tyr Leu Asn Pro Arg
            500                 505                 510

Gln Val Phe Asp Leu Ser Gln Gly Gly Pro Lys Glu Ala Leu Glu Met
        515                 520                 525

Tyr Arg Lys Val His Asn Leu Arg Ile Leu Ala Cys Gly Gly Asp Gly
    530                 535                 540

Thr Val Gly Trp Ile Leu Ser Thr Leu Asp Gln Leu Arg Leu Lys Pro
545                 550                 555                 560

Pro Pro Pro Val Ala Ile Leu Pro Leu Gly Thr Gly Asn Asp Leu Ala
                565                 570                 575

Arg Thr Leu Asn Trp Gly Gly Gly Tyr Thr Asp Glu Pro Val Ser Lys
            580                 585                 590

Ile Leu Ser His Val Glu Glu Gly Asn Val Val Gln Leu Asp Arg Trp
        595                 600                 605

Asp Leu His Ala Glu Pro Asn Pro Glu Ala Gly Pro Glu Asp Arg Asp
    610                 615                 620

Glu Gly Ala Thr Asp Arg Leu Pro Leu Asp Val Phe Asn Asn Tyr Phe
625                 630                 635                 640

Ser Leu Gly Phe Asp Ala His Val Thr Leu Glu Phe His Glu Ser Arg
                645                 650                 655

Glu Ala Asn Pro Glu Lys Phe Asn Ser Arg Phe Arg Asn Lys Met Phe
            660                 665                 670

Tyr Ala Gly Thr Ala Phe Ser Asp Phe Leu Met Gly Ser Ser Lys Asp
        675                 680                 685

Leu Ala Lys His Ile Arg Val Val Cys Asp Gly Met Asp Leu Thr Pro
    690                 695                 700

Lys Ile Gln Asp Leu Lys Pro Gln Cys Val Val Phe Leu Asn Ile Pro
705                 710                 715                 720

Arg Tyr Cys Ala Gly Thr Met Pro Trp Gly His Pro Gly Glu His His
                725                 730                 735

Asp Phe Glu Pro Gln Arg His Asp Asp Gly Tyr Leu Glu Val Ile Gly
            740                 745                 750

Phe Thr Met Thr Ser Leu Ala Ala Leu Gln Val Gly Gly His Gly Glu
        755                 760                 765

Arg Leu Thr Gln Cys Arg Glu Val Val Leu Thr Thr Ser Lys Ala Ile
    770                 775                 780

Pro Val Gln Val Asp Gly Glu Pro Cys Lys Leu Ala Ala Ser Arg Ile
785                 790                 795                 800

Arg Ile Ala Leu Arg Asn Gln Ala Thr Met Val Gln Lys Ala Lys Arg
                805                 810                 815

Arg Ser Ala Ala Pro Leu His Ser Asp Gln Gln Pro Val Pro Glu Gln
            820                 825                 830

Leu Arg Ile Gln Val Ser Arg Val Ser Met His Asp Tyr Glu Ala Leu
        835                 840                 845

His Tyr Asp Lys Glu Gln Leu Lys Glu Ala Ser Val Pro Leu Gly Thr
    850                 855                 860

Val Val Val Pro Gly Asp Ser Asp Leu Glu Leu Cys Arg Ala His Ile
865                 870                 875                 880

Glu Arg Leu Gln Gln Glu Pro Asp Gly Ala Gly Ala Lys Ser Pro Thr
                885                 890                 895

Cys Gln Lys Leu Ser Pro Lys Trp Cys Phe Leu Asp Ala Thr Thr Ala
            900                 905                 910

Ser Arg Phe Tyr Arg Ile Asp Arg Ala Gln Glu His Leu Asn Tyr Val
        915                 920                 925

Thr Glu Ile Ala Gln Asp Glu Ile Tyr Ile Leu Asp Pro Glu Leu Leu
    930                 935                 940

Gly Ala Ser Ala Arg Pro Asp Leu Pro Thr Pro Thr Ser Pro Leu Pro
945                 950                 955                 960

Thr Ser Pro Cys Ser Pro Thr Pro Arg Ser Leu Gln Gly Asp Ala Ala
                965                 970                 975

Pro Pro Gln Gly Glu Glu Leu Ile Glu Ala Ala Lys Arg Asn Asp Phe
            980                 985                 990

Cys Lys Leu Gln Glu Leu His Arg  Ala Gly Gly Asp Leu  Met His Arg
        995                 1000                1005

Asp Glu  Gln Ser Arg Thr Leu  Leu His His Ala Val  Ser Thr Gly
    1010                1015                1020

Ser Lys  Asp Val Val Arg Tyr  Leu Leu Asp His Ala  Pro Pro Glu
    1025                1030                1035

Ile Leu  Asp Ala Val Glu Glu  Asn Gly Glu Thr Cys  Leu His Gln
    1040                1045                1050

Ala Ala Ala Leu Gly Gln Arg Thr Ile Cys His Tyr Ile Val Glu
    1055                1060                1065

Ala Gly Ala Ser Leu Met Lys Thr Asp Gln Gln Gly Asp Thr Pro
    1070                1075                1080

Arg Gln Arg Ala Glu Lys Ala Gln Asp Thr Glu Leu Ala Ala Tyr
    1085                1090                1095

Leu Glu Asn Arg Gln His Tyr Gln Met Ile Gln Arg Glu Asp Gln
    1100                1105                1110

Glu Thr Ala Val
    1115

<210> 29
<211> 520
<212> PRT
<213> Homo sapiens

<400> 29

Met Arg Val Glu Ser Gly Ser Ala Gln Glu Arg Gly Ile Leu Leu Glu
1               5                   10                  15

Ser Leu Ser Thr Leu Leu Glu Lys Thr Thr Ala Ser His Glu Gly Arg
            20                  25                  30

Ala Pro Gly Asn Arg Glu Leu Thr Asp Leu Leu Pro Pro Glu Val Cys
        35                  40                  45

Ser Leu Leu Asn Pro Ala Ala Ile Tyr Ala Asn Asn Glu Ile Ser Leu
    50                  55                  60

Arg Asp Val Glu Val Tyr Gly Phe Asp Tyr Asp Tyr Thr Leu Ala Gln
65                  70                  75                  80

Tyr Ala Asp Ala Leu His Pro Glu Ile Phe Ser Thr Ala Arg Asp Ile
                85                  90                  95

Leu Ile Glu His Tyr Lys Tyr Pro Glu Gly Ile Arg Lys Tyr Asp Tyr
            100                 105                 110

Asn Pro Ser Phe Ala Ile Arg Gly Leu His Tyr Asp Ile Gln Lys Ser
        115                 120                 125

Leu Leu Met Lys Ile Asp Ala Phe His Tyr Val Gln Leu Gly Thr Ala
    130                 135                 140

Tyr Arg Gly Leu Gln Pro Val Pro Asp Glu Glu Val Ile Glu Leu Tyr
145                 150                 155                 160

Gly Gly Thr Gln His Ile Pro Leu Tyr Gln Met Ser Gly Phe Tyr Gly
                165                 170                 175

Lys Gly Pro Ser Ile Lys Gln Phe Met Asp Ile Phe Ser Leu Pro Glu
            180                 185                 190

Met Ala Leu Leu Ser Cys Val Val Asp Tyr Phe Leu Gly His Ser Leu
        195                 200                 205

Glu Phe Asp Gln Ala His Leu Tyr Lys Asp Val Thr Asp Ala Ile Arg
    210                 215                 220

Asp Val His Val Lys Gly Leu Met Tyr Gln Trp Ile Glu Gln Asp Met
225                 230                 235                 240

Glu Lys Tyr Ile Leu Arg Gly Asp Glu Thr Phe Ala Val Leu Ser Arg
                245                 250                 255

Leu Val Ala His Gly Lys Gln Leu Phe Leu Ile Thr Asn Ser Pro Phe
            260                 265                 270

Ser Phe Val Asp Lys Gly Met Arg His Met Val Gly Pro Asp Trp Arg
        275                 280                 285

Gln Leu Phe Asp Val Val Ile Val Gln Ala Asp Lys Pro Ser Phe Phe
    290                 295                 300

Thr Asp Arg Arg Lys Pro Phe Arg Lys Leu Asp Glu Lys Gly Ser Leu
305                 310                 315                 320

Gln Trp Asp Arg Ile Thr Arg Leu Glu Lys Gly Lys Ile Tyr Arg Gln
                325                 330                 335

Gly Asn Leu Phe Asp Phe Leu Arg Leu Thr Glu Trp Arg Gly Pro Arg
            340                 345                 350

Val Leu Tyr Phe Gly Asp His Leu Tyr Ser Asp Leu Ala Asp Leu Met
        355                 360                 365

Leu Arg His Gly Trp Arg Thr Gly Ala Ile Ile Pro Glu Leu Glu Arg
    370                 375                 380

Glu Ile Arg Ile Ile Asn Thr Glu Gln Tyr Met His Ser Leu Thr Trp

385                 390                 395                 400

Gln Gln Ala Leu Thr Gly Leu Leu Glu Arg Met Gln Thr Tyr Gln Asp
                405                 410                 415

Ala Glu Ser Arg Gln Val Leu Ala Ala Trp Met Lys Glu Arg Gln Glu
            420                 425                 430

Leu Arg Cys Ile Thr Lys Ala Leu Phe Asn Ala Gln Phe Gly Ser Ile
        435                 440                 445

Phe Arg Thr Phe His Asn Pro Thr Tyr Phe Ser Arg Arg Leu Val Arg
    450                 455                 460

Phe Ser Asp Leu Tyr Met Ala Ser Leu Ser Cys Leu Leu Asn Tyr Arg
465                 470                 475                 480

Val Asp Phe Thr Phe Tyr Pro Arg Arg Thr Pro Leu Gln His Glu Ala
                485                 490                 495

Pro Leu Trp Met Asp Gln Leu Cys Thr Gly Cys Met Lys Thr Pro Phe
            500                 505                 510

Leu Gly Asp Met Ala His Ile Arg
        515                 520

<210>  30
<211>  313
<212>  PRT
<213>  Homo sapiens

<400>  30

Met Asn Gln Leu Ser Phe Leu Leu Phe Leu Ile Ala Thr Thr Arg Gly
1               5                   10                  15

Trp Ser Thr Asp Glu Ala Asn Thr Tyr Phe Lys Glu Trp Thr Cys Ser
            20                  25                  30

Ser Ser Pro Ser Leu Pro Arg Ser Cys Lys Glu Ile Lys Asp Glu Cys
        35                  40                  45

Pro Ser Ala Phe Asp Gly Leu Tyr Phe Leu Arg Thr Glu Asn Gly Val
    50                  55                  60

Ile Tyr Gln Thr Phe Cys Asp Met Thr Ser Gly Gly Gly Gly Trp Thr
65                  70                  75                  80

Leu Val Ala Ser Val His Glu Asn Asp Met Arg Gly Lys Cys Thr Val

85                  90                  95

Gly Asp Arg Trp Ser Ser Gln Gln Gly Ser Lys Ala Val Tyr Pro Glu
            100                 105                 110

Gly Asp Gly Asn Trp Ala Asn Tyr Asn Thr Phe Gly Ser Ala Glu Ala
        115                 120                 125

Ala Thr Ser Asp Asp Tyr Lys Asn Pro Gly Tyr Tyr Asp Ile Gln Ala
    130                 135                 140

Lys Asp Leu Gly Ile Trp His Val Pro Asn Lys Ser Pro Met Gln His
145                 150                 155                 160

Trp Arg Asn Ser Ser Leu Leu Arg Tyr Arg Thr Asp Thr Gly Phe Leu
                165                 170                 175

Gln Thr Leu Gly His Asn Leu Phe Gly Ile Tyr Gln Lys Tyr Pro Val
            180                 185                 190

Lys Tyr Gly Glu Gly Lys Cys Trp Thr Asp Asn Gly Pro Val Ile Pro
        195                 200                 205

Val Val Tyr Asp Phe Gly Asp Ala Gln Lys Thr Ala Ser Tyr Tyr Ser
    210                 215                 220

Pro Tyr Gly Gln Arg Glu Phe Thr Ala Gly Phe Val Gln Phe Arg Val
225                 230                 235                 240

Phe Asn Asn Glu Arg Ala Ala Asn Ala Leu Cys Ala Gly Met Arg Val
                245                 250                 255

Thr Gly Cys Asn Thr Glu His His Cys Ile Gly Gly Gly Gly Tyr Phe
            260                 265                 270

Pro Glu Ala Ser Pro Gln Gln Cys Gly Asp Phe Ser Gly Phe Asp Trp
        275                 280                 285

Ser Gly Tyr Gly Thr His Val Gly Tyr Ser Ser Ser Arg Glu Ile Thr
    290                 295                 300

Glu Ala Ala Val Leu Leu Phe Tyr Arg
305                 310

<210>  31
<211>  344
<212>  PRT
<213>  Homo sapiens

<400>  31

Met Gly Val Cys Gly Tyr Leu Phe Leu Pro Trp Lys Cys Leu Val Val
1               5                   10                  15

Val Ser Leu Arg Leu Leu Phe Leu Val Pro Thr Gly Val Pro Val Arg
            20                  25                  30

Ser Gly Asp Ala Thr Phe Pro Lys Ala Met Asp Asn Val Thr Val Arg
        35                  40                  45

Gln Gly Glu Ser Ala Thr Leu Arg Cys Thr Ile Asp Asn Arg Val Thr
    50                  55                  60

Arg Val Ala Trp Leu Asn Arg Ser Thr Ile Leu Tyr Ala Gly Asn Asp
65                  70                  75                  80

Lys Trp Cys Leu Asp Pro Arg Val Val Leu Leu Ser Asn Thr Gln Thr
                85                  90                  95

Gln Tyr Ser Ile Glu Ile Gln Asn Val Asp Val Tyr Asp Glu Gly Pro
            100                 105                 110

Tyr Thr Cys Ser Val Gln Thr Asp Asn His Pro Lys Thr Ser Arg Val
        115                 120                 125

His Leu Ile Val Gln Val Ser Pro Lys Ile Val Glu Ile Ser Ser Asp
    130                 135                 140

Ile Ser Ile Asn Glu Gly Asn Asn Ile Ser Leu Thr Cys Ile Ala Thr
145                 150                 155                 160

Gly Arg Pro Glu Pro Thr Val Thr Trp Arg His Ile Ser Pro Lys Ala
                165                 170                 175

Val Gly Phe Val Ser Glu Asp Glu Tyr Leu Glu Ile Gln Gly Ile Thr
            180                 185                 190

Arg Glu Gln Ser Gly Asp Tyr Glu Cys Ser Ala Ser Asn Asp Val Ala
        195                 200                 205

Ala Pro Val Val Arg Arg Val Lys Val Thr Val Asn Tyr Pro Pro Tyr
    210                 215                 220

Ile Ser Glu Ala Lys Gly Thr Gly Val Pro Val Gly Gln Lys Gly Thr
225                 230                 235                 240

Leu Gln Cys Glu Ala Ser Ala Val Pro Ser Ala Glu Phe Gln Trp Tyr
                245                 250                 255

Lys Asp Asp Lys Arg Leu Ile Glu Gly Lys Lys Gly Val Lys Val Glu
            260                 265                 270

Asn Arg Pro Phe Leu Ser Lys Leu Ile Phe Phe Asn Val Ser Glu His
        275                 280                 285

Asp Tyr Gly Asn Tyr Thr Cys Val Ala Ser Asn Lys Leu Gly His Thr
    290                 295                 300

Asn Ala Ser Ile Met Leu Phe Gly Pro Gly Ala Val Ser Glu Val Ser
305                 310                 315                 320

Asn Gly Thr Ser Arg Arg Ala Gly Cys Val Trp Leu Leu Pro Leu Leu
                325                 330                 335

Val Leu His Leu Leu Leu Lys Phe
            340

<210>  32
<211>  762
<212>  PRT
<213>  Homo sapiens

<400>  32

Met Gly Asn Gly Ser Val Lys Pro Lys His Ser Lys His Pro Asp Gly
1               5                   10                  15

His Ser Gly Asn Leu Thr Thr Asp Ala Leu Arg Asn Lys Val Thr Glu
            20                  25                  30

Leu Glu Arg Glu Leu Arg Arg Lys Asp Ala Glu Ile Gln Glu Arg Glu
        35                  40                  45

Tyr His Leu Lys Glu Leu Arg Glu Gln Leu Ser Lys Gln Thr Val Ala
    50                  55                  60

Ile Ala Glu Leu Thr Glu Glu Leu Gln Asn Lys Cys Ile Gln Leu Asn
65                  70                  75                  80

Lys Leu Gln Asp Val Val His Met Gln Gly Gly Ser Pro Leu Gln Ala
                85                  90                  95

Ser Pro Asp Lys Val Pro Leu Glu Val His Arg Lys Thr Ser Gly Leu
            100                 105                 110

Val Ser Leu His Ser Arg Arg Gly Ala Lys Ala Gly Val Ser Ala Glu
        115                 120                 125

Pro Thr Thr Arg Thr Tyr Asp Leu Asn Lys Pro Pro Glu Phe Ser Phe
    130                 135                 140

Glu Lys Ala Arg Val Arg Lys Asp Ser Ser Glu Lys Lys Leu Ile Thr
145                 150                 155                 160

Asp Ala Leu Asn Lys Asn Gln Phe Leu Lys Arg Leu Asp Pro Gln Gln
                165                 170                 175

Ile Lys Asp Met Val Glu Cys Met Tyr Gly Arg Asn Tyr Gln Gln Gly
            180                 185                 190

Ser Tyr Ile Ile Lys Gln Gly Glu Pro Gly Asn His Ile Phe Val Leu
        195                 200                 205

Ala Glu Gly Arg Leu Glu Val Phe Gln Gly Glu Lys Leu Leu Ser Ser
    210                 215                 220

Ile Pro Met Trp Thr Thr Phe Gly Glu Leu Ala Ile Leu Tyr Asn Cys
225                 230                 235                 240

Thr Arg Thr Ala Ser Val Lys Ala Ile Thr Asn Val Lys Thr Trp Ala
                245                 250                 255

Leu Asp Arg Glu Val Phe Gln Asn Ile Met Arg Arg Thr Ala Gln Ala
            260                 265                 270

Arg Asp Glu Gln Tyr Arg Asn Phe Leu Arg Ser Val Ser Leu Leu Lys
        275                 280                 285

Asn Leu Pro Glu Asp Lys Leu Thr Lys Ile Ile Asp Cys Leu Glu Val
    290                 295                 300

Glu Tyr Tyr Asp Lys Gly Asp Tyr Ile Ile Arg Glu Gly Glu Glu Gly
305                 310                 315                 320

Ser Thr Phe Phe Ile Leu Ala Lys Gly Lys Val Lys Val Thr Gln Ser
                325                 330                 335

Thr Glu Gly His Asp Gln Pro Gln Leu Ile Lys Thr Leu Gln Lys Gly
            340                 345                 350

Glu Tyr Phe Gly Glu Lys Ala Leu Ile Ser Asp Asp Val Arg Ser Ala
        355                 360                 365

Asn Ile Ile Ala Glu Glu Asn Asp Val Ala Cys Leu Val Ile Asp Arg
    370                 375                 380

Glu Thr Phe Asn Gln Thr Val Gly Thr Phe Glu Glu Leu Gln Lys Tyr
385                 390                 395                 400

Leu Glu Gly Tyr Val Ala Asn Leu Asn Arg Asp Asp Glu Lys Arg His
                405                 410                 415

Ala Lys Arg Ser Met Ser Asn Trp Lys Leu Ser Lys Ala Leu Ser Leu
            420                 425                 430

Glu Met Ile Gln Leu Lys Glu Lys Val Ala Arg Phe Ser Ser Ser Ser
        435                 440                 445

Pro Phe Gln Asn Leu Glu Ile Ile Ala Thr Leu Gly Val Gly Gly Phe
    450                 455                 460

Gly Arg Val Glu Leu Val Lys Val Lys Asn Glu Asn Val Ala Phe Ala
465                 470                 475                 480

Met Lys Cys Ile Arg Lys Lys His Ile Val Asp Thr Lys Gln Gln Glu
                485                 490                 495

His Val Tyr Ser Glu Lys Arg Ile Leu Glu Glu Leu Cys Ser Pro Phe
            500                 505                 510

Ile Val Lys Leu Tyr Arg Thr Phe Lys Asp Asn Lys Tyr Val Tyr Met
        515                 520                 525

Leu Leu Glu Ala Cys Leu Gly Gly Glu Leu Trp Ser Ile Leu Arg Asp
    530                 535                 540

Arg Gly Ser Phe Asp Glu Pro Thr Ser Lys Phe Cys Val Ala Cys Val
545                 550                 555                 560

Thr Glu Ala Phe Asp Tyr Leu His Arg Leu Gly Ile Ile Tyr Arg Asp
                565                 570                 575

Leu Lys Pro Glu Asn Leu Ile Leu Asp Ala Glu Gly Tyr Leu Lys Leu
            580                 585                 590

Val Asp Phe Gly Phe Ala Lys Lys Ile Gly Ser Gly Gln Lys Thr Trp
        595                 600                 605

Thr Phe Cys Gly Thr Pro Glu Tyr Val Ala Pro Glu Val Ile Leu Asn
    610                 615                 620

Lys Gly His Asp Phe Ser Val Asp Phe Trp Ser Leu Gly Ile Leu Val
625                 630                 635                 640

Tyr Glu Leu Leu Thr Gly Asn Pro Pro Phe Ser Gly Val Asp Gln Met
                645                 650                 655

Met Thr Tyr Asn Leu Ile Leu Lys Gly Ile Glu Lys Met Asp Phe Pro
            660                 665                 670

Arg Lys Ile Thr Arg Arg Pro Glu Asp Leu Ile Arg Arg Leu Cys Arg
        675                 680                 685

Gln Asn Pro Thr Glu Arg Leu Gly Asn Leu Lys Asn Gly Ile Asn Asp
    690                 695                 700

Ile Lys Lys His Arg Trp Leu Asn Gly Phe Asn Trp Glu Gly Leu Lys
705                 710                 715                 720

Ala Arg Ser Leu Pro Ser Pro Leu Gln Arg Glu Leu Lys Gly Pro Ile
                725                 730                 735

Asp His Ser Tyr Phe Asp Lys Tyr Pro Pro Glu Lys Gly Met Pro Pro
            740                 745                 750

Asp Glu Leu Ser Gly Trp Asp Lys Asp Phe
        755                 760

<210>  33
<211>  677
<212>  PRT
<213>  Homo sapiens

<400>  33

Met Gln Pro Thr Leu Leu Leu Ser Leu Leu Gly Ala Val Gly Leu Ala
1               5                   10                  15

Ala Val Asn Ser Met Pro Val Asp Asn Arg Asn His Asn Glu Gly Met
            20                  25                  30

Val Thr Arg Cys Ile Ile Glu Val Leu Ser Asn Ala Leu Ser Lys Ser
        35                  40                  45

Ser Ala Pro Pro Ile Thr Pro Glu Cys Arg Gln Val Leu Lys Thr Ser
    50                  55                  60

Arg Lys Asp Val Lys Asp Lys Glu Thr Thr Glu Asn Glu Asn Thr Lys
65                  70                  75                  80

Phe Glu Val Arg Leu Leu Arg Asp Pro Ala Asp Ala Ser Glu Ala His
                85                  90                  95

Glu Ser Ser Ser Arg Gly Glu Ala Gly Ala Pro Gly Glu Glu Asp Ile
            100                 105                 110

Gln Gly Pro Thr Lys Ala Asp Thr Glu Lys Trp Ala Glu Gly Gly Gly
        115                 120                 125

His Ser Arg Glu Arg Ala Asp Glu Pro Gln Trp Ser Leu Tyr Pro Ser
    130                 135                 140

Asp Ser Gln Val Ser Glu Glu Val Lys Thr Arg His Ser Glu Lys Ser
145                 150                 155                 160

Gln Arg Glu Asp Glu Glu Glu Glu Glu Gly Glu Asn Tyr Gln Lys Gly
                165                 170                 175

Glu Arg Gly Glu Asp Ser Ser Glu Glu Lys His Leu Glu Glu Pro Gly
            180                 185                 190

Glu Thr Gln Asn Ala Phe Leu Asn Glu Arg Lys Gln Ala Ser Ala Ile
        195                 200                 205

Lys Lys Glu Glu Leu Val Ala Arg Ser Glu Thr His Ala Ala Gly His
    210                 215                 220

Ser Gln Glu Lys Thr His Ser Arg Glu Lys Ser Ser Gln Glu Ser Gly
225                 230                 235                 240

Glu Glu Thr Gly Ser Gln Glu Asn His Pro Gln Glu Ser Lys Gly Gln
                245                 250                 255

Pro Arg Ser Gln Glu Glu Ser Glu Glu Gly Glu Glu Asp Ala Thr Ser
            260                 265                 270

Glu Val Asp Lys Arg Arg Thr Arg Pro Arg His His His Gly Arg Ser
        275                 280                 285

Arg Pro Asp Arg Ser Ser Gln Gly Gly Ser Leu Pro Ser Glu Glu Lys
    290                 295                 300

Gly His Pro Gln Glu Glu Ser Glu Glu Ser Asn Val Ser Met Ala Ser
305                 310                 315                 320

Leu Gly Glu Lys Arg Asp His His Ser Thr His Tyr Arg Ala Ser Glu

                325                 330                 335

Glu Glu Pro Glu Tyr Gly Glu Glu Ile Lys Gly Tyr Pro Gly Val Gln
            340                 345                 350

Ala Pro Glu Asp Leu Glu Trp Glu Arg Tyr Arg Gly Arg Gly Ser Glu
        355                 360                 365

Glu Tyr Arg Ala Pro Arg Pro Gln Ser Glu Glu Ser Trp Asp Glu Glu
    370                 375                 380

Asp Lys Arg Asn Tyr Pro Ser Leu Glu Leu Asp Lys Met Ala His Gly
385                 390                 395                 400

Tyr Gly Glu Glu Ser Glu Glu Glu Arg Gly Leu Glu Pro Gly Lys Gly
                405                 410                 415

Arg His His Arg Gly Arg Gly Gly Glu Pro Arg Ala Tyr Phe Met Ser
            420                 425                 430

Asp Thr Arg Glu Glu Lys Arg Phe Leu Gly Glu Gly His His Arg Val
        435                 440                 445

Gln Glu Asn Gln Met Asp Lys Ala Arg Arg His Pro Gln Gly Ala Trp
    450                 455                 460

Lys Glu Leu Asp Arg Asn Tyr Leu Asn Tyr Gly Glu Glu Gly Ala Pro
465                 470                 475                 480

Gly Lys Trp Gln Gln Gln Gly Asp Leu Gln Asp Thr Lys Glu Asn Arg
                485                 490                 495

Glu Glu Ala Arg Phe Gln Asp Lys Gln Tyr Ser Ser His His Thr Ala
            500                 505                 510

Glu Lys Arg Lys Arg Leu Gly Glu Leu Phe Asn Pro Tyr Tyr Asp Pro
        515                 520                 525

Leu Gln Trp Lys Ser Ser His Phe Glu Arg Arg Asp Asn Met Asn Asp
    530                 535                 540

Asn Phe Leu Glu Gly Glu Glu Glu Asn Glu Leu Thr Leu Asn Glu Lys
545                 550                 555                 560

Asn Phe Phe Pro Glu Tyr Asn Tyr Asp Trp Trp Glu Lys Lys Pro Phe
                565                 570                 575

Ser Glu Asp Val Asn Trp Gly Tyr Glu Lys Arg Asn Leu Ala Arg Val
            580                 585                 590

Pro Lys Leu Asp Leu Lys Arg Gln Tyr Asp Arg Val Ala Gln Leu Asp
        595                 600                 605

Gln Leu Leu His Tyr Arg Lys Lys Ser Ala Glu Phe Pro Asp Phe Tyr
    610                 615                 620

Asp Ser Glu Glu Pro Val Ser Thr His Gln Glu Ala Glu Asn Glu Lys
625                 630                 635                 640

Asp Arg Ala Asp Gln Thr Val Leu Thr Glu Asp Glu Lys Lys Glu Leu
                645                 650                 655

Glu Asn Leu Ala Ala Met Asp Leu Glu Leu Gln Lys Ile Ala Glu Lys
            660                 665                 670

Phe Ser Gln Arg Gly
        675

<210> 34
<211> 1019
<212> PRT
<213> Homo sapiens

<400> 34

Met Gly Thr Arg Asp Asp Val Pro Glu Ala Lys Val Leu Val Pro Val
1               5                   10                  15

Ala Val Tyr Cys Gly Ser Ile Pro Arg Thr Ser Ala Gly Pro Arg Val
            20                  25                  30

Leu Pro Pro Gly Ser Ile Asn Ser Ser Leu Pro His Gly Glu Gly Ser
        35                  40                  45

Leu Gln Pro Glu Pro Arg Ala Leu Leu Asn Asn Glu Glu Pro Ser Gln
    50                  55                  60

Leu Leu Arg Gly Leu Gly Gln Leu Gly Gly Leu Lys Leu Asp Thr Pro
65                  70                  75                  80

Ser Lys Gly Trp Gln Ala Arg Asn Gly His Pro Arg Asn Leu Arg Ala
                85                  90                  95

Leu Ser Leu Gly Asp Gln Pro Leu Val Leu Leu Pro Ser Pro Glu Ser
            100                 105                 110

Glu Ala Asn Ser Val Ala Arg Asp Thr Ile Gln Ile Lys Asp Lys Leu
        115                 120                 125

Lys Lys Arg Arg Leu Ser Glu Gly Leu Ala Ala Ser Ser Arg Ala Ser
    130                 135                 140

Leu Asp Pro Gly Gly Gly Pro Gln Gly Val Pro Leu His Ser Thr Ile
145                 150                 155                 160

Pro Arg Ala Thr Ser Gln Arg Leu Leu Arg Val Pro Arg Pro Met Pro
                165                 170                 175

Leu Ile Gln Ser Ile Pro Thr Thr Pro Glu Ala Ser Gly Val Lys Glu
            180                 185                 190

Lys Gly Leu Asp Leu Pro Gly Ser Ile Pro Gly Pro His Glu Leu Arg
        195                 200                 205

Pro Gly Ala Gln Glu Ala Gln Ile Ser Trp Gln Tyr Leu His Cys Asn
    210                 215                 220

Asp Glu Lys Met Gln Lys Ser Leu Gly Ala Ile Val Ile Pro Pro Ile
225                 230                 235                 240

Pro Lys Ala Arg Thr Val Ala Ala Thr Pro Ser Arg Val Pro Gly Ser
                245                 250                 255

Leu Pro Ser Pro Leu Pro Pro Gly Gln Gly Val Leu Thr Gly Leu Arg
            260                 265                 270

Ala Pro Arg Thr Arg Leu Ala Arg Gly Ser Gly Pro Arg Glu Lys Thr
        275                 280                 285

Pro Ala Ser Leu Glu Pro Lys Pro Leu Ala Ser Pro Ile Arg Asp Arg
    290                 295                 300

Pro Ala Ala Ala Lys Lys Pro Ala Leu Pro Phe Ser Gln Ser Ala Pro
305                 310                 315                 320

Thr Leu Thr Ala Phe Ser Phe Asp Cys Ala Arg Glu Ala Cys Pro Pro
                325                 330                 335

Leu Lys Glu Glu Asp Gln Lys Glu Ile Gly Thr Lys Ile Gln Val Thr
            340                 345                 350

Ile Ser Lys Ser Ala Arg Glu Lys Met Gln Leu Lys Gln Met Lys Glu
        355                 360                 365

Met Glu Leu Leu Arg Arg Leu Glu Glu Pro Arg Thr Gly Gln Glu Leu
    370                 375                 380

Thr Ser Gln Cys Leu Gly Ser Gln Arg Ala Phe Met Lys Glu Gly Leu
385                 390                 395                 400

Leu Pro Leu Arg Gly Ser Gly Thr Leu Ser Val Pro Thr Arg Leu Ser
                405                 410                 415

Gly Pro Cys Arg Asn Asp Val Ser Ile Ile Leu Arg Lys Trp Ala Ser
            420                 425                 430

Arg Ala Ser Leu Pro Ser Ile Pro Ile Ser Arg Gln Glu Pro Arg Phe
        435                 440                 445

Ala Arg His Ala Ser Ala Asn Ser Leu Pro Ala Val Leu Thr Leu Gly
    450                 455                 460

Ser Pro Glu Trp Glu Glu Glu Glu Glu Met Asp Leu Arg Ala Cys Lys
465                 470                 475                 480

Glu Leu Arg Pro Phe Ser Asn Pro Glu Leu Gly Leu Arg Asp Ala Leu
                485                 490                 495

Gln Cys Leu Asn Ser Ser Asp Trp Gln Met Lys Glu Lys Gly Leu Val
            500                 505                 510

Ser Ile Gln Arg Leu Ala Ala Cys His Ser Glu Val Leu Thr Gly Lys
        515                 520                 525

Leu His Asp Val Cys Leu Val Val Thr Gly Glu Val Thr Asn Leu Arg
    530                 535                 540

Ser Lys Val Ser His Leu Ala Ile Ser Thr Leu Gly Asp Leu Phe Gln
545                 550                 555                 560

Ala Leu Lys Lys Asn Met Asp Gln Glu Ala Glu Glu Ile Ala Arg Cys
                565                 570                 575

Leu Leu Gln Lys Met Ala Asp Thr Asn Glu Phe Ile Gln Arg Ala Ala
            580                 585                 590

Gly Gln Ser Leu Arg Ala Met Val Glu Asn Val Thr Leu Ala Arg Ser
        595                 600                 605

Leu Val Val Leu Thr Ser Ala Gly Val Tyr His Arg Asn Pro Leu Ile
    610                 615                 620

Arg Lys Tyr Ala Ala Glu His Leu Ser Ala Val Leu Glu Gln Ile Gly
625                 630                 635                 640

Ala Glu Lys Leu Leu Ser Gly Thr Arg Asp Ser Thr Asp Met Leu Val
                645                 650                 655

His Asn Leu Val Arg Leu Ala Gln Asp Ser Asn Gln Asp Thr Arg Phe
            660                 665                 670

Tyr Gly Arg Lys Met Val Asn Ile Leu Met Ala Asn Thr Lys Phe Asp
        675                 680                 685

Ala Phe Leu Lys Gln Ser Leu Pro Ser Tyr Asp Leu Gln Lys Val Met
    690                 695                 700

Ala Ala Ile Lys Gln Gln Gly Ile Glu Asp Asn Asp Glu Leu Pro Ser
705                 710                 715                 720

Ala Lys Gly Arg Lys Val Leu Arg Ser Leu Val Val Cys Glu Asn Gly
                725                 730                 735

Leu Pro Ile Lys Glu Gly Leu Ser Cys Asn Gly Pro Arg Leu Val Gly
            740                 745                 750

Leu Arg Ser Thr Leu Gln Gly Arg Gly Glu Met Val Glu Gln Leu Arg
        755                 760                 765

Glu Leu Thr Arg Leu Leu Glu Ala Lys Asp Phe Arg Ser Arg Met Glu
    770                 775                 780

Gly Val Gly Gln Leu Leu Glu Leu Cys Lys Ala Lys Thr Glu Leu Val
785                 790                 795                 800

Thr Ala His Leu Val Gln Val Phe Asp Ala Phe Thr Pro Arg Leu Gln
                805                 810                 815

Asp Ser Asn Lys Lys Val Asn Gln Trp Ala Leu Glu Ser Phe Ala Lys
            820                 825                 830

Met Ile Pro Leu Leu Arg Glu Ser Leu His Pro Met Leu Leu Ser Ile
        835                 840                 845

Ile Ile Thr Val Ala Asp Asn Leu Asn Ser Lys Asn Ser Gly Ile Tyr
    850                 855                 860

Ala Ala Ala Val Ala Val Leu Asp Ala Met Val Glu Ser Leu Asp Asn

865                 870                 875                 880

Leu Cys Leu Leu Pro Ala Leu Ala Gly Arg Val Arg Phe Leu Ser Gly
                885                 890                 895

Arg Ala Val Leu Asp Val Thr Asp Arg Leu Ala Val Leu Val Ala Ser
            900                 905                 910

Val Tyr Pro Arg Lys Pro Gln Ala Val Glu Arg His Val Leu Pro Ile
        915                 920                 925

Leu Trp His Phe Leu Asn Thr Ala Thr Arg Asn Gly Thr Leu Pro Gly
    930                 935                 940

Pro Ser Gly Asn Ile Arg Gly Val Val Cys Arg Leu Ser Arg Ser Leu
945                 950                 955                 960

Gln Glu His Met Gly Ser Arg Leu Leu Asp Phe Ala Ala Ser Gln Pro
                965                 970                 975

Lys His Val Leu Lys Thr Leu Gln Glu Leu Leu Asp Ser Glu Ser Leu
            980                 985                 990

Gly Gly Ser Arg Lys Ala Thr Asp  Arg Gly Val Ala Pro  Asp Ser Lys
        995                 1000                1005

Thr Thr  Gly Ser Ser Tyr Pro  Phe Gln Leu Asp
    1010                1015

<210>  35
<211>  417
<212>  PRT
<213>  Homo sapiens

<400>  35

Met Ala Gly Pro Phe Ser Arg Leu Leu Ser Ala Arg Pro Gly Leu Arg
1               5                   10                  15

Leu Leu Ala Leu Ala Gly Ala Gly Ser Leu Ala Ala Gly Phe Leu Leu
            20                  25                  30

Arg Pro Glu Pro Val Arg Ala Ala Ser Glu Arg Arg Arg Leu Tyr Pro
        35                  40                  45

Pro Ser Ala Glu Tyr Pro Asp Leu Arg Lys His Asn Asn Cys Met Ala
    50                  55                  60

Ser His Leu Thr Pro Ala Val Tyr Ala Arg Leu Cys Asp Lys Thr Thr

65                  70                  75                  80

Pro Thr Gly Trp Thr Leu Asp Gln Cys Ile Gln Thr Gly Val Asp Asn
                85                  90                  95

Pro Gly His Pro Phe Ile Lys Thr Val Gly Met Val Ala Gly Asp Glu
            100                 105                 110

Glu Thr Tyr Glu Val Phe Ala Asp Leu Phe Asp Pro Val Ile Gln Glu
        115                 120                 125

Arg His Asn Gly Tyr Asp Pro Arg Thr Met Lys His Thr Thr Asp Leu
    130                 135                 140

Asp Ala Ser Lys Ile Arg Ser Gly Tyr Phe Asp Glu Arg Tyr Val Leu
145                 150                 155                 160

Ser Ser Arg Val Arg Thr Gly Arg Ser Ile Arg Gly Leu Ser Leu Pro
                165                 170                 175

Pro Ala Cys Thr Arg Ala Glu Arg Arg Glu Val Glu Arg Val Val Val
            180                 185                 190

Asp Ala Leu Ser Gly Leu Lys Gly Asp Leu Ala Gly Arg Tyr Tyr Arg
        195                 200                 205

Leu Ser Glu Met Thr Glu Ala Glu Gln Gln Gln Leu Ile Asp Asp His
    210                 215                 220

Phe Leu Phe Asp Lys Pro Val Ser Pro Leu Leu Thr Ala Ala Gly Met
225                 230                 235                 240

Ala Arg Asp Trp Pro Asp Ala Arg Gly Ile Trp His Asn Asn Glu Lys
                245                 250                 255

Ser Phe Leu Ile Trp Val Asn Glu Glu Asp His Thr Arg Val Ile Ser
            260                 265                 270

Met Glu Lys Gly Gly Asn Met Lys Arg Val Phe Glu Arg Phe Cys Arg
        275                 280                 285

Gly Leu Lys Glu Val Glu Arg Leu Ile Gln Glu Arg Gly Trp Glu Phe
    290                 295                 300

Met Trp Asn Glu Arg Leu Gly Tyr Ile Leu Thr Cys Pro Ser Asn Leu
305                 310                 315                 320

Gly Thr Gly Leu Arg Ala Gly Val His Ile Lys Leu Pro Leu Leu Ser
                325                 330                 335

Lys Asp Ser Arg Phe Pro Lys Ile Leu Glu Asn Leu Arg Leu Gln Lys
            340                 345                 350

Arg Gly Thr Gly Gly Val Asp Thr Ala Ala Thr Gly Gly Val Phe Asp
        355                 360                 365

Ile Ser Asn Leu Asp Arg Leu Gly Lys Ser Glu Val Glu Leu Val Gln
    370                 375                 380

Leu Val Ile Asp Gly Val Asn Tyr Leu Ile Asp Cys Glu Arg Arg Leu
385                 390                 395                 400

Glu Arg Gly Gln Asp Ile Arg Ile Pro Thr Pro Val Ile His Thr Lys
                405                 410                 415

His

<210>  36
<211>  298
<212>  PRT
<213>  Homo sapiens

<400>  36

Met Ala Gly Pro Phe Ser Arg Leu Leu Ser Ala Arg Pro Gly Leu Arg
1               5                   10                  15

Leu Leu Ala Leu Ala Gly Ala Gly Ser Leu Ala Ala Gly Phe Leu Leu
            20                  25                  30

Arg Pro Glu Pro Val Arg Ala Ala Ser Glu Arg Arg Arg Leu Tyr Pro
        35                  40                  45

Pro Ser Ala Glu Tyr Pro Asp Leu Arg Lys His Asn Asn Cys Met Ala
    50                  55                  60

Ser His Leu Thr Pro Ala Val Tyr Ala Arg Leu Cys Asp Lys Thr Thr
65                  70                  75                  80

Pro Thr Gly Trp Thr Leu Asp Gln Cys Ile Gln Thr Gly Val Asp Asn
                85                  90                  95

Pro Gly His Pro Phe Ile Lys Thr Val Gly Met Val Ala Gly Asp Glu
            100                 105                 110

Glu Thr Tyr Glu Val Phe Ala Asp Leu Phe Asp Pro Val Ile Gln Glu
        115                 120                 125

Arg His Asn Gly Tyr Asp Pro Arg Thr Met Lys His Thr Thr Asp Leu
    130                 135                 140

Asp Ala Ser Lys Ile Arg Ser Gly Tyr Phe Asp Glu Arg Tyr Val Leu
145                 150                 155                 160

Ser Ser Arg Val Arg Thr Gly Arg Ser Ile Arg Gly Leu Ser Leu Pro
                165                 170                 175

Pro Ala Cys Thr Arg Ala Glu Arg Arg Glu Val Glu Arg Val Val Val
            180                 185                 190

Asp Ala Leu Ser Gly Leu Lys Gly Asp Leu Ala Gly Arg Tyr Tyr Arg
        195                 200                 205

Leu Ser Glu Met Thr Glu Ala Glu Gln Gln Gln Leu Ile Asp Asp His
    210                 215                 220

Phe Leu Phe Asp Lys Pro Val Ser Pro Leu Leu Thr Ala Ala Gly Met
225                 230                 235                 240

Ala Arg Asp Trp Pro Asp Ala Arg Gly Ile Trp His Asn Asn Glu Lys
                245                 250                 255

Ser Phe Leu Ile Trp Val Asn Glu Glu Asp His Thr Arg Val Ile Ser
            260                 265                 270

Met Glu Lys Gly Gly Asn Met Lys Arg Val Phe Glu Arg Phe Cys Arg
        275                 280                 285

Gly Leu Lys Glu Val Val Val Gly Ile Lys
    290                 295

<210>  37
<211>  1303
<212>  PRT
<213>  Homo sapiens

<400>  37

<210> 38
<211> 244
<212> PRT
<213> Homo sapiens

<400> 38

Met Asp Ile Leu Val Pro Leu Leu Gln Leu Leu Val Leu Leu Leu Thr
1               5                   10                  15

Leu Pro Leu His Leu Met Ala Leu Leu Gly Cys Trp Gln Pro Leu Cys
            20                  25                  30

Lys Ser Tyr Phe Pro Tyr Leu Met Ala Val Leu Thr Pro Lys Ser Asn
        35                  40                  45

Arg Lys Met Glu Ser Lys Lys Arg Glu Leu Phe Ser Gln Ile Lys Gly
    50                  55                  60

Leu Thr Gly Ala Ser Gly Lys Val Ala Leu Leu Glu Leu Gly Cys Gly
65                  70                  75                  80

Thr Gly Ala Asn Phe Gln Phe Tyr Pro Pro Gly Cys Arg Val Thr Cys
                85                  90                  95

Leu Asp Pro Asn Pro His Phe Glu Lys Phe Leu Thr Lys Ser Met Ala
            100                 105                 110

Glu Asn Arg His Leu Gln Tyr Glu Arg Phe Val Val Ala Pro Gly Glu
        115                 120                 125

Asp Met Arg Gln Leu Ala Asp Gly Ser Met Asp Val Val Val Cys Thr
    130                 135                 140

Leu Val Leu Cys Ser Val Gln Ser Pro Arg Lys Val Leu Gln Glu Val
145                 150                 155                 160

Arg Arg Val Leu Arg Pro Gly Gly Val Leu Phe Phe Trp Glu His Val
                165                 170                 175

Ala Glu Pro Tyr Gly Ser Trp Ala Phe Met Trp Gln Gln Val Phe Glu
            180                 185                 190

Pro Thr Trp Lys His Ile Gly Asp Gly Cys Cys Leu Thr Arg Glu Thr
        195                 200                 205

Trp Lys Asp Leu Glu Asn Ala Gln Phe Ser Glu Ile Gln Met Glu Arg
    210                 215                 220

Gln Pro Pro Pro Leu Lys Trp Leu Pro Val Gly Pro His Ile Met Gly
225                 230                 235                 240

Lys Ala Val Lys

<210>  39
<211>  1821
<212>  PRT
<213>  Homo sapiens

<400>  39
   Met Arg Pro Arg Thr Lys Ala Arg Ser Pro Gly Arg Ala Leu Arg Asn
   1               5                   10                  15
   Pro Trp Arg Gly Phe Leu Pro Leu Thr Leu Ala Leu Phe Val Gly Ala
               20                  25                  30
   Gly His Ala Gln Arg Asp Pro Val Gly Arg Tyr Glu Pro Ala Gly Gly
           35                  40                  45
   Asp Ala Asn Arg Leu Arg Arg Pro Gly Gly Ser Tyr Pro Ala Ala Ala
       50                  55                  60
   Ala Ala Lys Val Tyr Ser Leu Phe Arg Glu Gln Asp Ala Pro Val Ala
   65                  70                  75                  80
   Gly Leu Gln Pro Val Glu Arg Ala Gln Pro Gly Trp Gly Ser Pro Arg
                   85                  90                  95
   Arg Pro Thr Glu Ala Glu Ala Arg Arg Pro Ser Arg Ala Gln Gln Ser
               100                 105                 110
   Arg Arg Val Gln Pro Pro Ala Gln Thr Arg Arg Ser Thr Pro Leu Gly
           115                 120                 125
   Gln Gln Gln Pro Ala Pro Arg Thr Arg Ala Ala Pro Ala Leu Pro Arg
       130                 135                 140
   Leu Gly Thr Pro Gln Arg Ser Gly Ala Ala Pro Pro Thr Pro Pro Arg
   145                 150                 155                 160
   Gly Arg Leu Thr Gly Arg Asn Val Cys Gly Gly Gln Cys Cys Pro Gly
                   165                 170                 175
   Trp Thr Thr Ala Asn Ser Thr Asn His Cys Ile Lys Pro Val Cys Glu
               180                 185                 190
   Pro Pro Cys Gln Asn Arg Gly Ser Cys Ser Arg Pro Gln Leu Cys Val
           195                 200                 205
   Cys Arg Ser Gly Phe Arg Gly Ala Arg Cys Glu Glu Val Ile Pro Asp
       210                 215                 220
   Glu Glu Phe Asp Pro Gln Asn Ser Arg Leu Ala Pro Arg Arg Trp Ala
   225                 230                 235                 240
   Glu Arg Ser Pro Asn Leu Arg Arg Ser Ser Ala Ala Gly Glu Gly Thr
                   245                 250                 255
   Leu Ala Arg Ala Gln Pro Pro Ala Pro Gln Ser Pro Pro Ala Pro Gln
               260                 265                 270
   Ser Pro Pro Ala Gly Thr Leu Ser Gly Leu Ser Gln Thr His Pro Ser
           275                 280                 285
   Gln Gln His Val Gly Leu Ser Arg Thr Val Arg Leu His Pro Thr Ala
       290                 295                 300
   Thr Ala Ser Ser Gln Leu Ser Ser Asn Ala Leu Pro Pro Gly Pro Gly
   305                 310                 315                 320
   Leu Glu Gln Arg Asp Gly Thr Gln Gln Ala Val Pro Leu Glu His Pro
                   325                 330                 335
   Ser Ser Pro Trp Gly Leu Asn Leu Thr Glu Lys Ile Lys Lys Ile Lys
               340                 345                 350
   Ile Val Phe Thr Pro Thr Ile Cys Lys Gln Thr Cys Ala Arg Gly His
           355                 360                 365
   Cys Ala Asn Ser Cys Glu Arg Gly Asp Thr Thr Thr Leu Tyr Ser Gln
       370                 375                 380
   Gly Gly His Gly His Asp Pro Lys Ser Gly Phe Arg Ile Tyr Phe Cys
   385                 390                 395                 400
   Gln Ile Pro Cys Leu Asn Gly Gly Arg Cys Ile Gly Arg Asp Glu Cys
                   405                 410                 415
   Trp Cys Pro Ala Asn Ser Thr Gly Lys Phe Cys His Leu Pro Ile Pro
   420 425 430
   Gln Pro Asp Arg Glu Pro Pro Gly Arg Gly Ser Arg Pro Arg Ala Leu
   435 440 445
   Leu Glu Ala Pro Leu Lys Gln Ser Thr Phe Thr Leu Pro Leu Ser Asn
   450 455 460
   Gln Leu Ala Ser Val Asn Pro Ser Leu Val Lys Val His Ile His His
   465 470 475 480
   Pro Pro Glu Ala Ser Val Gln Ile His Gln Val Ala Gln Val Arg Gly
   485 490 495
   Gly Val Glu Glu Ala Leu Val Glu Asn Ser Val Glu Thr Arg Pro Pro
   500 505 510
   Pro Trp Leu Pro Ala Ser Pro Gly His Ser Leu Trp Asp Ser Asn Asn
   515 520 525
   Ile Pro Ala Arg Ser Gly Glu Pro Pro Arg Pro Leu Pro Pro Ala Ala
   530 535 540
   Pro Arg Pro Arg Gly Leu Leu Gly Arg Cys Tyr Leu Asn Thr Val Asn
   545 550 555 560
   Gly Gln Cys Ala Asn Pro Leu Leu Glu Leu Thr Thr Gln Glu Asp Cys
   565 570 575
   Cys Gly Ser Val Gly Ala Phe Trp Gly Val Thr Leu Cys Ala Pro Cys
   580 585 590
   Pro Pro Arg Pro Ala Ser Pro Val Ile Glu Asn Gly Gln Leu Glu Cys
   595 600 605
   Pro Gln Gly Tyr Lys Arg Leu Asn Leu Thr His Cys Gln Asp Ile Asn
   610 615 620
   Glu Cys Leu Thr Leu Gly Leu Cys Lys Asp Ala Glu Cys Val Asn Thr
   625 630 635 640
   Arg Gly Ser Tyr Leu Cys Thr Cys Arg Pro Gly Leu Met Leu Asp Pro
   645 650 655
   Ser Arg Ser Arg Cys Val Ser Asp Lys Ala Ile Ser Met Leu Gln Gly
   660 665 670
   Leu Cys Tyr Arg Ser Leu Gly Pro Gly Thr Cys Thr Leu Pro Leu Ala
   675 680 685
   Gln Arg Ile Thr Lys Gln Ile Cys Cys Cys Ser Arg Val Gly Lys Ala
   690 695 700
   Trp Gly Ser Glu Cys Glu Lys Cys Pro Leu Pro Gly Thr Glu Ala Phe
   705 710 715 720
   Arg Glu Ile Cys Pro Ala Gly His Gly Tyr Thr Tyr Ala Ser Ser Asp
   725 730 735
   Ile Arg Leu Ser Met Arg Lys Ala Glu Glu Glu Glu Leu Ala Arg Pro
   740 745 750
   Pro Arg Glu Gln Gly Gln Arg Ser Ser Gly Ala Leu Pro Gly Pro Ala
   755 760 765
   Glu Arg Gln Pro Leu Arg Val Val Thr Asp Thr Trp Leu Glu Ala Gly
   770 775 780
   Thr Ile Pro Asp Lys Gly Asp Ser Gln Ala Gly Gln Val Thr Thr Ser
   785 790 795 800
   Val Thr His Ala Pro Ala Trp Val Thr Gly Asn Ala Thr Thr Pro Pro
   805 810 815
   Met Pro Glu Gln Gly Ile Ala Glu Ile Gln Glu Glu Gln Val Thr Pro
   820 825 830
   Ser Thr Asp Val Leu Val Thr Leu Ser Thr Pro Gly Ile Asp Arg Cys
   835 840 845
   Ala Ala Gly Ala Thr Asn Val Cys Gly Pro Gly Thr Cys Val Asn Leu
   850 855 860
   Pro Asp Gly Tyr Arg Cys Val Cys Ser Pro Gly Tyr Gln Leu His Pro
   865 870 875 880
   Ser Gln Ala Tyr Cys Thr Asp Asp Asn Glu Cys Leu Arg Asp Pro Cys
   885 890 895
   Lys Gly Lys Gly Arg Cys Ile Asn Arg Val Gly Ser Tyr Ser Cys Phe
   900 905 910
   Cys Tyr Pro Gly Tyr Thr Leu Ala Thr Ser Gly Ala Thr Gln Glu Cys
           915                 920                 925
   Gln Asp Ile Asn Glu Cys Glu Gln Pro Gly Val Cys Ser Gly Gly Gln
       930                 935                 940
   Cys Thr Asn Thr Glu Gly Ser Tyr His Cys Glu Cys Asp Gln Gly Tyr
   945                 950                 955                 960
   Ile Met Val Arg Lys Gly His Cys Gln Asp Ile Asn Glu Cys Arg His
                   965                 970                 975
   Pro Gly Thr Cys Pro Asp Gly Arg Cys Val Asn Ser Pro Gly Ser Tyr
               980                 985                 990
   Thr Cys Leu Ala Cys Glu Glu Gly  Tyr Arg Gly Gln Ser  Gly Ser Cys
           995                 1000                 1005
   Val Asp  Val Asn Glu Cys Leu  Thr Pro Gly Val Cys  Ala His Gly
       1010                 1015                 1020
   Lys Cys  Thr Asn Leu Glu Gly  Ser Phe Arg Cys Ser  Cys Glu Gln
       1025                 1030                 1035
   Gly Tyr  Glu Val Thr Ser Asp  Glu Lys Gly Cys Gln  Asp Val Asp
       1040                 1045                 1050
   Glu Cys  Ala Ser Arg Ala Ser  Cys Pro Thr Gly Leu  Cys Leu Asn
       1055                 1060                 1065
   Thr Glu  Gly Ser Phe Ala Cys  Ser Ala Cys Glu Asn  Gly Tyr Trp
       1070                 1075                 1080
   Val Asn  Glu Asp Gly Thr Ala  Cys Glu Asp Leu Asp  Glu Cys Ala
       1085                 1090                 1095
   Phe Pro  Gly Val Cys Pro Ser  Gly Val Cys Thr Asn  Thr Ala Gly
       1100                 1105                 1110
   Ser Phe  Ser Cys Lys Asp Cys  Asp Gly Gly Tyr Arg  Pro Ser Pro
       1115                 1120                 1125
   Leu Gly  Asp Ser Cys Glu Asp  Val Asp Glu Cys Glu  Asp Pro Gln
       1130                 1135                 1140
   Ser Ser  Cys Leu Gly Gly Glu  Cys Lys Asn Thr Val  Gly Ser Tyr
       1145                 1150                 1155
   Gln Cys Leu Cys Pro Gln Gly Phe Gln Leu Ala Asn Gly Thr Val
   1160 1165 1170
   Cys Glu Asp Val Asn Glu Cys Met Gly Glu Glu His Cys Ala Pro
   1175 1180 1185
   His Gly Glu Cys Leu Asn Ser His Gly Ser Phe Phe Cys Leu Cys
   1190 1195 1200
   Ala Pro Gly Phe Val Ser Ala Glu Gly Gly Thr Ser Cys Gln Asp
   1205 1210 1215
   Val Asp Glu Cys Ala Thr Thr Asp Pro Cys Val Gly Gly His Cys
   1220 1225 1230
   Val Asn Thr Glu Gly Ser Phe Asn Cys Leu Cys Glu Thr Gly Phe
   1235 1240 1245
   Gln Pro Ser Pro Glu Ser Gly Glu Cys Val Asp Ile Asp Glu Cys
   1250 1255 1260
   Glu Asp Tyr Gly Asp Pro Val Cys Gly Thr Trp Lys Cys Glu Asn
   1265 1270 1275
   Ser Pro Gly Ser Tyr Arg Cys Val Leu Gly Cys Gln Pro Gly Phe
   1280 1285 1290
   His Met Ala Pro Asn Gly Asp Cys Ile Asp Ile Asp Glu Cys Ala
   1295 1300 1305
   Asn Asp Thr Met Cys Gly Ser His Gly Phe Cys Asp Asn Thr Asp
   1310 1315 1320
   Gly Ser Phe Arg Cys Leu Cys Asp Gln Gly Phe Glu Ile Ser Pro
   1325 1330 1335
   Ser Gly Trp Asp Cys Val Asp Val Asn Glu Cys Glu Leu Met Leu
   1340 1345 1350
   Ala Val Cys Gly Ala Ala Leu Cys Glu Asn Val Glu Gly Ser Phe
   1355 1360 1365
   Leu Cys Leu Cys Ala Ser Asp Leu Glu Glu Tyr Asp Ala Gln Glu
   1370 1375 1380
   Gly His Cys Arg Pro Arg Gly Ala Gly Gly Gln Ser Met Ser Glu
   1385 1390 1395
   Ala Pro Thr Gly Asp His Ala Pro Ala Pro Thr Arg Met Asp Cys
       1400                1405                1410
   Tyr Ser Gly Gln Lys Gly His Ala Pro Cys Ser Ser Val Leu Gly
       1415                1420                1425
   Arg Asn Thr Thr Gln Ala Glu Cys Cys Cys Thr Gln Gly Ala Ser
       1430                1435                1440
   Trp Gly Asp Ala Cys Asp Leu Cys Pro Ser Glu Asp Ser Ala Glu
       1445                1450                1455
   Phe Ser Glu Ile Cys Pro Ser Gly Lys Gly Tyr Ile Pro Val Glu
       1460                1465                1470
   Gly Ala Trp Thr Phe Gly Gln Thr Met Tyr Thr Asp Ala Asp Glu
       1475                1480                1485
   Cys Val Ile Phe Gly Pro Gly Leu Cys Pro Asn Gly Arg Cys Leu
       1490                1495                1500
   Asn Thr Val Pro Gly Tyr Val Cys Leu Cys Asn Pro Gly Phe His
       1505                1510                1515
   Tyr Asp Ala Ser His Lys Lys Cys Glu Asp His Asp Glu Cys Gln
       1520                1525                1530
   Asp Leu Ala Cys Glu Asn Gly Glu Cys Val Asn Thr Glu Gly Ser
       1535                1540                1545
   Phe His Cys Phe Cys Ser Pro Pro Leu Thr Leu Asp Leu Ser Gln
       1550                1555                1560
   Gln Arg Cys Met Asn Ser Thr Ser Ser Thr Glu Asp Leu Pro Asp
       1565                1570                1575
   His Asp Ile His Met Asp Ile Cys Trp Lys Lys Val Thr Asn Asp
       1580                1585                1590
   Val Cys Ser Glu Pro Leu Arg Gly His Arg Thr Thr Tyr Thr Glu
       1595                1600                1605
   Cys Cys Cys Gln Asp Gly Glu Ala Trp Ser Gln Gln Cys Ala Leu
       1610                1615                1620
   Cys Pro Pro Arg Ser Ser Glu Val Tyr Ala Gln Leu Cys Asn Val
   1625                1630                1635
   Ala Arg Ile Glu Ala Glu Arg Glu Ala Gly Val His Phe Arg Pro
       1640                1645                1650
   Gly Tyr Glu Tyr Gly Pro Gly Pro Asp Asp Leu His Tyr Ser Ile
       1655                1660                1665
   Tyr Gly Pro Asp Gly Ala Pro Phe Tyr Asn Tyr Leu Gly Pro Glu
       1670                1675                1680
   Asp Thr Val Pro Glu Pro Ala Phe Pro Asn Thr Ala Gly His Ser
       1685                1690                1695
   Ala Asp Arg Thr Pro Ile Leu Glu Ser Pro Leu Gln Pro Ser Glu
       1700                1705                1710
   Leu Gln Pro His Tyr Val Ala Ser His Pro Glu Pro Pro Ala Gly
       1715                1720                1725
   Phe Glu Gly Leu Gln Ala Glu Glu Cys Gly Ile Leu Asn Gly Cys
       1730                1735                1740
   Glu Asn Gly Arg Cys Val Arg Val Arg Glu Gly Tyr Thr Cys Asp
       1745                1750                1755
   Cys Phe Glu Gly Phe Gln Leu Asp Ala Ala His Met Ala Cys Val
       1760                1765                1770
   Asp Val Asn Glu Cys Asp Asp Leu Asn Gly Pro Ala Val Leu Cys
       1775                1780                1785
   Val His Gly Tyr Cys Glu Asn Thr Glu Gly Ser Tyr Arg Cys His
       1790                1795                1800
   Cys Ser Pro Gly Tyr Val Ala Glu Ala Gly Pro Pro His Cys Thr
       1805                1810                1815
   Ala Lys Glu
       1820

<210>  40
<211>  1010
<212>  PRT
<213>  Homo sapiens

<400>  40

<210>  41
<211>  973
<212>  PRT
<213>  Homo sapiens

<400>  41

<210>  42
<211>  359
<212>  PRT
<213>  Homo sapiens

<400>  42

Met Glu Arg Arg Ala Ser Glu Thr Pro Glu Asp Gly Asp Pro Glu Glu
1               5                   10                  15

Asp Thr Ala Thr Ala Leu Gln Arg Leu Val Glu Leu Thr Thr Ser Arg
            20                  25                  30

Val Thr Pro Val Arg Ser Leu Arg Asp Gln Tyr His Leu Ile Arg Lys
        35                  40                  45

Leu Gly Ser Gly Ser Tyr Gly Arg Val Leu Leu Ala Gln Pro His Gln
    50                  55                  60

Gly Gly Pro Ala Val Ala Leu Lys Leu Leu Arg Arg Asp Leu Val Leu
65                  70                  75                  80

Arg Ser Thr Phe Leu Arg Glu Phe Cys Val Gly Arg Cys Val Ser Ala
                85                  90                  95

His Pro Gly Leu Leu Gln Thr Leu Ala Gly Pro Leu Gln Thr Pro Arg
            100                 105                 110

Tyr Phe Ala Phe Ala Gln Glu Tyr Ala Pro Cys Gly Asp Leu Ser Gly
        115                 120                 125

Met Leu Gln Glu Arg Gly Leu Pro Glu Leu Leu Val Lys Arg Val Val
    130                 135                 140

Ala Gln Leu Ala Gly Ala Leu Asp Phe Leu His Ser Arg Gly Leu Val
145                 150                 155                 160

His Ala Asp Val Lys Pro Asp Asn Val Leu Val Phe Asp Pro Val Cys
                165                 170                 175

Ser Arg Val Ala Leu Gly Asp Leu Gly Leu Thr Arg Pro Glu Gly Ser
            180                 185                 190

Pro Thr Pro Ala Pro Pro Val Pro Leu Pro Thr Ala Pro Pro Glu Leu
        195                 200                 205

Cys Leu Leu Leu Pro Pro Asp Thr Leu Pro Leu Arg Pro Ala Val Asp
    210                 215                 220

Ser Trp Gly Leu Gly Val Leu Leu Phe Cys Ala Ala Thr Ala Cys Phe
225                 230                 235                 240

Pro Trp Asp Val Ala Leu Ala Pro Asn Pro Glu Phe Glu Ala Phe Ala
                245                 250                 255

Gly Trp Val Thr Thr Lys Pro Gln Pro Pro Gln Pro Pro Pro Pro Trp
            260                 265                 270

Asp Gln Phe Ala Pro Pro Ala Leu Ala Leu Leu Gln Gly Leu Leu Asp
        275                 280                 285

Leu Asp Pro Glu Thr Arg Ser Pro Pro Leu Ala Val Leu Asp Phe Leu
    290                 295                 300

Gly Asp Asp Trp Gly Leu Gln Gly Asn Arg Glu Gly Pro Gly Val Leu
305                 310                 315                 320

Gly Ser Ala Val Ser Tyr Glu Asp Arg Glu Glu Gly Gly Ser Ser Leu
                325                 330                 335

Glu Glu Trp Thr Asp Glu Gly Asp Asp Ser Lys Ser Gly Gly Arg Thr
            340                 345                 350

Gly Thr Asp Gly Gly Ala Pro
        355

<210>  43
<211>  314
<212>  PRT
<213>  Homo sapiens

<400>  43

Met Gly Ile Gly Arg Ser Glu Gly Gly Arg Arg Gly Ala Ala Leu Gly
1               5                   10                  15

Val Leu Leu Ala Leu Gly Ala Ala Leu Leu Ala Val Gly Ser Ala Ser
            20                  25                  30

Glu Tyr Asp Tyr Val Ser Phe Gln Ser Asp Ile Gly Pro Tyr Gln Ser
        35                  40                  45

Gly Arg Phe Tyr Thr Lys Pro Pro Gln Cys Val Asp Ile Pro Ala Asp
    50                  55                  60

Leu Arg Leu Cys His Asn Val Gly Tyr Lys Lys Met Val Leu Pro Asn
65                  70                  75                  80

Leu Leu Glu His Glu Thr Met Ala Glu Val Lys Gln Gln Ala Ser Ser
                85                  90                  95

Trp Val Pro Leu Leu Asn Lys Asn Cys His Ala Gly Thr Gln Val Phe
            100                 105                 110

Leu Cys Ser Leu Phe Ala Pro Val Cys Leu Asp Arg Pro Ile Tyr Pro
        115                 120                 125

Cys Arg Trp Leu Cys Glu Ala Val Arg Asp Ser Cys Glu Pro Val Met
    130                 135                 140

Gln Phe Phe Gly Phe Tyr Trp Pro Glu Met Leu Lys Cys Asp Lys Phe
145                 150                 155                 160

Pro Glu Gly Asp Val Cys Ile Ala Met Thr Pro Pro Asn Ala Thr Glu
                165                 170                 175

Ala Ser Lys Pro Gln Gly Thr Thr Val Cys Pro Pro Cys Asp Asn Glu
            180                 185                 190

Leu Lys Ser Glu Ala Ile Ile Glu His Leu Cys Ala Ser Glu Phe Ala
        195                 200                 205

Leu Arg Met Lys Ile Lys Glu Val Lys Lys Glu Asn Gly Asp Lys Lys
    210                 215                 220

Ile Val Pro Lys Lys Lys Lys Pro Leu Lys Leu Gly Pro Ile Lys Lys
225                 230                 235                 240

Lys Asp Leu Lys Lys Leu Val Leu Tyr Leu Lys Asn Gly Ala Asp Cys
                245                 250                 255

Pro Cys His Gln Leu Asp Asn Leu Ser His His Phe Leu Ile Met Gly
            260                 265                 270

Arg Lys Val Lys Ser Gln Tyr Leu Leu Thr Ala Ile His Lys Trp Asp
        275                 280                 285

Lys Lys Asn Lys Glu Phe Lys Asn Phe Met Lys Lys Met Lys Asn His
    290                 295                 300

Glu Cys Pro Thr Phe Gln Ser Val Phe Lys
305                 310

<210>  44

<211>  279
<212>  PRT
<213>  Homo sapiens

<400>  44

Met Thr Glu Arg Phe Asp Cys His His Cys Asn Glu Ser Leu Phe Gly
1               5                   10                  15

Lys Lys Tyr Ile Leu Arg Glu Glu Ser Pro Tyr Cys Val Val Cys Phe
            20                  25                  30

Glu Thr Leu Phe Ala Asn Thr Cys Glu Glu Cys Gly Lys Pro Ile Gly
        35                  40                  45

Cys Asp Cys Lys Asp Leu Ser Tyr Lys Asp Arg His Trp His Glu Ala
    50                  55                  60

Cys Phe His Cys Ser Gln Cys Arg Asn Ser Leu Val Asp Lys Pro Phe
65                  70                  75                  80

Ala Ala Lys Glu Asp Gln Leu Leu Cys Thr Asp Cys Tyr Ser Asn Glu
                85                  90                  95

Tyr Ser Ser Lys Cys Gln Glu Cys Lys Lys Thr Ile Met Pro Gly Thr
            100                 105                 110

Arg Lys Met Glu Tyr Lys Gly Ser Ser Trp His Glu Thr Cys Phe Ile
        115                 120                 125

Cys His Arg Cys Gln Gln Pro Ile Gly Thr Lys Ser Phe Ile Pro Lys
    130                 135                 140

Asp Asn Gln Asn Phe Cys Val Pro Cys Tyr Glu Lys Gln His Ala Met
145                 150                 155                 160

Gln Cys Val Gln Cys Lys Lys Pro Ile Thr Thr Gly Gly Val Thr Tyr
                165                 170                 175

Arg Glu Gln Pro Trp His Lys Glu Cys Phe Val Cys Thr Ala Cys Arg
            180                 185                 190

Lys Gln Leu Ser Gly Gln Arg Phe Thr Ala Arg Asp Asp Phe Ala Tyr
        195                 200                 205

Cys Leu Asn Cys Phe Cys Asp Leu Tyr Ala Lys Lys Cys Ala Gly Cys
    210                 215                 220

Thr Asn Pro Ile Ser Gly Leu Gly Gly Thr Lys Tyr Ile Ser Phe Glu

225                 230                 235                 240

Glu Arg Gln Trp His Asn Asp Cys Phe Asn Cys Lys Lys Cys Ser Leu
                245                 250                 255

Ser Leu Val Gly Arg Gly Phe Leu Thr Glu Arg Asp Asp Ile Leu Cys
            260                 265                 270

Pro Asp Cys Gly Lys Asp Ile
        275

<210>  45
<211>  433
<212>  PRT
<213>  Homo sapiens

<400>  45

Met Arg Ala Glu Gly Leu Gly Gly Leu Glu Arg Phe Cys Ser Pro Gly
1               5                   10                  15

Lys Gly Arg Gly Leu Arg Ala Leu Gln Pro Phe Gln Val Gly Asp Leu
            20                  25                  30

Leu Phe Ser Cys Pro Ala Tyr Ala Tyr Val Leu Thr Val Asn Glu Arg
        35                  40                  45

Gly Asn His Cys Glu Tyr Cys Phe Thr Arg Lys Glu Gly Leu Ser Lys
    50                  55                  60

Cys Gly Arg Cys Lys Gln Ala Phe Tyr Cys Asn Val Glu Cys Gln Lys
65                  70                  75                  80

Glu Asp Trp Pro Met His Lys Leu Glu Cys Ser Pro Met Val Val Phe
                85                  90                  95

Gly Glu Asn Trp Asn Pro Ser Glu Thr Val Arg Leu Thr Ala Arg Ile
            100                 105                 110

Leu Ala Lys Gln Lys Ile His Pro Glu Arg Thr Pro Ser Glu Lys Leu
        115                 120                 125

Leu Ala Val Lys Glu Phe Glu Ser His Leu Asp Lys Leu Asp Asn Glu
    130                 135                 140

Lys Lys Asp Leu Ile Gln Ser Asp Ile Ala Ala Leu His His Phe Tyr
145                 150                 155                 160

Ser Lys His Leu Gly Phe Pro Asp Asn Asp Ser Leu Val Val Leu Phe

                165                 170                 175

Ala Gln Val Asn Cys Asn Gly Phe Thr Ile Glu Asp Glu Glu Leu Ser
            180                 185                 190

His Leu Gly Ser Ala Ile Phe Pro Asp Val Ala Leu Met Asn His Ser
        195                 200                 205

Cys Cys Pro Asn Val Ile Val Thr Tyr Lys Gly Thr Leu Ala Glu Val
    210                 215                 220

Arg Ala Val Gln Glu Ile Lys Pro Gly Glu Glu Val Phe Thr Ser Tyr
225                 230                 235                 240

Ile Asp Leu Leu Tyr Pro Thr Glu Asp Arg Asn Asp Arg Leu Arg Asp
                245                 250                 255

Ser Tyr Phe Phe Thr Cys Glu Cys Gln Glu Cys Thr Thr Lys Asp Lys
            260                 265                 270

Asp Lys Ala Lys Val Glu Ile Arg Lys Leu Ser Asp Pro Pro Lys Ala
        275                 280                 285

Glu Ala Ile Arg Asp Met Val Arg Tyr Ala Arg Asn Val Ile Glu Glu
    290                 295                 300

Phe Arg Arg Ala Lys His Tyr Lys Ser Pro Ser Glu Leu Leu Glu Ile
305                 310                 315                 320

Cys Glu Leu Ser Gln Glu Lys Met Ser Ser Val Phe Glu Asp Ser Asn
                325                 330                 335

Val Tyr Met Leu His Met Met Tyr Gln Ala Met Gly Val Cys Leu Tyr
            340                 345                 350

Met Gln Asp Trp Glu Gly Ala Leu Gln Tyr Gly Gln Lys Ile Ile Lys
        355                 360                 365

Pro Tyr Ser Lys His Tyr Pro Leu Tyr Ser Leu Asn Val Ala Ser Met
    370                 375                 380

Trp Leu Lys Leu Gly Arg Leu Tyr Met Gly Leu Glu His Lys Ala Ala
385                 390                 395                 400

Gly Glu Lys Ala Leu Lys Lys Ala Ile Ala Ile Met Glu Val Ala His
                405                 410                 415

Gly Lys Asp His Pro Tyr Ile Ser Glu Ile Lys Gln Glu Ile Glu Ser
            420                 425                 430

His

<210>  46
<211>  847
<212>  PRT
<213>  Homo sapiens

<400>  46

Met Lys Lys Thr Pro Val Phe Leu Glu Ser Leu Val Thr Asn Met Leu
1               5                   10                  15

Arg Leu Arg Ala Ile Cys Pro Phe Ser Trp Arg Val Phe Gln Phe Arg
            20                  25                  30

Pro Ile Ser Cys Glu Pro Leu Ile Ile Gln Met Asn Lys Cys Thr Asp
        35                  40                  45

Glu Glu Gln Met Phe Gly Phe Ile Glu Arg Asn Lys Ala Ile Leu Ser
    50                  55                  60

Glu Lys Gln Val Gly Cys Ala Phe Asp Met Leu Trp Lys Leu Gln Lys
65                  70                  75                  80

Gln Lys Thr Ser Leu Leu Lys Asn Ala Glu Tyr Val Arg Asp His Pro
                85                  90                  95

Gln Phe Leu Thr Leu His Asn Leu Ala Thr Asn Lys Phe Lys Leu Met
            100                 105                 110

Asn Asp Asp Thr Leu Val Asn Val Leu Tyr Val Thr Gln Gln Phe Ala
        115                 120                 125

Gly Glu Ala His Asp Pro Leu Val Glu Ala Leu Val Thr Glu Ala Trp
    130                 135                 140

Arg Arg Leu Glu Arg Phe Asp Ile Lys Leu Leu Ser Glu Phe Ser Ser
145                 150                 155                 160

Cys Leu Ala Asp Gln His Leu Tyr Phe Ser Pro Leu Met Gly Lys Ile
                165                 170                 175

Ala Asp Ile Val His Arg Asn Leu Glu Thr Thr Gln Asp Leu Ser Ser
            180                 185                 190

Leu Ser Val Leu Met Val Asn Ile Ser Ser Leu Ile Ser Arg His Phe
        195                 200                 205

Gln Gln Gln Leu Val Asn Lys Thr Glu Leu Leu Phe Asp Thr Ile Asp
    210                 215                 220

Ser Ser Glu Val Asn Val Ala Lys Ser Ile Ala Lys Phe Leu Arg Asn
225                 230                 235                 240

Val Arg Tyr Arg Tyr Gln Pro Leu Leu Glu Arg Cys Asn Asn Val Phe
                245                 250                 255

Leu Ser Asn Val Asp His Leu Asp Leu Asp Ser Ile Ser Lys Ile Leu
            260                 265                 270

Ser Val Tyr Lys Phe Leu Gln Phe Asn Ser Phe Glu Phe Ile Ile Met
        275                 280                 285

Ala Lys Lys Lys Leu Thr Glu Met Ile Pro Leu Cys Asn His Pro Ala
    290                 295                 300

Ser Phe Val Lys Leu Phe Val Ala Leu Gly Pro Ile Ala Gly Pro Glu
305                 310                 315                 320

Glu Lys Lys Gln Leu Lys Ser Thr Met Leu Leu Met Ser Glu Asp Leu
                325                 330                 335

Thr Gly Glu Gln Ala Leu Ala Val Leu Gly Ala Met Gly Asp Met Glu
            340                 345                 350

Ser Arg Asn Ser Cys Leu Ile Lys Arg Val Thr Ser Val Leu His Lys
        355                 360                 365

His Leu Asp Gly Tyr Lys Pro Leu Glu Leu Leu Lys Ile Thr Gln Glu
    370                 375                 380

Leu Thr Phe Leu His Phe Gln Arg Lys Glu Phe Phe Ala Lys Leu Arg
385                 390                 395                 400

Glu Leu Leu Leu Ser Tyr Leu Lys Asn Ser Phe Ile Pro Thr Glu Val
                405                 410                 415

Ser Val Leu Val Arg Ala Ile Ser Leu Leu Pro Ser Pro His Leu Asp
            420                 425                 430

Glu Val Gly Ile Ser Arg Ile Glu Ala Val Leu Pro Gln Cys Asp Leu
        435                 440                 445

Asn Asn Leu Ser Ser Phe Ala Thr Ser Val Leu Arg Trp Ile Gln His
    450                 455                 460

Asp His Met Tyr Leu Asp Asn Met Thr Ala Lys Gln Leu Lys Leu Leu
465                 470                 475                 480

Gln Lys Leu Asp His Tyr Gly Arg Gln Arg Leu Gln His Ser Asn Ser
                485                 490                 495

Leu Asp Leu Leu Arg Lys Glu Leu Lys Ser Leu Lys Gly Asn Thr Phe
            500                 505                 510

Pro Glu Ser Leu Leu Glu Glu Met Ile Ala Thr Leu Gln His Phe Met
        515                 520                 525

Asp Asp Ile Asn Tyr Ile Asn Val Gly Glu Ile Ala Ser Phe Ile Ser
    530                 535                 540

Ser Thr Asp Tyr Leu Ser Thr Leu Leu Leu Asp Arg Ile Ala Ser Val
545                 550                 555                 560

Ala Val Gln Gln Ile Glu Lys Ile His Pro Phe Thr Ile Pro Ala Ile
                565                 570                 575

Ile Arg Pro Phe Ser Val Leu Asn Tyr Asp Pro Pro Gln Arg Asp Glu
            580                 585                 590

Phe Leu Gly Thr Cys Val Gln His Leu Asn Ser Tyr Leu Gly Ile Leu
        595                 600                 605

Asp Pro Phe Ile Leu Val Phe Leu Gly Phe Ser Leu Ala Thr Leu Glu
    610                 615                 620

Tyr Phe Pro Glu Asp Leu Leu Lys Ala Ile Phe Asn Ile Lys Phe Leu
625                 630                 635                 640

Ala Arg Leu Asp Ser Gln Leu Glu Ile Leu Ser Pro Ser Arg Ser Ala
                645                 650                 655

Arg Val Gln Phe His Leu Met Glu Leu Asn Arg Ser Val Cys Leu Glu
            660                 665                 670

Cys Pro Glu Phe Gln Ile Pro Trp Phe His Asp Arg Phe Cys Gln Gln
        675                 680                 685

Tyr Asn Lys Gly Ile Gly Gly Met Asp Gly Thr Gln Gln Gln Ile Phe
    690                 695                 700

Lys Met Leu Ala Glu Val Leu Gly Gly Ile Asn Cys Val Lys Ala Ser
705                 710                 715                 720

Val Leu Thr Pro Tyr Tyr His Lys Val Asp Phe Glu Cys Ile Leu Asp
                725                 730                 735

Lys Arg Lys Lys Pro Leu Pro Tyr Gly Ser His Asn Ile Ala Leu Gly
            740                 745                 750

Gln Leu Pro Glu Met Pro Trp Glu Ser Asn Ile Glu Ile Val Gly Ser
        755                 760                 765

Arg Leu Pro Pro Gly Ala Glu Arg Ile Ala Leu Glu Phe Leu Asp Ser
    770                 775                 780

Lys Ala Leu Cys Arg Asn Ile Pro His Met Lys Gly Lys Ser Ala Met
785                 790                 795                 800

Lys Lys Arg His Leu Glu Ile Leu Gly Tyr Arg Val Ile Gln Ile Ser
                805                 810                 815

Gln Phe Glu Trp Asn Ser Met Ala Leu Ser Thr Lys Asp Ala Arg Met
            820                 825                 830

Asp Tyr Leu Arg Glu Cys Ile Phe Gly Glu Val Lys Ser Cys Leu
        835                 840                 845

<210>  47
<211>  331
<212>  PRT
<213>  Homo sapiens

<400>  47

Met Gly Pro Gln Val Val Ala Ser Ala Pro Glu Pro Thr Arg Pro Pro
1               5                   10                  15

Ser Gly Phe Val Pro Val Ser Gly Gly Gly Gly Thr His Val Thr Gln
            20                  25                  30

Val His Ile Gln Leu Ala Pro Ser Pro His Asn Gly Thr Pro Glu Pro
        35                  40                  45

Pro Arg Thr Ala Pro Glu Val Gly Ser Asn Ser Gln Asp Gly Asp Ala
    50                  55                  60

Thr Pro Ser Pro Pro Arg Ala Gln Pro Leu Val Pro Val Ala His Ile
65                  70                  75                  80

Arg Pro Leu Pro Thr Thr Val Gln Ala Ala Ser Pro Leu Pro Glu Glu
                85                  90                  95

Pro Pro Val Pro Arg Pro Pro Pro Gly Phe Gln Ala Ser Val Pro Arg
            100                 105                 110

Glu Ala Ser Ala Arg Val Val Val Pro Ile Ala Pro Thr Cys Arg Ser
        115                 120                 125

Leu Glu Ser Ser Pro His Ser Leu Val Pro Met Gly Pro Gly Arg Glu
    130                 135                 140

His Leu Glu Glu Pro Pro Met Ala Gly Pro Ala Ala Glu Ala Glu Arg
145                 150                 155                 160

Val Ser Ser Pro Ala Trp Ala Ser Ser Pro Thr Pro Pro Ser Gly Pro
                165                 170                 175

His Pro Cys Pro Val Pro Lys Val Ala Pro Lys Pro Arg Leu Ser Gly
            180                 185                 190

Trp Thr Trp Leu Lys Lys Gln Leu Leu Glu Glu Ala Pro Glu Pro Pro
        195                 200                 205

Cys Pro Glu Pro Arg Gln Ser Leu Glu Pro Glu Val Pro Thr Pro Thr
    210                 215                 220

Glu Gln Glu Val Pro Ala Pro Thr Glu Gln Glu Val Pro Ala Leu Thr
225                 230                 235                 240

Ala Pro Arg Ala Pro Ala Ser Arg Thr Ser Arg Met Trp Asp Ala Val
                245                 250                 255

Leu Tyr Arg Met Ser Val Ala Glu Ala Gln Gly Arg Leu Ala Gly Pro
            260                 265                 270

Ser Gly Gly Glu His Thr Pro Ala Ser Leu Thr Arg Leu Pro Phe Leu
        275                 280                 285

Tyr Arg Pro Arg Phe Asn Ala Arg Lys Leu Gln Glu Ala Thr Arg Pro
    290                 295                 300

Pro Pro Thr Val Arg Ser Ile Leu Glu Leu Ser Pro Gln Pro Lys Asn
305                 310                 315                 320

Phe Asn Arg Thr Ala Thr Gly Trp Arg Leu Gln

325                 330

<210>  48
<211>  1100
<212>  PRT
<213>  Homo sapiens

<400>  48

Met Ser Tyr Tyr Gln Arg Pro Phe Ser Pro Ser Ala Tyr Ser Leu Pro
1               5                   10                  15

Ala Ser Leu Asn Ser Ser Ile Val Met Gln His Gly Thr Ser Leu Asp
            20                  25                  30

Ser Thr Asp Thr Tyr Pro Gln His Ala Gln Ser Leu Asp Gly Thr Thr
        35                  40                  45

Ser Ser Ser Ile Pro Leu Tyr Arg Ser Ser Glu Glu Glu Lys Arg Val
    50                  55                  60

Thr Val Ile Lys Ala Pro His Tyr Pro Gly Ile Gly Pro Val Asp Glu
65                  70                  75                  80

Ser Gly Ile Pro Thr Ala Ile Arg Thr Thr Val Asp Arg Pro Lys Asp
                85                  90                  95

Trp Tyr Lys Thr Met Phe Lys Gln Ile His Met Val His Lys Pro Asp
            100                 105                 110

Asp Asp Thr Asp Met Tyr Asn Thr Pro Tyr Thr Tyr Asn Ala Gly Leu
        115                 120                 125

Tyr Asn Pro Pro Tyr Ser Ala Gln Ser His Pro Ala Ala Lys Thr Gln
    130                 135                 140

Thr Tyr Arg Pro Leu Ser Lys Ser His Ser Asp Asn Ser Pro Asn Ala
145                 150                 155                 160

Phe Lys Asp Ala Ser Ser Pro Val Pro Pro Pro His Val Pro Pro Pro
                165                 170                 175

Val Pro Pro Leu Arg Pro Arg Asp Arg Ser Ser Thr Glu Lys His Asp
            180                 185                 190

Trp Asp Pro Pro Asp Arg Lys Val Asp Thr Arg Lys Phe Arg Ser Glu
        195                 200                 205

Pro Arg Ser Ile Phe Glu Tyr Glu Pro Gly Lys Ser Ser Ile Leu Gln

210                 215                 220

His Glu Arg Pro Ala Ser Leu Tyr Gln Ser Ser Ile Asp Arg Ser Leu
225                 230                 235                 240

Glu Arg Pro Met Ser Ser Ala Ser Met Ala Ser Asp Phe Arg Lys Arg
                245                 250                 255

Arg Lys Ser Glu Pro Ala Val Gly Pro Pro Arg Gly Leu Gly Asp Gln
            260                 265                 270

Ser Ala Ser Arg Thr Ser Pro Gly Arg Val Asp Leu Pro Gly Ser Ser
        275                 280                 285

Thr Thr Leu Thr Lys Ser Phe Thr Ser Ser Ser Pro Ser Ser Pro Ser
    290                 295                 300

Arg Ala Lys Gly Gly Asp Asp Ser Lys Ile Cys Pro Ser Leu Cys Ser
305                 310                 315                 320

Tyr Ser Gly Leu Asn Gly Asn Pro Ser Ser Glu Leu Asp Tyr Cys Ser
                325                 330                 335

Thr Tyr Arg Gln His Leu Asp Val Pro Arg Asp Ser Pro Arg Ala Ile
            340                 345                 350

Ser Phe Lys Asn Gly Trp Gln Met Ala Arg Gln Asn Ala Glu Ile Trp
        355                 360                 365

Ser Ser Thr Glu Glu Thr Val Ser Pro Lys Ile Lys Ser Arg Ser Cys
    370                 375                 380

Asp Asp Leu Leu Asn Asp Asp Cys Asp Ser Phe Pro Asp Pro Lys Val
385                 390                 395                 400

Lys Ser Glu Ser Met Gly Ser Leu Leu Cys Glu Glu Asp Ser Lys Glu
                405                 410                 415

Ser Cys Pro Met Ala Trp Gly Ser Pro Tyr Val Pro Glu Val Arg Ser
            420                 425                 430

Asn Gly Arg Ser Arg Ile Arg His Arg Ser Ala Arg Asn Ala Pro Gly
        435                 440                 445

Phe Leu Lys Met Tyr Lys Lys Met His Arg Ile Asn Arg Lys Asp Leu
    450                 455                 460

Met Asn Ser Glu Val Ile Cys Ser Val Lys Ser Arg Ile Leu Gln Tyr
465                 470                 475                 480

Glu Ser Glu Gln Gln His Lys Asp Leu Leu Arg Ala Trp Ser Gln Cys
                485                 490                 495

Ser Thr Glu Glu Val Pro Arg Asp Met Val Pro Thr Arg Ile Ser Glu
            500                 505                 510

Phe Glu Lys Leu Ile Gln Lys Ser Lys Ser Met Pro Asn Leu Gly Asp
        515                 520                 525

Asp Met Leu Ser Pro Val Thr Leu Glu Pro Pro Gln Asn Gly Leu Cys
    530                 535                 540

Pro Lys Arg Arg Phe Ser Ile Glu Tyr Leu Leu Glu Glu Glu Asn Gln
545                 550                 555                 560

Ser Gly Pro Pro Ala Arg Gly Arg Arg Gly Cys Gln Ser Asn Ala Leu
                565                 570                 575

Val Pro Ile His Ile Glu Val Thr Ser Asp Glu Gln Pro Arg Ala His
            580                 585                 590

Val Glu Phe Ser Asp Ser Asp Gln Asp Gly Val Val Ser Asp His Ser
        595                 600                 605

Asp Tyr Ile His Leu Glu Gly Ser Ser Phe Cys Ser Glu Ser Asp Phe
    610                 615                 620

Asp His Phe Ser Phe Thr Ser Ser Glu Ser Phe Tyr Gly Ser Ser His
625                 630                 635                 640

His His His His His His His His His His Arg His Leu Ile Ser Ser
                645                 650                 655

Cys Lys Gly Arg Cys Pro Ala Ser Tyr Thr Arg Phe Thr Thr Met Leu
            660                 665                 670

Lys His Glu Arg Ala Arg His Glu Asn Thr Glu Glu Pro Arg Arg Gln
        675                 680                 685

Glu Met Asp Pro Gly Leu Ser Lys Leu Ala Phe Leu Val Ser Pro Val
    690                 695                 700

Pro Phe Arg Arg Lys Lys Asn Ser Ala Pro Lys Lys Gln Thr Glu Lys
705                 710                 715                 720

Ala Lys Cys Lys Ala Ser Val Phe Glu Ala Leu Asp Ser Ala Leu Lys
                725                 730                 735

Asp Ile Cys Asp Gln Ile Lys Ala Glu Lys Lys Arg Gly Ser Leu Pro
            740                 745                 750

Asp Asn Ser Ile Leu His Arg Leu Ile Ser Glu Leu Leu Pro Asp Val
        755                 760                 765

Pro Glu Arg Asn Ser Ser Leu Arg Ala Leu Arg Arg Ser Pro Leu His
    770                 775                 780

Gln Pro Leu His Pro Leu Pro Pro Asp Gly Ala Ile His Cys Pro Pro
785                 790                 795                 800

Tyr Gln Asn Asp Cys Gly Arg Met Pro Arg Ser Ala Ser Phe Gln Asp
                805                 810                 815

Val Asp Thr Ala Asn Ser Ser Cys His His Gln Asp Arg Gly Gly Ala
            820                 825                 830

Leu Gln Asp Arg Glu Ser Pro Arg Ser Tyr Ser Ser Thr Leu Thr Asp
        835                 840                 845

Met Gly Arg Ser Ala Pro Arg Glu Arg Arg Gly Thr Pro Glu Lys Glu
    850                 855                 860

Lys Leu Pro Ala Lys Ala Val Tyr Asp Phe Lys Ala Gln Thr Ser Lys
865                 870                 875                 880

Glu Leu Ser Phe Lys Lys Gly Asp Thr Val Tyr Ile Leu Arg Lys Ile
                885                 890                 895

Asp Gln Asn Trp Tyr Glu Gly Glu His His Gly Arg Val Gly Ile Phe
            900                 905                 910

Pro Ile Ser Tyr Val Glu Lys Leu Thr Pro Pro Glu Lys Ala Gln Pro
        915                 920                 925

Ala Arg Pro Pro Pro Pro Ala Gln Pro Gly Glu Ile Gly Glu Ala Ile
    930                 935                 940

Ala Lys Tyr Asn Phe Asn Ala Asp Thr Asn Val Glu Leu Ser Leu Arg
945                 950                 955                 960

Lys Gly Asp Arg Val Ile Leu Leu Lys Arg Val Asp Gln Asn Trp Tyr
                965                 970                 975

Glu Gly Lys Ile Pro Gly Thr Asn Arg Gln Gly Ile Phe Pro Val Ser
            980                 985                 990

Tyr Val Glu Val Val Lys Lys Asn Thr Lys Gly Ala Glu Asp Tyr Pro
        995                 1000                1005

Asp Pro Pro Ile Pro His Ser Tyr Ser Ser Asp Arg Ile His Ser
    1010                1015                1020

Leu Ser Ser Asn Lys Pro Gln Arg Pro Val Phe Thr His Glu Asn
    1025                1030                1035

Ile Gln Gly Gly Gly Glu Pro Phe Gln Ala Leu Tyr Asn Tyr Thr
    1040                1045                1050

Pro Arg Asn Glu Asp Glu Leu Glu Leu Arg Glu Ser Asp Val Ile
    1055                1060                1065

Asp Val Met Glu Lys Cys Asp Asp Gly Trp Phe Val Gly Thr Ser
    1070                1075                1080

Arg Arg Thr Lys Phe Phe Gly Thr Phe Pro Gly Asn Tyr Val Lys
    1085                1090                1095

Arg Leu
    1100

<210> 49
<211> 500
<212> PRT
<213> Homo sapiens

<400> 49

Met Lys Cys Thr Ala Arg Glu Trp Leu Arg Val Thr Thr Val Leu Phe
1               5                   10                  15

Met Ala Arg Ala Ile Pro Ala Met Val Val Pro Asn Ala Thr Leu Leu
            20                  25                  30

Glu Lys Leu Leu Glu Lys Tyr Met Asp Glu Asp Gly Glu Trp Trp Ile
        35                  40                  45

Ala Lys Gln Arg Gly Lys Arg Ala Ile Thr Asp Asn Asp Met Gln Ser
    50                  55                  60

Ile Leu Asp Leu His Asn Lys Leu Arg Ser Gln Val Tyr Pro Thr Ala
65                  70                  75                  80

Ser Asn Met Glu Tyr Met Thr Trp Asp Val Glu Leu Glu Arg Ser Ala
                85                  90                  95

Glu Ser Trp Ala Glu Ser Cys Leu Trp Glu His Gly Pro Ala Ser Leu
            100                 105                 110

Leu Pro Ser Ile Gly Gln Asn Leu Gly Ala His Trp Gly Arg Tyr Arg
        115                 120                 125

Pro Pro Thr Phe His Val Gln Ser Trp Tyr Asp Glu Val Lys Asp Phe
    130                 135                 140

Ser Tyr Pro Tyr Glu His Glu Cys Asn Pro Tyr Cys Pro Phe Arg Cys
145                 150                 155                 160

Ser Gly Pro Val Cys Thr His Tyr Thr Gln Val Val Trp Ala Thr Ser
                165                 170                 175

Asn Arg Ile Gly Cys Ala Ile Asn Leu Cys His Asn Met Asn Ile Trp
            180                 185                 190

Gly Gln Ile Trp Pro Lys Ala Val Tyr Leu Val Cys Asn Tyr Ser Pro
        195                 200                 205

Lys Gly Asn Trp Trp Gly His Ala Pro Tyr Lys His Gly Arg Pro Cys
    210                 215                 220

Ser Ala Cys Pro Pro Ser Phe Gly Gly Gly Cys Arg Glu Asn Leu Cys
225                 230                 235                 240

Tyr Lys Glu Gly Ser Asp Arg Tyr Tyr Pro Pro Arg Glu Glu Glu Thr
                245                 250                 255

Asn Glu Ile Glu Arg Gln Gln Ser Gln Val His Asp Thr His Val Arg
            260                 265                 270

Thr Arg Ser Asp Asp Ser Ser Arg Asn Glu Val Ile Ser Ala Gln Gln
        275                 280                 285

Met Ser Gln Ile Val Ser Cys Glu Val Arg Leu Arg Asp Gln Cys Lys
    290                 295                 300

Gly Thr Thr Cys Asn Arg Tyr Glu Cys Pro Ala Gly Cys Leu Asp Ser
305                 310                 315                 320

Lys Ala Lys Val Ile Gly Ser Val His Tyr Glu Met Gln Ser Ser Ile

325                 330                 335

Cys Arg Ala Ala Ile His Tyr Gly Ile Ile Asp Asn Asp Gly Gly Trp
            340                 345                 350

Val Asp Ile Thr Arg Gln Gly Arg Lys His Tyr Phe Ile Lys Ser Asn
        355                 360                 365

Arg Asn Gly Ile Gln Thr Ile Gly Lys Tyr Gln Ser Ala Asn Ser Phe
    370                 375                 380

Thr Val Ser Lys Val Thr Val Gln Ala Val Thr Cys Glu Thr Thr Val
385                 390                 395                 400

Glu Gln Leu Cys Pro Phe His Lys Pro Ala Ser His Cys Pro Arg Val
                405                 410                 415

Tyr Cys Pro Arg Asn Cys Met Gln Ala Asn Pro His Tyr Ala Arg Val
            420                 425                 430

Ile Gly Thr Arg Val Tyr Ser Asp Leu Ser Ser Ile Cys Arg Ala Ala
        435                 440                 445

Val His Ala Gly Val Val Arg Asn His Gly Gly Tyr Val Asp Val Met
    450                 455                 460

Pro Val Asp Lys Arg Lys Thr Tyr Ile Ala Ser Phe Gln Asn Gly Ile
465                 470                 475                 480

Phe Ser Glu Ser Leu Gln Asn Pro Pro Gly Gly Lys Ala Phe Arg Val
                485                 490                 495

Phe Ala Val Val
            500

<210>  50
<211>  1426
<212>  PRT
<213>  Homo sapiens

<400>  50

<210> 51
<211> 360
<212> PRT
<213> Homo sapiens

<400> 51

Met Ala Lys Ala Pro Ser Trp Ala Gly Val Gly Ala Leu Ala Tyr Lys
1               5                   10                  15

Ala Pro Glu Ala Leu Trp Pro Ala Glu Ala Val Met Asp Gly Thr Met
            20                  25                  30

Glu Asp Ser Glu Ala Val Gln Arg Ala Thr Ala Leu Ile Glu Gln Arg
        35                  40                  45

Leu Ala Gln Glu Glu Glu Asn Glu Lys Leu Arg Gly Asp Ala Arg Gln
    50                  55                  60

Lys Leu Pro Met Asp Leu Leu Val Leu Glu Asp Glu Lys His His Gly
65                  70                  75                  80

Ala Gln Ser Ala Ala Leu Gln Lys Val Lys Gly Gln Glu Arg Val Arg
                85                  90                  95

Lys Thr Ser Leu Asp Leu Arg Arg Glu Ile Ile Asp Val Gly Gly Ile
            100                 105                 110

Gln Asn Leu Ile Glu Leu Arg Lys Lys Arg Lys Gln Lys Lys Arg Asp
        115                 120                 125

Ala Leu Ala Ala Ser His Glu Pro Pro Pro Glu Pro Glu Glu Ile Thr
    130                 135                 140

Gly Pro Val Asp Glu Glu Thr Phe Leu Lys Ala Ala Val Glu Gly Lys
145                 150                 155                 160

Met Lys Val Ile Glu Lys Phe Leu Ala Asp Gly Gly Ser Ala Asp Thr
                165                 170                 175

Cys Asp Gln Phe Arg Arg Thr Ala Leu His Arg Ala Ser Leu Glu Gly
            180                 185                 190

His Met Glu Ile Leu Glu Lys Leu Leu Asp Asn Gly Ala Thr Val Asp
        195                 200                 205

Phe Gln Asp Arg Leu Asp Cys Thr Ala Met His Trp Ala Cys Arg Gly
    210                 215                 220

Gly His Leu Glu Val Val Lys Leu Leu Gln Ser His Gly Ala Asp Thr
225                 230                 235                 240

Asn Val Arg Asp Lys Leu Leu Ser Thr Pro Leu His Val Ala Val Arg
                245                 250                 255

Thr Gly Gln Val Glu Ile Val Glu His Phe Leu Ser Leu Gly Leu Glu
            260                 265                 270

Ile Asn Ala Arg Asp Arg Glu Gly Asp Thr Ala Leu His Asp Ala Val
        275                 280                 285

Arg Leu Asn Arg Tyr Lys Ile Ile Lys Leu Leu Leu Leu His Gly Ala
    290                 295                 300

Asp Met Met Thr Lys Asn Leu Ala Gly Lys Thr Pro Thr Asp Leu Val
305                 310                 315                 320

Gln Leu Trp Gln Ala Asp Thr Arg His Ala Leu Glu His Pro Glu Pro
                325                 330                 335

Gly Ala Glu His Asn Gly Leu Glu Gly Pro Asn Asp Ser Gly Arg Glu
            340                 345                 350

Thr Pro Gln Pro Val Pro Ala Gln
        355                 360

<210>  52
<211>  161
<212>  PRT
<213>  Homo sapiens

<400>  52

Met Ala Lys Glu Glu Pro Gln Ser Ile Ser Arg Asp Leu Gln Glu Leu
1               5                   10                  15

Gln Lys Lys Leu Ser Leu Leu Ile Asp Ser Phe Gln Asn Asn Ser Lys
            20                  25                  30

Val Val Ala Phe Met Lys Ser Pro Val Gly Gln Tyr Leu Asp Ser His
        35                  40                  45

Pro Phe Leu Ala Phe Thr Leu Leu Val Phe Ile Val Met Ser Ala Val
    50                  55                  60

Pro Val Gly Phe Phe Leu Leu Ile Val Val Leu Thr Thr Leu Ala Ala
65                  70                  75                  80

Leu Leu Gly Val Ile Ile Leu Glu Gly Leu Val Ile Ser Val Gly Gly
                85                  90                  95

Phe Ser Leu Leu Cys Ile Leu Cys Gly Leu Gly Phe Val Ser Leu Ala
            100                 105                 110

Met Ser Gly Met Met Ile Ala Ser Tyr Val Val Val Ser Ser Leu Ile
        115                 120                 125

Ser Cys Trp Phe Ser Pro Arg Pro Leu Thr Gln Gln Asn Thr Ser Cys
    130                 135                 140

Asp Phe Leu Pro Ala Met Lys Ser Ala Glu Phe Glu Gly Leu Tyr Gln
145                 150                 155                 160

Glu

<210>  53
<211>  1500
<212>  PRT
<213>  Homo sapiens

<400>  53

Met Asp Pro Ser Met Gly Val Asn Ser Val Thr Ile Ser Val Glu Gly
1               5                   10                  15

Met Thr Cys Asn Ser Cys Val Trp Thr Ile Glu Gln Gln Ile Gly Lys
            20                  25                  30

Val Asn Gly Val His His Ile Lys Val Ser Leu Glu Glu Lys Asn Ala
        35                  40                  45

Thr Ile Ile Tyr Asp Pro Lys Leu Gln Thr Pro Lys Thr Leu Gln Glu
    50                  55                  60

Ala Ile Asp Asp Met Gly Phe Asp Ala Val Ile His Asn Pro Asp Pro
65                  70                  75                  80

Leu Pro Val Leu Thr Asp Thr Leu Phe Leu Thr Val Thr Ala Ser Leu
                85                  90                  95

Thr Leu Pro Trp Asp His Ile Gln Ser Thr Leu Leu Lys Thr Lys Gly
            100                 105                 110

Val Thr Asp Ile Lys Ile Tyr Pro Gln Lys Arg Thr Val Ala Val Thr
        115                 120                 125

Ile Ile Pro Ser Ile Val Asn Ala Asn Gln Ile Lys Glu Leu Val Pro
    130                 135                 140

Glu Leu Ser Leu Asp Thr Gly Thr Leu Glu Lys Lys Ser Gly Ala Cys
145                 150                 155                 160

Glu Asp His Ser Met Ala Gln Ala Gly Glu Val Val Leu Lys Met Lys
                165                 170                 175

Val Glu Gly Met Thr Cys His Ser Cys Thr Ser Thr Ile Glu Gly Lys
            180                 185                 190

Ile Gly Lys Leu Gln Gly Val Gln Arg Ile Lys Val Ser Leu Asp Asn
        195                 200                 205

Gln Glu Ala Thr Ile Val Tyr Gln Pro His Leu Ile Ser Val Glu Glu
    210                 215                 220

Met Lys Lys Gln Ile Glu Ala Met Gly Phe Pro Ala Phe Val Lys Lys
225                 230                 235                 240

Gln Pro Lys Tyr Leu Lys Leu Gly Ala Ile Asp Val Glu Arg Leu Lys
                245                 250                 255

Asn Thr Pro Val Lys Ser Ser Glu Gly Ser Gln Gln Arg Ser Pro Ser
            260                 265                 270

Tyr Thr Asn Asp Ser Thr Ala Thr Phe Ile Ile Asp Gly Met His Cys
        275                 280                 285

Lys Ser Cys Val Ser Asn Ile Glu Ser Thr Leu Ser Ala Leu Gln Tyr
    290                 295                 300

Val Ser Ser Ile Val Val Ser Leu Glu Asn Arg Ser Ala Ile Val Lys
305                 310                 315                 320

Tyr Asn Ala Ser Ser Val Thr Pro Glu Ser Leu Arg Lys Ala Ile Glu
                325                 330                 335

Ala Val Ser Pro Gly Leu Tyr Arg Val Ser Ile Thr Ser Glu Val Glu
            340                 345                 350

Ser Thr Ser Asn Ser Pro Ser Ser Ser Ser Leu Gln Lys Ile Pro Leu
        355                 360                 365

Asn Val Val Ser Gln Pro Leu Thr Gln Glu Thr Val Ile Asn Ile Asp
    370                 375                 380

Gly Met Thr Cys Asn Ser Cys Val Gln Ser Ile Glu Gly Val Ile Ser
385                 390                 395                 400

Lys Lys Pro Gly Val Lys Ser Ile Arg Val Ser Leu Ala Asn Ser Asn
                405                 410                 415

Gly Thr Val Glu Tyr Asp Pro Leu Leu Thr Ser Pro Glu Thr Leu Arg
            420                 425                 430

Gly Ala Ile Glu Asp Met Gly Phe Asp Ala Thr Leu Ser Asp Thr Asn
        435                 440                 445

Glu Pro Leu Val Val Ile Ala Gln Pro Ser Ser Glu Met Pro Leu Leu
    450                 455                 460

Thr Ser Thr Asn Glu Phe Tyr Thr Lys Gly Met Thr Pro Val Gln Asp
465                 470                 475                 480

Lys Glu Glu Gly Lys Asn Ser Ser Lys Cys Tyr Ile Gln Val Thr Gly
                485                 490                 495

Met Thr Cys Ala Ser Cys Val Ala Asn Ile Glu Arg Asn Leu Arg Arg
            500                 505                 510

Glu Glu Gly Ile Tyr Ser Ile Leu Val Ala Leu Met Ala Gly Lys Ala
        515                 520                 525

Glu Val Arg Tyr Asn Pro Ala Val Ile Gln Pro Pro Met Ile Ala Glu
    530                 535                 540

Phe Ile Arg Glu Leu Gly Phe Gly Ala Thr Val Ile Glu Asn Ala Asp
545                 550                 555                 560

Glu Gly Asp Gly Val Leu Glu Leu Val Val Arg Gly Met Thr Cys Ala
                565                 570                 575

Ser Cys Val His Lys Ile Glu Ser Ser Leu Thr Lys His Arg Gly Ile
            580                 585                 590

Leu Tyr Cys Ser Val Ala Leu Ala Thr Asn Lys Ala His Ile Lys Tyr
        595                 600                 605

Asp Pro Glu Ile Ile Gly Pro Arg Asp Ile Ile His Thr Ile Glu Ser
    610                 615                 620

Leu Gly Phe Glu Ala Ser Leu Val Lys Lys Asp Arg Ser Ala Ser His
625                 630                 635                 640

Leu Asp His Lys Arg Glu Ile Arg Gln Trp Arg Arg Ser Phe Leu Val
                645                 650                 655

Ser Leu Phe Phe Cys Ile Pro Val Met Gly Leu Met Ile Tyr Met Met
            660                 665                 670

Val Met Asp His His Phe Ala Thr Leu His His Asn Gln Asn Met Ser
        675                 680                 685

Lys Glu Glu Met Ile Asn Leu His Ser Ser Met Phe Leu Glu Arg Gln
    690                 695                 700

Ile Leu Pro Gly Leu Ser Val Met Asn Leu Leu Ser Phe Leu Leu Cys
705                 710                 715                 720

Val Pro Val Gln Phe Phe Gly Gly Trp Tyr Phe Tyr Ile Gln Ala Tyr

725                 730                 735

Lys Ala Leu Lys His Lys Thr Ala Asn Met Asp Val Leu Ile Val Leu
        740                 745                 750

Ala Thr Thr Ile Ala Phe Ala Tyr Ser Leu Ile Ile Leu Leu Val Ala
        755                 760                 765

Met Tyr Glu Arg Ala Lys Val Asn Pro Ile Thr Phe Phe Asp Thr Pro
    770                 775                 780

Pro Met Leu Phe Val Phe Ile Ala Leu Gly Arg Trp Leu Glu His Ile
785                 790                 795                 800

Ala Lys Gly Lys Thr Ser Glu Ala Leu Ala Lys Leu Ile Ser Leu Gln
                805                 810                 815

Ala Thr Glu Ala Thr Ile Val Thr Leu Asp Ser Asp Asn Ile Leu Leu
            820                 825                 830

Ser Glu Glu Gln Val Asp Val Glu Leu Val Gln Arg Gly Asp Ile Ile
        835                 840                 845

Lys Val Val Pro Gly Gly Lys Phe Pro Val Asp Gly Arg Val Ile Glu
    850                 855                 860

Gly His Ser Met Val Asp Glu Ser Leu Ile Thr Gly Glu Ala Met Pro
865                 870                 875                 880

Val Ala Lys Lys Pro Gly Ser Thr Val Ile Ala Gly Ser Ile Asn Gln
                885                 890                 895

Asn Gly Ser Leu Leu Ile Cys Ala Thr His Val Gly Ala Asp Thr Thr
            900                 905                 910

Leu Ser Gln Ile Val Lys Leu Val Glu Glu Ala Gln Thr Ser Lys Ala
        915                 920                 925

Pro Ile Gln Gln Phe Ala Asp Lys Leu Ser Gly Tyr Phe Val Pro Phe
    930                 935                 940

Ile Val Phe Val Ser Ile Ala Thr Leu Leu Val Trp Ile Val Ile Gly
945                 950                 955                 960

Phe Leu Asn Phe Glu Ile Val Glu Thr Tyr Phe Pro Gly Tyr Asn Arg
                965                 970                 975

Ser Ile Ser Arg Thr Glu Thr Ile Ile Arg Phe Ala Phe Gln Ala Ser
            980                 985                 990

Ile Thr Val Leu Cys Ile Ala Cys  Pro Cys Ser Leu Gly  Leu Ala Thr
        995                 1000                 1005

Pro Thr  Ala Val Met Val Gly  Thr Gly Val Gly Ala  Gln Asn Gly
    1010                 1015                 1020

Ile Leu  Ile Lys Gly Gly Glu  Pro Leu Glu Met Ala  His Lys Val
    1025                 1030                 1035

Lys Val  Val Val Phe Asp Lys  Thr Gly Thr Ile Thr  His Gly Thr
    1040                 1045                 1050

Pro Val  Val Asn Gln Val Lys  Val Leu Thr Glu Ser  Asn Arg Ile
    1055                 1060                 1065

Ser His  His Lys Ile Leu Ala  Ile Val Gly Thr Ala  Glu Ser Asn
    1070                 1075                 1080

Ser Glu  His Pro Leu Gly Thr  Ala Ile Thr Lys Tyr  Cys Lys Gln
    1085                 1090                 1095

Glu Leu  Asp Thr Glu Thr Leu  Gly Thr Cys Ile Asp  Phe Gln Val
    1100                 1105                 1110

Val Pro  Gly Cys Gly Ile Ser  Cys Lys Val Thr Asn  Ile Glu Gly
    1115                 1120                 1125

Leu Leu  His Lys Asn Asn Trp  Asn Ile Glu Asp Asn  Asn Ile Lys
    1130                 1135                 1140

Asn Ala  Ser Leu Val Gln Ile  Asp Ala Ser Asn Glu  Gln Ser Ser
    1145                 1150                 1155

Thr Ser  Ser Ser Met Ile Ile  Asp Ala Gln Ile Ser  Asn Ala Leu
    1160                 1165                 1170

Asn Ala  Gln Gln Tyr Lys Val  Leu Ile Gly Asn Arg  Glu Trp Met
    1175                 1180                 1185

Ile Arg  Asn Gly Leu Val Ile  Asn Asn Asp Val Asn  Asp Phe Met
    1190                 1195                 1200

Thr Glu  His Glu Arg Lys Gly  Arg Thr Ala Val Leu  Val Ala Val
    1205                 1210                 1215

Asp Asp Glu Leu Cys Gly Leu Ile Ala Ile Ala Asp Thr Val Lys
    1220                1225                1230

Pro Glu Ala Glu Leu Ala Ile His Ile Leu Lys Ser Met Gly Leu
    1235                1240                1245

Glu Val Val Leu Met Thr Gly Asp Asn Ser Lys Thr Ala Arg Ser
    1250                1255                1260

Ile Ala Ser Gln Val Gly Ile Thr Lys Val Phe Ala Glu Val Leu
    1265                1270                1275

Pro Ser His Lys Val Ala Lys Val Lys Gln Leu Gln Glu Glu Gly
    1280                1285                1290

Lys Arg Val Ala Met Val Gly Asp Gly Ile Asn Asp Ser Pro Ala
    1295                1300                1305

Leu Ala Met Ala Asn Val Gly Ile Ala Ile Gly Thr Gly Thr Asp
    1310                1315                1320

Val Ala Ile Glu Ala Ala Asp Val Val Leu Ile Arg Asn Asp Leu
    1325                1330                1335

Leu Asp Val Val Ala Ser Ile Asp Leu Ser Arg Glu Thr Val Lys
    1340                1345                1350

Arg Ile Arg Ile Asn Phe Val Phe Ala Leu Ile Tyr Asn Leu Val
    1355                1360                1365

Gly Ile Pro Ile Ala Ala Gly Val Phe Met Pro Ile Gly Leu Val
    1370                1375                1380

Leu Gln Pro Trp Met Gly Ser Ala Ala Met Ala Ala Ser Ser Val
    1385                1390                1395

Ser Val Val Leu Ser Ser Leu Phe Leu Lys Leu Tyr Arg Lys Pro
    1400                1405                1410

Thr Tyr Glu Ser Tyr Glu Leu Pro Ala Arg Ser Gln Ile Gly Gln
    1415                1420                1425

Lys Ser Pro Ser Glu Ile Ser Val His Val Gly Ile Asp Asp Thr
    1430                1435                1440

Ser Arg Asn Ser Pro Lys Leu Gly Leu Leu Asp Arg Ile Val Asn
    1445                1450                1455

Tyr Ser Arg Ala Ser Ile Asn Ser Leu Leu Ser Asp Lys Arg Ser
    1460                1465                1470

Leu Asn Ser Val Val Thr Ser Glu Pro Asp Lys His Ser Leu Leu
    1475                1480                1485

Val Gly Asp Phe Arg Glu Asp Asp Asp Thr Ala Leu
    1490                1495                1500

<210> 54
<211> 71
<212> PRT
<213> Homo sapiens

<400> 54

Met Pro Ala Gly Val Pro Met Ser Thr Tyr Leu Lys Met Phe Ala Ala
1               5                   10                  15

Ser Leu Leu Ala Met Cys Ala Gly Ala Glu Val Val His Arg Tyr Tyr
            20                  25                  30

Arg Pro Asp Leu Thr Ile Pro Glu Ile Pro Pro Lys Arg Gly Glu Leu
        35                  40                  45

Lys Thr Glu Leu Leu Gly Leu Lys Glu Arg Lys His Lys Pro Gln Val
    50                  55                  60

Ser Gln Gln Glu Glu Leu Lys
65                  70

<210> 55
<211> 529
<212> PRT
<213> Homo sapiens

<400> 55

Met Leu Gly Phe Val Gly Arg Val Ala Ala Ala Pro Ala Ser Gly Ala
1               5                   10                  15

Leu Arg Arg Leu Thr Pro Ser Ala Ser Leu Pro Pro Ala Gln Leu Leu
            20                  25                  30

Leu Arg Ala Ala Pro Thr Ala Val His Pro Val Arg Asp Tyr Ala Ala
        35                  40                  45

Gln Thr Ser Pro Ser Pro Lys Ala Gly Ala Ala Thr Gly Arg Ile Val
    50                  55                  60

Ala Val Ile Gly Ala Val Val Asp Val Gln Phe Asp Glu Gly Leu Pro
65                  70                  75                  80

Pro Ile Leu Asn Ala Leu Glu Val Gln Gly Arg Glu Thr Arg Leu Val
                85                  90                  95

Leu Glu Val Ala Gln His Leu Gly Glu Ser Thr Val Arg Thr Ile Ala
            100                 105                 110

Met Asp Gly Thr Glu Gly Leu Val Arg Gly Gln Lys Val Leu Asp Ser
        115                 120                 125

Gly Ala Pro Ile Lys Ile Pro Val Gly Pro Glu Thr Leu Gly Arg Ile
    130                 135                 140

Met Asn Val Ile Gly Glu Pro Ile Asp Glu Arg Gly Pro Ile Lys Thr
145                 150                 155                 160

Lys Gln Phe Ala Pro Ile His Ala Glu Ala Pro Glu Phe Met Glu Met
                165                 170                 175

Ser Val Glu Gln Glu Ile Leu Val Thr Gly Ile Lys Val Val Asp Leu
            180                 185                 190

Leu Ala Pro Tyr Ala Lys Gly Gly Lys Ile Gly Leu Phe Gly Gly Ala
        195                 200                 205

Gly Val Gly Lys Thr Val Leu Ile Met Glu Leu Ile Asn Asn Val Ala
    210                 215                 220

Lys Ala His Gly Gly Tyr Ser Val Phe Ala Gly Val Gly Glu Arg Thr
225                 230                 235                 240

Arg Glu Gly Asn Asp Leu Tyr His Glu Met Ile Glu Ser Gly Val Ile
                245                 250                 255

Asn Leu Lys Asp Ala Thr Ser Lys Val Ala Leu Val Tyr Gly Gln Met
            260                 265                 270

Asn Glu Pro Pro Gly Ala Arg Ala Arg Val Ala Leu Thr Gly Leu Thr
        275                 280                 285

Val Ala Glu Tyr Phe Arg Asp Gln Glu Gly Gln Asp Val Leu Leu Phe
    290                 295                 300

Ile Asp Asn Ile Phe Arg Phe Thr Gln Ala Gly Ser Glu Val Ser Ala
305                 310                 315                 320

Leu Leu Gly Arg Ile Pro Ser Ala Val Gly Tyr Gln Pro Thr Leu Ala
                325                 330                 335

Thr Asp Met Gly Thr Met Gln Glu Arg Ile Thr Thr Thr Lys Lys Gly
            340                 345                 350

Ser Ile Thr Ser Val Gln Ala Ile Tyr Val Pro Ala Asp Asp Leu Thr
        355                 360                 365

Asp Pro Ala Pro Ala Thr Thr Phe Ala His Leu Asp Ala Thr Thr Val
    370                 375                 380

Leu Ser Arg Ala Ile Ala Glu Leu Gly Ile Tyr Pro Ala Val Asp Pro
385                 390                 395                 400

Leu Asp Ser Thr Ser Arg Ile Met Asp Pro Asn Ile Val Gly Ser Glu
                405                 410                 415

His Tyr Asp Val Ala Arg Gly Val Gln Lys Ile Leu Gln Asp Tyr Lys
            420                 425                 430

Ser Leu Gln Asp Ile Ile Ala Ile Leu Gly Met Asp Glu Leu Ser Glu
        435                 440                 445

Glu Asp Lys Leu Thr Val Ser Arg Ala Arg Lys Ile Gln Arg Phe Leu
    450                 455                 460

Ser Gln Pro Phe Gln Val Ala Glu Val Phe Thr Gly His Met Gly Lys
465                 470                 475                 480

Leu Val Pro Leu Lys Glu Thr Ile Lys Gly Phe Gln Gln Ile Leu Ala
                485                 490                 495

Gly Glu Tyr Asp His Leu Pro Glu Gln Ala Phe Tyr Met Val Gly Pro
            500                 505                 510

Ile Glu Glu Ala Val Ala Lys Ala Asp Lys Leu Ala Glu Glu His Ser
        515                 520                 525

Ser

<210>  56
<211>  170
<212>  PRT
<213>  Homo sapiens

<400>  56

Met Ser His Arg Thr Ser Ser Thr Phe Arg Ala Glu Arg Ser Phe His
1               5                   10                  15

Ser Ser Ser Ser Ser Ser Ser Ser Ser Thr Ser Ser Ser Ala Ser Arg
            20                  25                  30

Ala Leu Pro Ala Gln Asp Pro Pro Met Glu Lys Ala Leu Ser Met Phe
        35                  40                  45

Ser Asp Asp Phe Gly Ser Phe Met Arg Pro His Ser Glu Pro Leu Ala
    50                  55                  60

Phe Pro Ala Arg Pro Gly Gly Ala Gly Asn Ile Lys Thr Leu Gly Asp
65                  70                  75                  80

Ala Tyr Glu Phe Ala Val Asp Val Arg Asp Phe Ser Pro Glu Asp Ile
                85                  90                  95

Ile Val Thr Thr Ser Asn Asn His Ile Glu Val Arg Ala Glu Lys Leu
            100                 105                 110

Ala Ala Asp Gly Thr Val Met Asn Thr Phe Ala His Lys Cys Gln Leu
        115                 120                 125

Pro Glu Asp Val Asp Pro Thr Ser Val Thr Ser Ala Leu Arg Glu Asp
    130                 135                 140

Gly Ser Leu Thr Ile Arg Ala Arg Arg His Pro His Thr Glu His Val
145                 150                 155                 160

Gln Gln Thr Phe Arg Thr Glu Ile Lys Ile
                165                 170


<210>  57
<211>  159
<212>  PRT
<213>  Homo sapiens

<400>  57

Met Ala Asp Val Pro Gly Ala Gln Arg Ala Val Pro Gly Asp Gly Pro
1               5                   10                  15

Glu Pro Arg Asp Pro Leu Asp Cys Trp Ala Cys Ala Val Leu Val Thr
            20                  25                  30

Ala Gln Asn Leu Leu Val Ala Ala Phe Asn Leu Leu Leu Leu Val Leu
        35                  40                  45

Val Leu Gly Thr Ile Leu Leu Pro Ala Val Thr Met Leu Gly Phe Gly
    50                  55                  60

Phe Leu Cys His Ser Gln Phe Leu Arg Ser Gln Ala Pro Pro Cys Thr
65                  70                  75                  80

Ala His Leu Arg Asp Pro Gly Phe Thr Ala Leu Leu Val Thr Gly Phe
                85                  90                  95

Leu Leu Leu Val Pro Leu Leu Val Leu Ala Leu Ala Ser Tyr Arg Arg
            100                 105                 110

Leu Cys Leu Arg Leu Arg Leu Ala Asp Cys Leu Val Pro Tyr Ser Arg
        115                 120                 125

Ala Leu Tyr Arg Arg Arg Arg Ala Pro Gln Pro Arg Gln Ile Arg Ala
    130                 135                 140

Ser Pro Gly Ser Gln Ala Val Pro Thr Ser Gly Lys Val Trp Val
145                 150                 155

<210>  58
<211>  1518
<212>  PRT
<213>  Homo sapiens

<400>  58

Met Ala Ala Val Gly Ser Gly Gly Tyr Ala Arg Asn Asp Ala Gly Glu
1               5                   10                  15

Lys Leu Pro Ser Val Met Ala Gly Val Pro Ala Arg Arg Gly Gln Ser
            20                  25                  30

Ser Pro Pro Pro Ala Pro Pro Ile Cys Leu Arg Arg Arg Thr Arg Leu
        35                  40                  45

Ser Thr Ala Ser Glu Glu Thr Val Gln Asn Arg Val Ser Leu Glu Lys
    50                  55                  60

Val Leu Gly Ile Thr Ala Gln Asn Ser Ser Gly Leu Thr Cys Asp Pro
65                  70                  75                  80

Gly Thr Gly His Val Ala Tyr Leu Ala Gly Cys Val Val Val Ile Leu
                85                  90                  95

Asp Pro Lys Glu Asn Lys Gln Gln His Ile Phe Asn Thr Ala Arg Lys
            100                 105                 110

Ser Leu Ser Ala Leu Ala Phe Ser Pro Asp Gly Lys Tyr Ile Val Thr
        115                 120                 125

Gly Glu Asn Gly His Arg Pro Ala Val Arg Ile Trp Asp Val Glu Glu
    130                 135                 140

Lys Asn Gln Val Ala Glu Met Leu Gly His Lys Tyr Gly Val Ala Cys
145                 150                 155                 160

Val Ala Phe Ser Pro Asn Met Lys His Ile Val Ser Met Gly Tyr Gln
                165                 170                 175

His Asp Met Val Leu Asn Val Trp Asp Trp Lys Lys Asp Ile Val Val
            180                 185                 190

Ala Ser Asn Lys Val Ser Cys Arg Val Ile Ala Leu Ser Phe Ser Glu
        195                 200                 205

Asp Ser Ser Tyr Phe Val Thr Val Gly Asn Arg His Val Arg Phe Trp
    210                 215                 220

Phe Leu Glu Val Ser Thr Glu Thr Lys Val Thr Ser Thr Val Pro Leu
225                 230                 235                 240

Val Gly Arg Ser Gly Ile Leu Gly Glu Leu His Asn Asn Ile Phe Cys
                245                 250                 255

Gly Val Ala Cys Gly Arg Gly Arg Met Ala Gly Ser Thr Phe Cys Val
            260                 265                 270

Ser Tyr Ser Gly Leu Leu Cys Gln Phe Asn Glu Lys Arg Val Leu Glu
        275                 280                 285

Lys Trp Ile Asn Leu Lys Val Ser Leu Ser Ser Cys Leu Cys Val Ser
    290                 295                 300

Gln Glu Leu Ile Phe Cys Gly Cys Thr Asp Gly Ile Val Arg Ile Phe
305                 310                 315                 320

Gln Ala His Ser Leu His Tyr Leu Ala Asn Leu Pro Lys Pro His Tyr
                325                 330                 335

Leu Gly Val Asp Val Ala Gln Gly Leu Glu Pro Ser Phe Leu Phe His
            340                 345                 350

Arg Lys Ala Glu Ala Val Tyr Pro Asp Thr Val Ala Leu Thr Phe Asp

355                 360                 365

Pro Ile His Gln Trp Leu Ser Cys Val Tyr Lys Asp His Ser Ile Tyr
    370                 375                 380

Ile Trp Asp Val Lys Asp Ile Asn Arg Val Gly Lys Val Trp Ser Glu
385                 390                 395                 400

Leu Phe His Ser Ser Tyr Val Trp Asn Val Glu Val Tyr Pro Glu Phe
                405                 410                 415

Glu Asp Gln Arg Ala Cys Leu Pro Ser Gly Ser Phe Leu Thr Cys Ser
            420                 425                 430

Ser Asp Asn Thr Ile Arg Phe Trp Asn Leu Asp Ser Ser Pro Asp Ser
        435                 440                 445

His Trp Gln Lys Asn Ile Phe Ser Asn Thr Leu Leu Lys Val Val Tyr
    450                 455                 460

Val Glu Asn Asp Ile Gln His Leu Gln Asp Met Ser His Phe Pro Asp
465                 470                 475                 480

Arg Gly Ser Glu Asn Gly Thr Pro Met Asp Val Lys Ala Gly Val Arg
                485                 490                 495

Val Met Gln Val Ser Pro Asp Gly Gln His Leu Ala Ser Gly Asp Arg
            500                 505                 510

Ser Gly Asn Leu Arg Ile His Glu Leu His Phe Met Asp Glu Leu Val
        515                 520                 525

Lys Val Glu Ala His Asp Ala Glu Val Leu Cys Leu Glu Tyr Ser Lys
    530                 535                 540

Pro Glu Thr Gly Leu Thr Leu Leu Ala Ser Ala Ser Arg Asp Arg Leu
545                 550                 555                 560

Ile His Val Leu Asn Val Glu Lys Asn Tyr Asn Leu Glu Gln Thr Leu
                565                 570                 575

Asp Asp His Ser Ser Ser Ile Thr Ala Ile Lys Phe Ala Gly Asn Arg
            580                 585                 590

Asp Ile Gln Met Ile Ser Cys Gly Ala Asp Lys Ser Ile Tyr Phe Arg
        595                 600                 605

Ser Ala Gln Gln Gly Ser Asp Gly Leu His Phe Val Arg Thr His His
    610                 615                 620

Val Ala Glu Lys Thr Thr Leu Tyr Asp Met Asp Ile Asp Ile Thr Gln
625                 630                 635                 640

Lys Tyr Val Ala Val Ala Cys Gln Asp Arg Asn Val Arg Val Tyr Asn
                645                 650                 655

Thr Val Asn Gly Lys Gln Lys Lys Cys Tyr Lys Gly Ser Gln Gly Asp
            660                 665                 670

Glu Gly Ser Leu Leu Lys Val His Val Asp Pro Ser Gly Thr Phe Leu
        675                 680                 685

Ala Thr Ser Cys Ser Asp Lys Ser Ile Ser Val Ile Asp Phe Tyr Ser
    690                 695                 700

Gly Glu Cys Ile Ala Lys Met Phe Gly His Ser Glu Ile Ile Thr Ser
705                 710                 715                 720

Met Lys Phe Thr Tyr Asp Cys His His Leu Ile Thr Val Ser Gly Asp
                725                 730                 735

Ser Cys Val Phe Ile Trp His Leu Gly Pro Glu Ile Thr Asn Cys Met
            740                 745                 750

Lys Gln His Leu Leu Glu Ile Asp His Arg Gln Gln Gln Gln His Thr
        755                 760                 765

Asn Asp Lys Lys Arg Ser Gly His Pro Arg Gln Asp Thr Tyr Val Ser
    770                 775                 780

Thr Pro Ser Glu Ile His Ser Leu Ser Pro Gly Glu Gln Thr Glu Asp
785                 790                 795                 800

Asp Leu Glu Glu Glu Cys Glu Pro Glu Glu Met Leu Lys Thr Pro Ser
                805                 810                 815

Lys Asp Ser Leu Asp Pro Asp Pro Arg Cys Leu Leu Thr Asn Gly Lys
            820                 825                 830

Leu Pro Leu Trp Ala Lys Arg Leu Leu Gly Asp Asp Asp Val Ala Asp
        835                 840                 845

Gly Leu Ala Phe His Ala Lys Arg Ser Tyr Gln Pro His Gly Arg Trp
    850                 855                 860

Ala Glu Arg Ala Gly Gln Glu Pro Leu Lys Thr Ile Leu Asp Ala Gln
865                 870                 875                 880

Asp Leu Asp Cys Tyr Phe Thr Pro Met Lys Pro Glu Ser Leu Glu Asn
                885                 890                 895

Ser Ile Leu Asp Ser Leu Glu Pro Gln Ser Leu Ala Ser Leu Leu Ser
            900                 905                 910

Glu Ser Glu Ser Pro Gln Glu Ala Gly Arg Gly His Pro Ser Phe Leu
        915                 920                 925

Pro Gln Gln Lys Glu Ser Ser Glu Ala Ser Glu Leu Ile Leu Tyr Ser
    930                 935                 940

Leu Glu Ala Glu Val Thr Val Thr Gly Thr Asp Ser Gln Tyr Cys Arg
945                 950                 955                 960

Lys Glu Val Glu Ala Gly Pro Gly Asp Gln Gln Gly Asp Ser Tyr Leu
                965                 970                 975

Arg Val Ser Ser Asp Ser Pro Lys Asp Gln Ser Pro Pro Glu Asp Ser
            980                 985                 990

Gly Glu Ser Glu Ala Asp Leu Glu  Cys Ser Phe Ala Ala  Ile His Ser
        995                 1000                 1005

Pro Ala  Pro Pro Pro Asp Pro  Ala Pro Arg Phe Ala  Thr Ser Leu
    1010                 1015                 1020

Pro His  Phe Pro Gly Cys Ala  Gly Pro Thr Glu Asp  Glu Leu Ser
    1025                 1030                 1035

Leu Pro  Glu Gly Pro Ser Val  Pro Ser Ser Ser Leu  Pro Gln Thr
    1040                 1045                 1050

Pro Glu  Gln Glu Lys Phe Leu  Arg His His Phe Glu  Thr Leu Thr
    1055                 1060                 1065

Glu Ser  Pro Cys Arg Ala Leu  Gly Asp Val Glu Ala  Ser Glu Ala
    1070                 1075                 1080

Glu Asp  His Phe Phe Asn Pro  Arg Leu Ser Ile Ser  Thr Gln Phe
    1085                 1090                 1095

Leu Ser  Ser Leu Gln Lys Ala  Ser Arg Phe Thr His  Thr Phe Pro
    1100                 1105                 1110

Pro Arg Ala Thr Gln Cys Leu Val Lys Ser Pro Glu Val Lys Leu
    1115                1120                1125

Met Asp Arg Gly Gly Ser Gln Pro Arg Ala Gly Thr Gly Tyr Ala
    1130                1135                1140

Ser Pro Asp Arg Thr His Val Leu Ala Ala Gly Lys Ala Glu Glu
    1145                1150                1155

Thr Leu Glu Ala Trp Arg Pro Pro Pro Pro Cys Leu Thr Ser Leu
    1160                1165                1170

Ala Ser Cys Val Pro Ala Ser Ser Val Leu Pro Thr Asp Arg Asn
    1175                1180                1185

Leu Pro Thr Pro Thr Ser Ala Pro Thr Pro Gly Leu Ala Gln Gly
    1190                1195                1200

Val His Ala Pro Ser Thr Cys Ser Tyr Met Glu Ala Thr Ala Ser
    1205                1210                1215

Ser Arg Ala Arg Ile Ser Arg Ser Ile Ser Leu Gly Asp Ser Glu
    1220                1225                1230

Gly Pro Ile Val Ala Thr Leu Ala Gln Pro Leu Arg Arg Pro Ser
    1235                1240                1245

Ser Val Gly Glu Leu Ala Ser Leu Gly Gln Glu Leu Gln Ala Ile
    1250                1255                1260

Thr Thr Ala Thr Thr Pro Ser Leu Asp Ser Glu Gly Gln Glu Pro
    1265                1270                1275

Ala Leu Arg Ser Trp Gly Asn His Glu Ala Arg Ala Asn Leu Arg
    1280                1285                1290

Leu Thr Leu Ser Ser Ala Cys Asp Gly Leu Leu Gln Pro Pro Val
    1295                1300                1305

Asp Thr Gln Pro Gly Val Thr Val Pro Ala Val Ser Phe Pro Ala
    1310                1315                1320

Pro Ser Pro Val Glu Glu Ser Ala Leu Arg Leu His Gly Ser Ala
    1325                1330                1335

Phe Arg Pro Ser Leu Pro Ala Pro Glu Ser Pro Gly Leu Pro Ala

1340                    1345                    1350

His Pro Ser Asn Pro Gln Leu Pro Glu Ala Arg Pro Gly Ile Pro
    1355                    1360                    1365

Gly Gly Thr Ala Ser Leu Leu Glu Pro Thr Ser Gly Ala Leu Gly
    1370                    1375                    1380

Leu Leu Gln Gly Ser Pro Ala Arg Trp Ser Glu Pro Trp Val Pro
    1385                    1390                    1395

Val Glu Ala Leu Pro Pro Ser Pro Leu Glu Leu Ser Arg Val Gly
    1400                    1405                    1410

Asn Ile Leu His Arg Leu Gln Thr Thr Phe Gln Glu Ala Leu Asp
    1415                    1420                    1425

Leu Tyr Arg Val Leu Val Ser Ser Gly Gln Val Asp Thr Gly Gln
    1430                    1435                    1440

Gln Gln Ala Arg Thr Glu Leu Val Ser Thr Phe Leu Trp Ile His
    1445                    1450                    1455

Ser Gln Leu Glu Ala Glu Cys Leu Val Gly Thr Ser Val Ala Pro
    1460                    1465                    1470

Ala Gln Ala Leu Pro Ser Pro Gly Pro Pro Ser Pro Pro Thr Leu
    1475                    1480                    1485

Tyr Pro Leu Ala Ser Pro Asp Leu Gln Ala Leu Leu Glu His Tyr
    1490                    1495                    1500

Ser Glu Leu Leu Val Gln Ala Val Arg Arg Lys Ala Arg Gly His
    1505                    1510                    1515

<210>  59
<211>  901
<212>  PRT
<213>  Homo sapiens

<400>  59

Met Met Met Val Met Gln Pro Glu Gly Leu Gly Ala Gly Glu Gly Arg
1               5                   10                  15

Phe Ala Gly Gly Gly Gly Gly Gly Glu Tyr Met Glu Gln Glu Glu Asp
            20                  25                  30

Trp Asp Arg Asp Leu Leu Leu Asp Pro Ala Trp Glu Lys Gln Gln Arg

35                  40                  45

Lys Thr Phe Thr Ala Trp Cys Asn Ser His Leu Arg Lys Ala Gly Thr
    50                  55                  60

Gln Ile Glu Asn Ile Glu Glu Asp Phe Arg Asn Gly Leu Lys Leu Met
65                  70                  75                  80

Leu Leu Leu Glu Val Ile Ser Gly Glu Arg Leu Pro Arg Pro Asp Lys
                85                  90                  95

Gly Lys Met Arg Phe His Lys Ile Ala Asn Val Asn Lys Ala Leu Asp
            100                 105                 110

Phe Ile Ala Ser Lys Gly Val Lys Leu Val Ser Ile Gly Ala Glu Glu
        115                 120                 125

Ile Val Asp Gly Asn Leu Lys Met Thr Leu Gly Met Ile Trp Thr Ile
    130                 135                 140

Ile Leu Arg Phe Ala Ile Gln Asp Ile Ser Val Glu Glu Thr Ser Ala
145                 150                 155                 160

Lys Glu Gly Leu Leu Leu Trp Cys Gln Arg Lys Thr Ala Pro Tyr Arg
                165                 170                 175

Asn Val Asn Val Gln Asn Phe His Thr Ser Trp Lys Asp Gly Leu Ala
            180                 185                 190

Leu Cys Ala Leu Ile His Arg His Arg Pro Asp Leu Ile Asp Tyr Ala
        195                 200                 205

Lys Leu Arg Lys Asp Asp Pro Ile Gly Asn Leu Asn Thr Ala Phe Glu
    210                 215                 220

Val Ala Glu Lys Tyr Leu Asp Ile Pro Lys Met Leu Asp Ala Glu Asp
225                 230                 235                 240

Ile Val Asn Thr Pro Lys Pro Asp Glu Lys Ala Ile Met Thr Tyr Val
                245                 250                 255

Ser Cys Phe Tyr His Ala Phe Ala Gly Ala Glu Gln Ala Glu Thr Ala
            260                 265                 270

Ala Asn Arg Ile Cys Lys Val Leu Ala Val Asn Gln Glu Asn Glu Lys
        275                 280                 285

Leu Met Glu Glu Tyr Glu Lys Leu Ala Ser Glu Leu Leu Glu Trp Ile
    290                 295                 300

Arg Arg Thr Val Pro Trp Leu Glu Asn Arg Val Gly Glu Pro Ser Met
305                 310                 315                 320

Ser Ala Met Gln Arg Lys Leu Glu Asp Phe Arg Asp Tyr Arg Arg Leu
                325                 330                 335

His Lys Pro Pro Arg Ile Gln Glu Lys Cys Gln Leu Glu Ile Asn Phe
            340                 345                 350

Asn Thr Leu Gln Thr Lys Leu Arg Leu Ser His Arg Pro Ala Phe Met
        355                 360                 365

Pro Ser Glu Gly Lys Leu Val Ser Asp Ile Ala Asn Ala Trp Arg Gly
    370                 375                 380

Leu Glu Gln Val Glu Lys Gly Tyr Glu Asp Trp Leu Leu Ser Glu Ile
385                 390                 395                 400

Arg Arg Leu Gln Arg Leu Gln His Leu Ala Glu Lys Phe Arg Gln Lys
                405                 410                 415

Ala Ser Leu His Glu Ala Trp Thr Arg Gly Lys Glu Glu Met Leu Ser
            420                 425                 430

Gln Arg Asp Tyr Asp Ser Ala Leu Leu Gln Glu Val Arg Ala Leu Leu
        435                 440                 445

Arg Arg His Glu Ala Phe Glu Ser Asp Leu Ala Ala His Gln Asp Arg
    450                 455                 460

Val Glu His Ile Ala Ala Leu Ala Gln Glu Leu Asn Glu Leu Asp Tyr
465                 470                 475                 480

His Glu Ala Ala Ser Val Asn Ser Arg Cys Gln Ala Ile Cys Asp Gln
                485                 490                 495

Trp Asp Asn Leu Gly Thr Leu Thr Gln Lys Arg Arg Asp Ala Leu Glu
            500                 505                 510

Arg Met Glu Lys Leu Leu Glu Thr Ile Asp Arg Leu Gln Leu Glu Phe
        515                 520                 525

Ala Arg Arg Ala Ala Pro Phe Asn Asn Trp Leu Asp Gly Ala Val Glu
    530                 535                 540

Asp Leu Gln Asp Val Trp Leu Val His Ser Val Glu Glu Thr Gln Ser
545                 550                 555                 560

Leu Leu Thr Ala His Asp Gln Phe Lys Ala Thr Leu Pro Glu Ala Asp
                565                 570                 575

Arg Glu Arg Gly Ala Ile Met Gly Ile Gln Gly Glu Ile Gln Lys Ile
            580                 585                 590

Cys Gln Thr Tyr Gly Leu Arg Pro Cys Ser Thr Asn Pro Tyr Ile Thr
        595                 600                 605

Leu Ser Pro Gln Asp Ile Asn Thr Lys Trp Asp Met Val Arg Lys Leu
    610                 615                 620

Val Pro Ser Cys Asp Gln Thr Leu Gln Glu Glu Leu Ala Arg Gln Gln
625                 630                 635                 640

Val Asn Glu Arg Leu Arg Arg Gln Phe Ala Ala Gln Ala Asn Ala Ile
                645                 650                 655

Gly Pro Trp Ile Gln Ala Lys Val Glu Glu Val Gly Arg Leu Ala Ala
            660                 665                 670

Gly Leu Ala Gly Ser Leu Glu Glu Gln Met Ala Gly Leu Arg Gln Gln
        675                 680                 685

Glu Gln Asn Ile Ile Asn Tyr Lys Thr Asn Ile Asp Arg Leu Glu Gly
    690                 695                 700

Asp His Gln Leu Leu Gln Glu Ser Leu Val Phe Asp Asn Lys His Thr
705                 710                 715                 720

Val Tyr Ser Met Glu His Ile Arg Val Gly Trp Glu Gln Leu Leu Thr
                725                 730                 735

Ser Ile Ala Arg Thr Ile Asn Glu Val Glu Asn Gln Val Leu Thr Arg
            740                 745                 750

Asp Ala Lys Gly Leu Ser Gln Glu Gln Leu Asn Glu Phe Arg Ala Ser
        755                 760                 765

Phe Asn His Phe Asp Arg Lys Gln Asn Gly Met Met Glu Pro Asp Asp
    770                 775                 780

Phe Arg Ala Cys Leu Ile Ser Met Gly Tyr Asp Leu Gly Glu Val Glu
785                 790                 795                 800

Phe Ala Arg Ile Met Thr Met Val Asp Pro Asn Ala Ala Gly Val Val
                805                 810                 815

Thr Phe Gln Ala Phe Ile Asp Phe Met Thr Arg Glu Thr Ala Glu Thr
            820                 825                 830

Asp Thr Thr Glu Gln Val Val Ala Ser Phe Lys Ile Leu Ala Gly Asp
        835                 840                 845

Lys Asn Tyr Ile Thr Pro Glu Glu Leu Arg Arg Glu Leu Pro Ala Lys
    850                 855                 860

Gln Ala Glu Tyr Cys Ile Arg Arg Met Val Pro Tyr Lys Gly Ser Gly
865                 870                 875                 880

Ala Pro Ala Gly Ala Leu Asp Tyr Val Ala Phe Ser Ser Ala Leu Tyr
                885                 890                 895

Gly Glu Ser Asp Leu
            900

<210>  60
<211>  477
<212>  PRT
<213>  Homo sapiens

<400>  60

Met Ser Ala Ala Pro Gly Leu Leu His Gln Glu Leu Ser Cys Pro Leu
1               5                   10                  15

Cys Leu Gln Leu Phe Asp Ala Pro Val Thr Ala Glu Cys Gly His Ser
            20                  25                  30

Phe Cys Arg Ala Cys Leu Gly Arg Val Ala Gly Glu Pro Ala Ala Asp
        35                  40                  45

Gly Thr Val Leu Cys Pro Cys Cys Gln Ala Pro Thr Arg Pro Gln Ala
    50                  55                  60

Leu Ser Thr Asn Leu Gln Leu Ala Arg Leu Val Glu Gly Leu Ala Gln
65                  70                  75                  80

Val Pro Gln Gly His Cys Glu Glu His Leu Asp Pro Leu Ser Ile Tyr
                85                  90                  95

Cys Glu Gln Asp Arg Ala Leu Val Cys Gly Val Cys Ala Ser Leu Gly
            100                 105                 110

Ser His Arg Gly His Arg Leu Leu Pro Ala Ala Glu Ala His Ala Arg
        115                 120                 125

Leu Lys Thr Gln Leu Pro Gln Gln Lys Leu Gln Leu Gln Glu Ala Cys
    130                 135                 140

Met Arg Lys Glu Lys Ser Val Ala Val Leu Glu His Gln Leu Val Glu
145                 150                 155                 160

Val Glu Glu Thr Val Arg Gln Phe Arg Gly Ala Val Gly Glu Gln Leu
                165                 170                 175

Gly Lys Met Arg Val Phe Leu Ala Ala Leu Glu Gly Ser Leu Asp Arg
            180                 185                 190

Glu Ala Glu Arg Val Arg Gly Glu Ala Gly Val Ala Leu Arg Arg Glu
        195                 200                 205

Leu Gly Ser Leu Asn Ser Tyr Leu Glu Gln Leu Arg Gln Met Glu Lys
    210                 215                 220

Val Leu Glu Glu Val Ala Asp Lys Pro Gln Thr Glu Phe Leu Met Lys
225                 230                 235                 240

Tyr Cys Leu Val Thr Ser Arg Leu Gln Lys Ile Leu Ala Glu Ser Pro
                245                 250                 255

Pro Pro Ala Arg Leu Asp Ile Gln Leu Pro Ile Ile Ser Asp Asp Phe
            260                 265                 270

Lys Phe Gln Val Trp Arg Lys Met Phe Arg Ala Leu Met Pro Ala Leu
        275                 280                 285

Glu Glu Leu Thr Phe Asp Pro Ser Ser Ala His Pro Ser Leu Val Val
    290                 295                 300

Ser Ser Ser Gly Arg Arg Val Glu Cys Ser Glu Gln Lys Ala Pro Pro
305                 310                 315                 320

Ala Gly Glu Asp Pro Arg Gln Phe Asp Lys Ala Val Ala Val Val Ala
                325                 330                 335

His Gln Gln Leu Ser Glu Gly Glu His Tyr Trp Glu Val Asp Val Gly
            340                 345                 350

Asp Lys Pro Arg Trp Ala Leu Gly Val Ile Ala Ala Glu Ala Pro Arg

355                 360                 365

Arg Gly Arg Leu His Ala Val Pro Ser Gln Gly Leu Trp Leu Leu Gly
    370                 375                 380

Leu Arg Glu Gly Lys Ile Leu Glu Ala His Val Glu Ala Lys Glu Pro
385                 390                 395                 400

Arg Ala Leu Arg Ser Pro Glu Arg Arg Pro Thr Arg Ile Gly Leu Tyr
                405                 410                 415

Leu Ser Phe Gly Asp Gly Val Leu Ser Phe Tyr Asp Ala Ser Asp Ala
            420                 425                 430

Asp Ala Leu Val Pro Leu Phe Ala Phe His Glu Arg Leu Pro Arg Pro
        435                 440                 445

Val Tyr Pro Phe Phe Asp Val Cys Trp His Asp Lys Gly Lys Asn Ala
    450                 455                 460

Gln Pro Leu Leu Leu Val Gly Pro Glu Gly Ala Glu Ala
465                 470                 475

<210>  61
<211>  1017
<212>  PRT
<213>  Homo sapiens

<400>  61

Met Asp Leu Ile Arg Gly Val Leu Leu Arg Leu Leu Leu Leu Ala Ser
1               5                   10                  15

Ser Leu Gly Pro Gly Ala Val Ser Leu Arg Ala Ala Ile Arg Lys Pro
            20                  25                  30

Gly Lys Val Gly Pro Pro Leu Asp Ile Lys Leu Gly Ala Leu Asn Cys
        35                  40                  45

Thr Ala Phe Ser Ile Gln Trp Lys Met Pro Arg His Pro Gly Ser Pro
    50                  55                  60

Ile Leu Gly Tyr Thr Val Phe Tyr Ser Glu Val Gly Ala Asp Lys Ser
65                  70                  75                  80

Leu Gln Glu Gln Leu His Ser Val Pro Leu Ser Arg Asp Ile Pro Thr
                85                  90                  95

Thr Glu Glu Val Ile Gly Asp Leu Lys Pro Gly Thr Glu Tyr Arg Val

            100                 105                 110

Ser Ile Ala Ala Tyr Ser Gln Ala Gly Lys Gly Arg Leu Ser Ser Pro
        115                 120                 125

Arg His Val Thr Thr Leu Ser Gln Asp Ser Cys Leu Pro Pro Ala Ala
    130                 135                 140

Pro Gln Gln Pro His Val Ile Val Val Ser Asp Ser Glu Val Ala Leu
145                 150                 155                 160

Ser Trp Lys Pro Gly Ala Ser Glu Gly Ser Ala Pro Ile Gln Tyr Tyr
                165                 170                 175

Ser Val Glu Phe Ile Arg Pro Asp Phe Asp Lys Lys Trp Thr Ser Ile
            180                 185                 190

His Glu Arg Ile Gln Met Asp Ser Met Val Ile Lys Gly Leu Asp Pro
        195                 200                 205

Asp Thr Asn Tyr Gln Phe Ala Val Arg Ala Met Asn Ser His Gly Pro
    210                 215                 220

Ser Pro Arg Ser Trp Pro Ser Asp Ile Ile Arg Thr Leu Cys Pro Glu
225                 230                 235                 240

Glu Ala Gly Ser Gly Arg Tyr Gly Pro Arg Tyr Ile Thr Asp Met Gly
                245                 250                 255

Ala Gly Glu Asp Asp Glu Gly Phe Glu Asp Asp Leu Asp Leu Asp Ile
            260                 265                 270

Ser Phe Glu Glu Val Lys Pro Leu Pro Ala Thr Lys Gly Gly Asn Lys
        275                 280                 285

Lys Phe Leu Val Glu Ser Lys Lys Met Ser Ile Ser Asn Pro Lys Thr
    290                 295                 300

Ile Ser Arg Leu Ile Pro Pro Thr Ser Ala Ser Leu Pro Val Thr Thr
305                 310                 315                 320

Val Ala Pro Gln Pro Ile Pro Ile Gln Arg Lys Gly Lys Asn Gly Val
                325                 330                 335

Ala Ile Met Ser Arg Leu Phe Asp Met Pro Cys Asp Glu Thr Leu Cys
            340                 345                 350

Ser Ala Asp Ser Phe Cys Val Asn Asp Tyr Thr Trp Gly Gly Ser Arg
        355                 360                 365

Cys Gln Cys Thr Leu Gly Lys Gly Gly Glu Ser Cys Ser Glu Asp Ile
    370                 375                 380

Val Ile Gln Tyr Pro Gln Phe Phe Gly His Ser Tyr Val Thr Phe Glu
385                 390                 395                 400

Pro Leu Lys Asn Ser Tyr Gln Ala Phe Gln Ile Thr Leu Glu Phe Arg
                405                 410                 415

Ala Glu Ala Glu Asp Gly Leu Leu Leu Tyr Cys Gly Glu Asn Glu His
            420                 425                 430

Gly Arg Gly Asp Phe Met Ser Leu Ala Ile Ile Arg Arg Ser Leu Gln
        435                 440                 445

Phe Arg Phe Asn Cys Gly Thr Gly Val Ala Ile Ile Val Ser Glu Thr
    450                 455                 460

Lys Ile Lys Leu Gly Gly Trp His Thr Val Met Leu Tyr Arg Asp Gly
465                 470                 475                 480

Leu Asn Gly Leu Leu Gln Leu Asn Asn Gly Thr Pro Val Thr Gly Gln
                485                 490                 495

Ser Gln Gly Gln Tyr Ser Lys Ile Thr Phe Arg Thr Pro Leu Tyr Leu
            500                 505                 510

Gly Gly Ala Pro Ser Ala Tyr Trp Leu Val Arg Ala Thr Gly Thr Asn
        515                 520                 525

Arg Gly Phe Gln Gly Cys Val Gln Ser Leu Ala Val Asn Gly Arg Arg
    530                 535                 540

Ile Asp Met Arg Pro Trp Pro Leu Gly Lys Ala Leu Ser Gly Ala Asp
545                 550                 555                 560

Val Gly Glu Cys Ser Ser Gly Ile Cys Asp Glu Ala Ser Cys Ile His
                565                 570                 575

Gly Gly Thr Cys Thr Ala Ile Lys Ala Asp Ser Tyr Ile Cys Leu Cys
            580                 585                 590

Pro Leu Gly Phe Lys Gly Arg His Cys Glu Asp Ala Phe Thr Leu Thr
        595                 600                 605

Ile Pro Gln Phe Arg Glu Ser Leu Arg Ser Tyr Ala Ala Thr Pro Trp
    610                 615                 620

Pro Leu Glu Pro Gln His Tyr Leu Ser Phe Met Glu Phe Glu Ile Thr
625                 630                 635                 640

Phe Arg Pro Asp Ser Gly Asp Gly Val Leu Leu Tyr Ser Tyr Asp Thr
                645                 650                 655

Gly Ser Lys Asp Phe Leu Ser Ile Asn Leu Ala Gly Gly His Val Glu
            660                 665                 670

Phe Arg Phe Asp Cys Gly Ser Gly Thr Gly Val Leu Arg Ser Glu Asp
        675                 680                 685

Pro Leu Thr Leu Gly Asn Trp His Glu Leu Arg Val Ser Arg Thr Ala
    690                 695                 700

Lys Asn Gly Ile Leu Gln Val Asp Lys Gln Lys Ile Val Glu Gly Met
705                 710                 715                 720

Ala Glu Gly Gly Phe Thr Gln Ile Lys Cys Asn Thr Asp Ile Phe Ile
                725                 730                 735

Gly Gly Val Pro Asn Tyr Asp Asp Val Lys Lys Asn Ser Gly Val Leu
            740                 745                 750

Lys Pro Phe Ser Gly Ser Ile Gln Lys Ile Ile Leu Asn Asp Arg Thr
        755                 760                 765

Ile His Val Lys His Asp Phe Thr Ser Gly Val Asn Val Glu Asn Ala
    770                 775                 780

Ala His Pro Cys Val Arg Ala Pro Cys Ala His Gly Gly Ser Cys Arg
785                 790                 795                 800

Pro Arg Lys Glu Gly Tyr Asp Cys Asp Cys Pro Leu Gly Phe Glu Gly
                805                 810                 815

Leu His Cys Gln Lys Glu Cys Gly Asn Tyr Cys Leu Asn Thr Ile Ile
            820                 825                 830

Glu Ala Ile Glu Ile Pro Gln Phe Ile Gly Arg Ser Tyr Leu Thr Tyr
        835                 840                 845

Asp Asn Pro Asp Ile Leu Lys Arg Val Ser Gly Ser Arg Ser Asn Val
    850                 855                 860

Phe Met Arg Phe Lys Thr Thr Ala Lys Asp Gly Leu Leu Leu Trp Arg
865                 870                 875                 880

Gly Asp Ser Pro Met Arg Pro Asn Ser Asp Phe Ile Ser Leu Gly Leu
                885                 890                 895

Arg Asp Gly Ala Leu Val Phe Ser Tyr Asn Leu Gly Ser Gly Val Ala
            900                 905                 910

Ser Ile Met Val Asn Gly Ser Phe Asn Asp Gly Arg Trp His Arg Val
        915                 920                 925

Lys Ala Val Arg Asp Gly Gln Ser Gly Lys Ile Thr Val Asp Asp Tyr
    930                 935                 940

Gly Ala Arg Thr Gly Lys Ser Pro Gly Met Met Arg Gln Leu Asn Ile
945                 950                 955                 960

Asn Gly Ala Leu Tyr Val Gly Gly Met Lys Glu Ile Ala Leu His Thr
                965                 970                 975

Asn Arg Gln Tyr Met Arg Gly Leu Val Gly Cys Ile Ser His Phe Thr
            980                 985                 990

Leu Ser Thr Asp Tyr His Ile Ser  Leu Val Glu Asp Ala  Val Asp Gly
        995                 1000                1005

Lys Asn  Ile Asn Thr Cys Gly  Ala Lys
    1010                1015

<210>  62
<211>  335
<212>  PRT
<213>  Homo sapiens

<400>  62

Met Thr Glu Asn Val Val Cys Thr Gly Ala Val Asn Ala Val Lys Glu
1               5                   10                  15

Val Trp Glu Lys Arg Ile Lys Lys Leu Asn Glu Asp Leu Lys Arg Glu
            20                  25                  30

Lys Glu Phe Gln His Lys Leu Val Arg Ile Trp Glu Glu Arg Val Ser
        35                  40                  45

Leu Thr Lys Leu Arg Glu Lys Val Thr Arg Glu Asp Gly Arg Val Ile
    50                  55                  60

Leu Lys Ile Glu Lys Glu Glu Trp Lys Thr Leu Pro Ser Ser Leu Leu
65                  70                  75                  80

Lys Leu Asn Gln Leu Gln Glu Trp Gln Leu His Arg Thr Gly Leu Leu
                85                  90                  95

Lys Ile Pro Glu Phe Ile Gly Arg Phe Gln Asn Leu Ile Val Leu Asp
            100                 105                 110

Leu Ser Arg Asn Thr Ile Ser Glu Ile Pro Pro Gly Ile Gly Leu Leu
        115                 120                 125

Thr Arg Leu Gln Glu Leu Ile Leu Ser Tyr Asn Lys Ile Lys Thr Val
    130                 135                 140

Pro Lys Glu Leu Ser Asn Cys Ala Ser Leu Glu Lys Leu Glu Leu Ala
145                 150                 155                 160

Val Asn Arg Asp Ile Cys Asp Leu Pro Gln Glu Leu Ser Asn Leu Leu
                165                 170                 175

Lys Leu Thr His Leu Asp Leu Ser Met Asn Asp Phe Thr Thr Ile Pro
            180                 185                 190

Leu Ala Val Leu Asn Met Pro Ala Leu Glu Trp Leu Asp Met Gly Ser
        195                 200                 205

Asn Lys Leu Glu Gln Leu Pro Asp Thr Ile Glu Arg Met Gln Asn Leu
    210                 215                 220

His Thr Leu Trp Leu Gln Arg Asn Glu Ile Thr Cys Leu Pro Gln Thr
225                 230                 235                 240

Ile Ser Asn Met Lys Asn Leu Gly Thr Leu Val Leu Ser Asn Asn Lys
                245                 250                 255

Leu Gln Asp Ile Pro Val Cys Met Glu Glu Met Ala Asn Leu Arg Phe
            260                 265                 270

Val Asn Phe Arg Asp Asn Pro Leu Lys Leu Lys Val Ser Leu Pro Pro
        275                 280                 285

Ser Glu Gly Thr Asp Glu Glu Glu Glu Arg Glu Leu Phe Gly Leu Gln
    290                 295                 300

Phe Met His Thr Tyr Ile Gln Glu Ser Arg Arg Arg Ala Asp His Gln

305                 310                 315                 320

Val Asn Gly Ser Thr Thr Leu Pro Ile Ser Ile Asn Thr Asp Gly
                325                 330                 335

<210>  63
<211>  572
<212>  PRT
<213>  Homo sapiens

<400>  63

Met Ser Phe Gln Gly Lys Lys Ser Ile Pro Arg Ile Thr Ser Asp Arg
1               5                   10                  15

Leu Leu Ile Arg Gly Gly Arg Ile Val Asn Asp Asp Gln Ser Phe Tyr
            20                  25                  30

Ala Asp Val His Val Glu Asp Gly Leu Ile Lys Gln Ile Gly Glu Asn
        35                  40                  45

Leu Ile Val Pro Gly Gly Ile Lys Thr Ile Asp Ala His Gly Leu Met
    50                  55                  60

Val Leu Pro Gly Gly Val Asp Val His Thr Arg Leu Gln Met Pro Val
65                  70                  75                  80

Leu Gly Met Thr Pro Ala Asp Asp Phe Cys Gln Gly Thr Lys Ala Ala
                85                  90                  95

Leu Ala Gly Gly Thr Thr Met Ile Leu Asp His Val Phe Pro Asp Thr
            100                 105                 110

Gly Val Ser Leu Leu Ala Ala Tyr Glu Gln Trp Arg Glu Arg Ala Asp
        115                 120                 125

Ser Ala Ala Cys Cys Asp Tyr Ser Leu His Val Asp Ile Thr Arg Trp
    130                 135                 140

His Glu Ser Ile Lys Glu Glu Leu Glu Ala Leu Val Lys Glu Lys Gly
145                 150                 155                 160

Val Asn Ser Phe Leu Val Phe Met Ala Tyr Lys Asp Arg Cys Gln Cys
                165                 170                 175

Ser Asp Ser Gln Met Tyr Glu Ile Phe Ser Ile Ile Arg Asp Leu Gly
            180                 185                 190

Ala Leu Ala Gln Val His Ala Glu Asn Gly Asp Ile Val Glu Glu Glu

        195                 200                 205

Gln Lys Arg Leu Leu Glu Leu Gly Ile Thr Gly Pro Glu Gly His Val
    210                 215                 220

Leu Ser His Pro Glu Glu Val Glu Ala Glu Ala Val Tyr Arg Ala Val
225                 230                 235                 240

Thr Ile Ala Lys Gln Ala Asn Cys Pro Leu Tyr Val Thr Lys Val Met
                245                 250                 255

Ser Lys Gly Ala Ala Asp Ala Ile Ala Gln Ala Lys Arg Arg Gly Val
            260                 265                 270

Val Val Phe Gly Glu Pro Ile Thr Ala Ser Leu Gly Thr Asp Gly Ser
        275                 280                 285

His Tyr Trp Ser Lys Asn Trp Ala Lys Ala Ala Ala Phe Val Thr Ser
    290                 295                 300

Pro Pro Val Asn Pro Asp Pro Thr Thr Ala Asp His Leu Thr Cys Leu
305                 310                 315                 320

Leu Ser Ser Gly Asp Leu Gln Val Thr Gly Ser Ala His Cys Thr Phe
                325                 330                 335

Thr Thr Ala Gln Lys Ala Val Gly Lys Asp Asn Phe Ala Leu Ile Pro
            340                 345                 350

Glu Gly Thr Asn Gly Ile Glu Glu Arg Met Ser Met Val Trp Glu Lys
        355                 360                 365

Cys Val Ala Ser Gly Lys Met Asp Glu Asn Glu Phe Val Ala Val Thr
    370                 375                 380

Ser Thr Asn Ala Ala Lys Ile Phe Asn Phe Tyr Pro Arg Lys Gly Arg
385                 390                 395                 400

Val Ala Val Gly Ser Asp Ala Asp Leu Val Ile Trp Asn Pro Lys Ala
                405                 410                 415

Thr Lys Ile Ile Ser Ala Lys Thr His Asn Leu Asn Val Glu Tyr Asn
            420                 425                 430

Ile Phe Glu Gly Val Glu Cys Arg Gly Ala Pro Ala Val Val Ile Ser
        435                 440                 445

Gln Gly Arg Val Ala Leu Glu Asp Gly Lys Met Phe Val Thr Pro Gly
    450                 455                 460

Ala Gly Arg Phe Val Pro Arg Lys Thr Phe Pro Asp Phe Val Tyr Lys
465                 470                 475                 480

Arg Ile Lys Ala Arg Asn Arg Leu Ala Glu Ile His Gly Val Pro Arg
                485                 490                 495

Gly Leu Tyr Asp Gly Pro Val His Glu Val Met Val Pro Ala Lys Pro
            500                 505                 510

Gly Ser Gly Ala Pro Ala Arg Ala Ser Cys Pro Gly Lys Ile Ser Val
        515                 520                 525

Pro Pro Val Arg Asn Leu His Gln Ser Gly Phe Ser Leu Ser Gly Ser
    530                 535                 540

Gln Ala Asp Asp His Ile Ala Arg Arg Thr Ala Gln Lys Ile Met Ala
545                 550                 555                 560

Pro Pro Gly Gly Arg Ser Asn Ile Thr Ser Leu Ser
                565                 570


<210>  64
<211>  505
<212>  PRT
<213>  Homo sapiens

<400>  64

Met Ser Gly Ala Ser Ser Ser Glu Gln Asn Asn Asn Ser Tyr Glu Thr
1               5                   10                  15

Lys Thr Pro Asn Leu Arg Met Ser Glu Lys Lys Cys Ser Trp Ala Ser
            20                  25                  30

Tyr Met Thr Asn Ser Pro Thr Leu Ile Val Met Ile Gly Leu Pro Ala
        35                  40                  45

Arg Gly Lys Thr Tyr Val Ser Lys Lys Leu Thr Arg Tyr Leu Asn Trp
    50                  55                  60

Ile Gly Val Pro Thr Lys Val Phe Asn Leu Gly Val Tyr Arg Arg Glu
65                  70                  75                  80

Ala Val Lys Ser Tyr Lys Ser Tyr Asp Phe Phe Arg His Asp Asn Glu
                85                  90                  95

Glu Ala Met Lys Ile Arg Lys Gln Cys Ala Leu Val Ala Leu Glu Asp
            100                 105                 110

Val Lys Ala Tyr Leu Thr Glu Glu Asn Gly Gln Ile Ala Val Phe Asp
        115                 120                 125

Ala Thr Asn Thr Thr Arg Glu Arg Arg Asp Met Ile Leu Asn Phe Ala
    130                 135                 140

Glu Gln Asn Ser Phe Lys Val Phe Phe Val Glu Ser Val Cys Asp Asp
145                 150                 155                 160

Pro Asp Val Ile Ala Ala Asn Ile Leu Glu Val Lys Val Ser Ser Pro
                165                 170                 175

Asp Tyr Pro Glu Arg Asn Arg Glu Asn Val Met Glu Asp Phe Leu Lys
            180                 185                 190

Arg Ile Glu Cys Tyr Lys Val Thr Tyr Arg Pro Leu Asp Pro Asp Asn
        195                 200                 205

Tyr Asp Lys Asp Leu Ser Phe Ile Lys Val Ile Asn Val Gly Gln Arg
    210                 215                 220

Phe Leu Val Asn Arg Val Gln Asp Tyr Ile Gln Ser Lys Ile Val Tyr
225                 230                 235                 240

Tyr Leu Met Asn Ile His Val Gln Pro Arg Thr Ile Tyr Leu Cys Arg
                245                 250                 255

His Gly Glu Ser Glu Phe Asn Leu Leu Gly Lys Ile Gly Gly Asp Ser
            260                 265                 270

Gly Leu Ser Val Arg Gly Lys Gln Phe Ala Gln Ala Leu Arg Lys Phe
        275                 280                 285

Leu Glu Glu Gln Glu Ile Thr Asp Leu Lys Val Trp Thr Ser Gln Leu
    290                 295                 300

Lys Arg Thr Ile Gln Thr Ala Glu Ser Leu Gly Val Pro Tyr Glu Gln
305                 310                 315                 320

Trp Lys Ile Leu Asn Glu Ile Asp Ala Gly Val Cys Glu Glu Met Thr
                325                 330                 335

Tyr Ala Glu Ile Glu Lys Arg Tyr Pro Glu Glu Phe Ala Leu Arg Asp
            340                 345                 350

Gln Glu Lys Tyr Leu Tyr Arg Tyr Pro Gly Gly Glu Ser Tyr Gln Asp
        355                 360                 365

Leu Val Gln Arg Leu Glu Pro Val Ile Met Glu Leu Glu Arg Gln Gly
    370                 375                 380

Asn Val Leu Val Ile Ser His Gln Ala Val Met Arg Cys Leu Leu Ala
385                 390                 395                 400

Tyr Phe Leu Asp Lys Gly Ala Asp Glu Leu Pro Tyr Leu Arg Cys Pro
                405                 410                 415

Leu His Thr Ile Phe Lys Leu Thr Pro Val Ala Tyr Gly Cys Lys Val
            420                 425                 430

Glu Thr Ile Lys Leu Asn Val Glu Ala Val Asn Thr His Arg Asp Lys
        435                 440                 445

Pro Thr Asn Asn Phe Pro Lys Asn Gln Thr Pro Val Arg Met Arg Arg
    450                 455                 460

Asn Ser Phe Thr Pro Leu Ser Ser Ser Asn Thr Ile Arg Arg Pro Arg
465                 470                 475                 480

Asn Tyr Ser Val Gly Ser Arg Pro Leu Lys Pro Leu Ser Pro Leu Arg
                485                 490                 495

Ala Gln Asp Met Gln Glu Gly Ala Asp
            500                 505

<210>  65
<211>  842
<212>  PRT
<213>  Homo sapiens

<400>  65

<210> 66
<211> 378
<212> PRT
<213> Homo sapiens

<400> 66

Met Ala Gly Ser Tyr Pro Glu Gly Ala Pro Ala Ile Leu Ala Asp Lys
1               5                   10                  15

Arg Gln Gln Phe Gly Ser Arg Phe Leu Ser Asp Pro Ala Arg Val Phe
            20                  25                  30

His His Asn Ala Trp Asp Asn Val Glu Trp Ser Glu Glu Gln Ala Ala
        35                  40                  45

Ala Ala Glu Arg Lys Val Gln Glu Asn Ser Ile Gln Arg Val Cys Gln
    50                  55                  60

Glu Lys Gln Val Asp Tyr Glu Ile Asn Ala His Lys Tyr Trp Asn Asp
65                  70                  75                  80

Phe Tyr Lys Ile His Glu Asn Gly Phe Phe Lys Asp Arg His Trp Leu
                85                  90                  95

Phe Thr Glu Phe Pro Glu Leu Ala Pro Ser Gln Asn Gln Asn His Leu
            100                 105                 110

Lys Asp Trp Phe Leu Glu Asn Lys Ser Glu Val Cys Glu Cys Arg Asn
        115                 120                 125

Asn Glu Asp Gly Pro Gly Leu Ile Met Glu Glu Gln His Lys Cys Ser
    130                 135                 140

Ser Lys Ser Leu Glu His Lys Thr Gln Thr Pro Pro Val Glu Glu Asn
145                 150                 155                 160

Val Thr Gln Lys Ile Ser Asp Leu Glu Ile Cys Ala Asp Glu Phe Pro
                165                 170                 175

Gly Ser Ser Ala Thr Tyr Arg Ile Leu Glu Val Gly Cys Gly Val Gly
            180                 185                 190

Asn Thr Val Phe Pro Ile Leu Gln Thr Asn Asn Asp Pro Gly Leu Phe
        195                 200                 205

Val Tyr Cys Cys Asp Phe Ser Ser Thr Ala Ile Glu Leu Val Gln Thr
    210                 215                 220

Asn Ser Glu Tyr Asp Pro Ser Arg Cys Phe Ala Phe Val His Asp Leu
225                 230                 235                 240

Cys Asp Glu Glu Lys Ser Tyr Pro Val Pro Lys Gly Ser Leu Asp Ile
                245                 250                 255

Ile Ile Leu Ile Phe Val Leu Ser Ala Val Val Pro Asp Lys Met Gln
            260                 265                 270

Lys Ala Ile Asn Arg Leu Ser Arg Leu Leu Lys Pro Gly Gly Met Val
        275                 280                 285

Leu Leu Arg Asp Tyr Gly Arg Tyr Asp Met Ala Gln Leu Arg Phe Lys
    290                 295                 300

Lys Gly Gln Cys Leu Ser Gly Asn Phe Tyr Val Arg Gly Asp Gly Thr
305                 310                 315                 320

Arg Val Tyr Phe Phe Thr Gln Glu Glu Leu Asp Thr Leu Phe Thr Thr
                325                 330                 335

Ala Gly Leu Glu Lys Val Gln Asn Leu Val Asp Arg Arg Leu Gln Val
            340                 345                 350

Asn Arg Gly Lys Gln Leu Thr Met Tyr Arg Val Trp Ile Gln Cys Lys
        355                 360                 365

Tyr Cys Lys Pro Leu Leu Ser Ser Thr Ser
    370                 375

<210>  67
<211>  378
<212>  PRT
<213>  Homo sapiens

<400>  67

Met Ala Gly Ser Tyr Pro Glu Gly Ala Pro Ala Val Leu Ala Asp Lys
1               5                   10                  15

Arg Gln Gln Phe Gly Ser Arg Phe Leu Arg Asp Pro Ala Arg Val Phe
            20                  25                  30

His His Asn Ala Trp Asp Asn Val Glu Trp Ser Glu Glu Gln Ala Ala
        35                  40                  45

Ala Ala Glu Arg Lys Val Gln Glu Asn Ser Ile Gln Arg Val Cys Gln
    50                  55                  60

Glu Lys Gln Val Asp Tyr Glu Ile Asn Ala His Lys Tyr Trp Asn Asp
65                  70                  75                  80

Phe Tyr Lys Ile His Glu Asn Gly Phe Phe Lys Asp Arg His Trp Leu
                85                  90                  95

Phe Thr Glu Phe Pro Glu Leu Ala Pro Ser Gln Asn Gln Asn His Leu
            100                 105                 110

Lys Asp Trp Phe Leu Glu Asn Lys Ser Glu Val Pro Glu Cys Arg Asn
        115                 120                 125

Asn Glu Asp Gly Pro Gly Leu Ile Met Glu Glu Gln His Lys Cys Ser
    130                 135                 140

Ser Lys Ser Leu Glu His Lys Thr Gln Thr Leu Pro Val Glu Glu Asn
145                 150                 155                 160

Val Thr Gln Lys Ile Ser Asp Leu Glu Ile Cys Ala Asp Glu Phe Pro
                165                 170                 175

Gly Ser Ser Ala Thr Tyr Arg Ile Leu Glu Val Gly Cys Gly Val Gly
            180                 185                 190

Asn Thr Val Phe Pro Ile Leu Gln Thr Asn Asn Asp Pro Gly Leu Phe
        195                 200                 205

Val Tyr Cys Cys Asp Phe Ser Ser Thr Ala Ile Glu Leu Val Gln Thr
    210                 215                 220

Asn Ser Glu Tyr Asp Pro Ser Arg Cys Phe Ala Phe Val His Asp Leu
225                 230                 235                 240

Cys Asp Glu Glu Lys Ser Tyr Pro Val Pro Lys Gly Ser Leu Asp Ile
                245                 250                 255

Ile Ile Leu Ile Phe Val Leu Ser Ala Ile Val Pro Asp Lys Met Gln
            260                 265                 270

Lys Ala Ile Asn Arg Leu Ser Arg Leu Leu Lys Pro Gly Gly Met Met
        275                 280                 285

Leu Leu Arg Asp Tyr Gly Arg Tyr Asp Met Ala Gln Leu Arg Phe Lys
    290                 295                 300

Lys Gly Gln Cys Leu Ser Gly Asn Phe Tyr Val Arg Gly Asp Gly Thr
305                 310                 315                 320

Arg Val Tyr Phe Phe Thr Gln Glu Glu Leu Asp Thr Leu Phe Thr Thr
                325                 330                 335

Ala Gly Leu Glu Lys Val Gln Asn Leu Val Asp Arg Arg Leu Gln Val
            340                 345                 350

Asn Arg Gly Lys Gln Leu Thr Met Tyr Arg Val Trp Ile Gln Cys Lys
        355                 360                 365

Tyr Cys Lys Pro Leu Leu Ser Ser Thr Ser
    370                 375

<210>  68
<211>  827
<212>  PRT
<213>  Homo sapiens

<400>  68
   Met Leu Leu Cys Leu Ser Pro Ala Trp Leu Met Lys Val Pro Ala Pro
   1               5                   10                  15
   Gly Gln Pro Gly Glu Ala Ala Leu Leu Val Ser Lys Ala Val Ser Phe
               20                  25                  30
   His Pro Gly Gly Leu Thr Phe Leu Asp Asp Phe Val Pro Pro Arg Arg
           35                  40                  45
   Ala Thr Tyr Phe Leu Ala Gly Leu Gly Leu Gly Pro Gly Arg Gly Arg
       50                  55                  60
   Glu Ala Ala Glu Leu Ala Arg Asp Leu Thr Cys Pro Thr Gly Ala Ser
   65                  70                  75                  80
   Ala Glu Leu Ala Arg Leu Leu Glu Asp Arg Leu Leu Thr Arg Gln Leu
                   85                  90                  95
   Leu Ala Gln Gln Gly Gly Val Ala Val Pro Ala Thr Leu Ala Phe Thr
               100                 105                 110
   Tyr Lys Pro Pro Gly Leu Leu Arg Gly Gly Asp Ala Ser Leu Gly Leu
           115                 120                 125
   Arg Leu Val Glu Leu Ser Gly Lys Glu Gly Gln Glu Thr Leu Val Lys
       130                 135                 140
   Glu Glu Val Glu Ala Phe Leu Arg Ser Glu Ala Leu Gly Asp Ile Leu
   145                 150                 155                 160
   Gln Val Ala Val Lys Leu Ser Gly Trp Arg Trp Arg Gly Arg Gln Ala
                   165                 170                 175
   Trp Arg Leu His Pro Arg Ala Glu Leu Gly Ala Val Val Asp Thr Val
               180                 185                 190
   Leu Ala Leu Leu Glu Lys Leu Glu Glu Glu Glu Ser Val Leu Val Glu
           195                 200                 205
   Ala Val Tyr Pro Pro Ala Gln Leu Pro Cys Ser Asp Gly Pro Ser Pro
       210                 215                 220
   Gly Pro Gly Leu Ala Val Arg Ile Cys Ala Val Val Cys Arg Thr Gln
   225                 230                 235                 240
   Gly Asp Arg Pro Leu Leu Ser Lys Val Val Cys Gly Val Gly Arg Gly
                   245                 250                 255
   Asp Arg Pro Leu Arg His His Asn Ser Leu Pro Arg Thr Leu Glu Val
               260                 265                 270
   Ala Leu Ala Gln Cys Gly Leu Gly Glu Glu Ala Gln Val Ala Ala Val
           275                 280                 285
   Arg Gln Arg Val Lys Ala Ala Ala Glu Ala Ala Leu Ala Ala Val Leu
       290                 295                 300
   Ala Leu Glu Ala Gly Leu Ser Ala Glu Gln Arg Gly Gly Arg Arg Ala
   305                 310                 315                 320
   His Thr Asp Phe Leu Gly Val Asp Phe Ala Leu Thr Ala Ala Gly Gly
                   325                 330                 335
   Val Leu Thr Pro Val Ala Leu Glu Leu Asn Gly Gly Leu Cys Leu Glu
               340                 345                 350
   Ala Cys Gly Ala Leu Glu Gly Leu Trp Ala Ala Pro Arg Leu Gly Pro
           355                 360                 365
   Ala Ala Asp Glu Ala Val Ala Ala Pro Leu Val Glu Thr Met Leu Arg
       370                 375                 380
   Arg Ser Ala Arg Cys Leu Met Glu Gly Lys Gln Leu Leu Val Val Gly
   385                 390                 395                 400
   Ala Gly Gly Val Ser Lys Lys Phe Val Trp Glu Ala Ala Arg Asp Tyr
                   405                 410                 415
   Gly Leu Gln Leu His Leu Val Glu Ser Asp Pro Asn His Phe Ala Ser
               420                 425                 430
   Gln Leu Val Gln Thr Phe Ile His Phe Asp Met Thr Glu His Arg Arg
           435                 440                 445
   Asp Glu Glu Asn Ala Arg Leu Leu Ala Glu Leu Val Arg Ala Arg Gly
       450                 455                 460
   Leu Lys Leu Asp Gly Cys Phe Ser Tyr Trp Asp Asp Cys Leu Val Leu
   465                 470                 475                 480
   Thr Ala Leu Leu Cys Gln Glu Leu Gly Leu Pro Cys Ser Ser Pro Ala
                   485                 490                 495
   Ala Met Arg Leu Ala Lys Gln Lys Ser Leu Thr Gln Leu His Leu Leu
               500                 505                 510
   His His His Gly Pro Pro Trp Pro Ala Pro Ser Leu His Ala Val Pro
           515                 520                 525
   Cys Cys Pro Leu Glu Ser Glu Ala Asp Val Glu Arg Ala Val His Gln
       530                 535                 540
   Val Pro Leu Pro Gly Val Met Lys Leu Glu Phe Gly Ala Gly Ala Val
   545                 550                 555                 560
   Gly Val Arg Leu Val Glu Asp Ala Pro Gln Cys His Glu His Phe Ser
                   565                 570                 575
   Arg Ile Thr Arg Asp Leu Gln Gly Glu Ala Asp His Pro Gly Ile Gly
               580                 585                 590
   Leu Gly Trp Gly Asn Ala Met Leu Leu Met Glu Phe Val Glu Gly Thr
           595                 600                 605
   Glu His Asp Val Asp Leu Val Leu Phe Gly Gly Arg Leu Leu Ala Ala
       610                 615                 620
   Phe Val Ser Asp Asn Gly Pro Thr Arg Leu Pro Gly Phe Thr Glu Thr
   625                 630                 635                 640
   Ala Ala Cys Met Pro Thr Gly Leu Ala Pro Glu Gln Glu Ala Gln Met
                   645                 650                 655
   Val Gln Ala Ala Phe Arg Cys Cys Leu Gly Cys Gly Leu Leu Asp Gly
               660                 665                 670
   Val Phe Asn Val Glu Leu Lys Leu Thr Gly Ala Gly Pro Arg Leu Ile
           675                 680                 685
   Glu Ile Asn Pro Arg Met Gly Gly Phe Tyr Leu Arg Asp Trp Ile Leu
       690                 695                 700
   Glu Leu Tyr Gly Val Asp Leu Leu Leu Ala Ala Val Met Val Ala Cys
   705                 710                 715                 720
   Gly Leu Arg Pro Ala Leu Pro Thr Arg Pro Arg Ala Arg Gly His Leu
                   725                 730                 735
   Val Gly Val Met Cys Leu Val Ser Gln His Leu Gln Ala Leu Ser Ser
               740                 745                 750
   Thr Ala Ser Arg Glu Thr Leu Gln Ala Leu His Asp Arg Gly Leu Leu
           755                 760                 765
   Arg Leu Asn Leu Leu Glu Glu Ala Leu Val Pro Gly Glu Tyr Glu Glu
       770                 775                 780
   Pro Tyr Cys Ser Val Ala Cys Ala Gly Pro Ser Pro Thr Glu Ala Arg
   785                 790                 795                 800
   Leu Arg Leu Leu Gly Leu Cys Gln Gly Leu Gly Ile Asp Gly Pro Ser
                   805                 810                 815
   Tyr Pro Val Ala His Phe Leu Ser His Phe Lys
               820                 825

<210>  69
<211>  2364
<212>  PRT
<213>  Homo sapiens

<400>  69

Met Thr Thr Thr Val Ala Thr Asp Tyr Asp Asn Ile Glu Ile Gln Gln
1               5                   10                  15

Gln Tyr Ser Asp Val Asn Asn Arg Trp Asp Val Asp Asp Trp Asp Asn

20                  25                  30

Glu Asn Ser Ser Ala Arg Leu Phe Glu Arg Ser Arg Ile Lys Ala Leu
        35                  40                  45

Ala Asp Glu Arg Glu Ala Val Gln Lys Lys Thr Phe Thr Lys Trp Val
    50                  55                  60

Asn Ser His Leu Ala Arg Val Ser Cys Arg Ile Thr Asp Leu Tyr Thr
65                  70                  75                  80

Asp Leu Arg Asp Gly Arg Met Leu Ile Lys Leu Leu Glu Val Leu Ser
                85                  90                  95

Gly Glu Arg Leu Pro Lys Pro Thr Lys Gly Arg Met Arg Ile His Cys
            100                 105                 110

Leu Glu Asn Val Asp Lys Ala Leu Gln Phe Leu Lys Glu Gln Arg Val
        115                 120                 125

His Leu Glu Asn Met Gly Ser His Asp Ile Val Asp Gly Asn His Arg
    130                 135                 140

Leu Thr Leu Gly Leu Ile Trp Thr Ile Ile Leu Arg Phe Gln Ile Gln
145                 150                 155                 160

Asp Ile Ser Val Glu Thr Glu Asp Asn Lys Glu Lys Lys Ser Ala Lys
                165                 170                 175

Asp Ala Leu Leu Leu Trp Cys Gln Met Lys Thr Ala Gly Tyr Pro Asn
            180                 185                 190

Val Asn Ile His Asn Phe Thr Thr Ser Trp Arg Asp Gly Met Ala Phe
        195                 200                 205

Asn Ala Leu Ile His Lys His Arg Pro Asp Leu Ile Asp Phe Asp Lys
    210                 215                 220

Leu Lys Lys Ser Asn Ala His Tyr Asn Leu Gln Asn Ala Phe Asn Leu
225                 230                 235                 240

Ala Glu Gln His Leu Gly Leu Thr Lys Leu Leu Asp Pro Glu Asp Ile
                245                 250                 255

Ser Val Asp His Pro Asp Glu Lys Ser Ile Ile Thr Tyr Val Val Thr
            260                 265                 270

Tyr Tyr His Tyr Phe Ser Lys Met Lys Ala Leu Ala Val Glu Gly Lys
        275                 280                 285

Arg Ile Gly Lys Val Leu Asp Asn Ala Ile Glu Thr Glu Lys Met Ile
    290                 295                 300

Glu Lys Tyr Glu Ser Leu Ala Ser Asp Leu Leu Glu Trp Ile Glu Gln
305                 310                 315                 320

Thr Ile Ile Ile Leu Asn Asn Arg Lys Phe Ala Asn Ser Leu Val Gly
                325                 330                 335

Val Gln Gln Gln Leu Gln Ala Phe Asn Thr Tyr Arg Thr Val Glu Lys
            340                 345                 350

Pro Pro Lys Phe Thr Glu Lys Gly Asn Leu Glu Val Leu Leu Phe Thr
        355                 360                 365

Ile Gln Ser Lys Met Arg Ala Asn Asn Gln Lys Val Tyr Met Pro Arg
    370                 375                 380

Glu Gly Lys Leu Ile Ser Asp Ile Asn Lys Ala Trp Glu Arg Leu Glu
385                 390                 395                 400

Lys Ala Glu His Glu Arg Glu Leu Ala Leu Arg Asn Glu Leu Ile Arg
                405                 410                 415

Gln Glu Lys Leu Glu Gln Leu Ala Arg Arg Phe Asp Arg Lys Ala Ala
            420                 425                 430

Met Arg Glu Thr Trp Leu Ser Glu Asn Gln Arg Leu Val Ser Gln Asp
        435                 440                 445

Asn Phe Gly Phe Asp Leu Pro Ala Val Glu Ala Ala Thr Lys Lys His
    450                 455                 460

Glu Ala Ile Glu Thr Asp Ile Ala Ala Tyr Glu Glu Arg Val Gln Ala
465                 470                 475                 480

Val Val Ala Val Ala Arg Glu Leu Glu Ala Glu Asn Tyr His Asp Ile
                485                 490                 495

Lys Arg Ile Thr Ala Arg Lys Asp Asn Val Ile Arg Leu Trp Glu Tyr
            500                 505                 510

Leu Leu Glu Leu Leu Arg Ala Arg Arg Gln Arg Leu Glu Met Asn Leu
        515                 520                 525

Gly Leu Gln Lys Ile Phe Gln Glu Met Leu Tyr Ile Met Asp Trp Met
    530                 535                 540

Asp Glu Met Lys Val Leu Val Leu Ser Gln Asp Tyr Gly Lys His Leu
545                 550                 555                 560

Leu Gly Val Glu Asp Leu Leu Gln Lys His Thr Leu Val Glu Ala Asp
                565                 570                 575

Ile Gly Ile Gln Ala Glu Arg Val Arg Gly Val Asn Ala Ser Ala Gln
            580                 585                 590

Lys Phe Ala Thr Asp Gly Glu Gly Tyr Lys Pro Cys Asp Pro Gln Val
        595                 600                 605

Ile Arg Asp Arg Val Ala His Met Glu Phe Cys Tyr Gln Glu Leu Cys
    610                 615                 620

Gln Leu Ala Ala Glu Arg Arg Ala Arg Leu Glu Glu Ser Arg Arg Leu
625                 630                 635                 640

Trp Lys Phe Phe Trp Glu Met Ala Glu Glu Glu Gly Trp Ile Arg Glu
                645                 650                 655

Lys Glu Lys Ile Leu Ser Ser Asp Asp Tyr Gly Lys Asp Leu Thr Ser
            660                 665                 670

Val Met Arg Leu Leu Ser Lys His Arg Ala Phe Glu Asp Glu Met Ser
        675                 680                 685

Gly Arg Ser Gly His Phe Glu Gln Ala Ile Lys Glu Gly Glu Asp Met
    690                 695                 700

Ile Ala Glu Glu His Phe Gly Ser Glu Lys Ile Arg Glu Arg Ile Ile
705                 710                 715                 720

Tyr Ile Arg Glu Gln Trp Ala Asn Leu Glu Gln Leu Ser Ala Ile Arg
                725                 730                 735

Lys Lys Arg Leu Glu Glu Ala Ser Leu Leu His Gln Phe Gln Ala Asp
            740                 745                 750

Ala Asp Asp Ile Asp Ala Trp Met Leu Asp Ile Leu Lys Ile Val Ser
        755                 760                 765

Ser Ser Asp Val Gly His Asp Glu Tyr Ser Thr Gln Ser Leu Val Lys
    770                 775                 780

Lys His Lys Asp Val Ala Glu Glu Ile Ala Asn Tyr Arg Pro Thr Leu
785                 790                 795                 800

Asp Thr Leu His Glu Gln Ala Ser Ala Leu Pro Gln Glu His Ala Glu
                805                 810                 815

Ser Pro Asp Val Arg Gly Arg Leu Ser Gly Ile Glu Glu Arg Tyr Lys
            820                 825                 830

Glu Val Ala Glu Leu Thr Arg Leu Arg Lys Gln Ala Leu Gln Asp Thr
        835                 840                 845

Leu Ala Leu Tyr Lys Met Phe Ser Glu Ala Asp Ala Cys Glu Leu Trp
    850                 855                 860

Ile Asp Glu Lys Glu Gln Trp Leu Asn Asn Met Gln Ile Pro Glu Lys
865                 870                 875                 880

Leu Glu Asp Leu Glu Val Ile Gln His Arg Phe Glu Ser Leu Glu Pro
                885                 890                 895

Glu Met Asn Asn Gln Ala Ser Arg Val Ala Val Val Asn Gln Ile Ala
            900                 905                 910

Arg Gln Leu Met His Ser Gly His Pro Ser Glu Lys Glu Ile Lys Ala
        915                 920                 925

Gln Gln Asp Lys Leu Asn Thr Arg Trp Ser Gln Phe Arg Glu Leu Val
    930                 935                 940

Asp Arg Lys Lys Asp Ala Leu Leu Ser Ala Leu Ser Ile Gln Asn Tyr
945                 950                 955                 960

His Leu Glu Cys Asn Glu Thr Lys Ser Trp Ile Arg Glu Lys Thr Lys
                965                 970                 975

Val Ile Glu Ser Thr Gln Asp Leu Gly Asn Asp Leu Ala Gly Val Met
            980                 985                 990

Ala Leu Gln Arg Lys Leu Thr Gly  Met Glu Arg Asp Leu  Val Ala Ile
        995                 1000                1005

Glu Ala  Lys Leu Ser Asp Leu  Gln Lys Glu Ala Glu  Lys Leu Glu
    1010                1015                1020

Ser Glu  His Pro Asp Gln Ala  Gln Ala Ile Leu Ser  Arg Leu Ala

1025                1030                1035

Glu Ile Ser Asp Val Trp Glu Glu Met Lys Thr Thr Leu Lys Asn
    1040                1045                1050

Arg Glu Ala Ser Leu Gly Glu Ala Ser Lys Leu Gln Gln Phe Leu
    1055                1060                1065

Arg Asp Leu Asp Asp Phe Gln Ser Trp Leu Ser Arg Thr Gln Thr
    1070                1075                1080

Ala Ile Ala Ser Glu Asp Met Pro Asn Thr Leu Thr Glu Ala Glu
    1085                1090                1095

Lys Leu Leu Thr Gln His Glu Asn Ile Lys Asn Glu Ile Asp Asn
    1100                1105                1110

Tyr Glu Glu Asp Tyr Gln Lys Met Arg Asp Met Gly Glu Met Val
    1115                1120                1125

Thr Gln Gly Gln Thr Asp Ala Gln Tyr Met Phe Leu Arg Gln Arg
    1130                1135                1140

Leu Gln Ala Leu Asp Thr Gly Trp Asn Glu Leu His Lys Met Trp
    1145                1150                1155

Glu Asn Arg Gln Asn Leu Leu Ser Gln Ser His Ala Tyr Gln Gln
    1160                1165                1170

Phe Leu Arg Asp Thr Lys Gln Ala Glu Ala Phe Leu Asn Asn Gln
    1175                1180                1185

Glu Tyr Val Leu Ala His Thr Glu Met Pro Thr Thr Leu Glu Gly
    1190                1195                1200

Ala Glu Ala Ala Ile Lys Lys Gln Glu Asp Phe Met Thr Thr Met
    1205                1210                1215

Asp Ala Asn Glu Glu Lys Ile Asn Ala Val Val Glu Thr Gly Arg
    1220                1225                1230

Arg Leu Val Ser Asp Gly Asn Ile Asn Ser Asp Arg Ile Gln Glu
    1235                1240                1245

Lys Val Asp Ser Ile Asp Asp Arg His Arg Lys Asn Arg Glu Thr
    1250                1255                1260

Ala Ser Glu Leu Leu Met Arg Leu Lys Asp Asn Arg Asp Leu Gln
    1265                1270                1275

Lys Phe Leu Gln Asp Cys Gln Glu Leu Ser Leu Trp Ile Asn Glu
    1280                1285                1290

Lys Met Leu Thr Ala Gln Asp Met Ser Tyr Asp Glu Ala Arg Asn
    1295                1300                1305

Leu His Ser Lys Trp Leu Lys His Gln Ala Phe Met Ala Glu Leu
    1310                1315                1320

Ala Ser Asn Lys Glu Trp Leu Asp Lys Ile Glu Lys Glu Gly Met
    1325                1330                1335

Gln Leu Ile Ser Glu Lys Pro Glu Thr Glu Ala Val Val Lys Glu
    1340                1345                1350

Lys Leu Thr Gly Leu His Lys Met Trp Glu Val Leu Glu Ser Thr
    1355                1360                1365

Thr Gln Thr Lys Ala Gln Arg Leu Phe Asp Ala Asn Lys Ala Glu
    1370                1375                1380

Leu Phe Thr Gln Ser Cys Ala Asp Leu Asp Lys Trp Leu His Gly
    1385                1390                1395

Leu Glu Ser Gln Ile Gln Ser Asp Asp Tyr Gly Lys Asp Leu Thr
    1400                1405                1410

Ser Val Asn Ile Leu Leu Lys Lys Gln Gln Met Leu Glu Asn Gln
    1415                1420                1425

Met Glu Val Arg Lys Lys Glu Ile Glu Glu Leu Gln Ser Gln Ala
    1430                1435                1440

Gln Ala Leu Ser Gln Glu Gly Lys Ser Thr Asp Glu Val Asp Ser
    1445                1450                1455

Lys Arg Leu Thr Val Gln Thr Lys Phe Met Glu Leu Leu Glu Pro
    1460                1465                1470

Leu Asn Glu Arg Lys His Asn Leu Leu Ala Ser Lys Glu Ile His
    1475                1480                1485

Gln Phe Asn Arg Asp Val Glu Asp Glu Ile Leu Trp Val Gly Glu
    1490                1495                1500

Arg Met Pro Leu Ala Thr Ser Thr Asp His Gly His Asn Leu Gln
    1505                1510                1515

Thr Val Gln Leu Leu Ile Lys Lys Asn Gln Thr Leu Gln Lys Glu
    1520                1525                1530

Ile Gln Gly His Gln Pro Arg Ile Asp Asp Ile Phe Glu Arg Ser
    1535                1540                1545

Gln Asn Ile Val Thr Asp Ser Ser Ser Leu Ser Ala Glu Ala Ile
    1550                1555                1560

Arg Gln Arg Leu Ala Asp Leu Lys Gln Leu Trp Gly Leu Leu Ile
    1565                1570                1575

Glu Glu Thr Glu Lys Arg His Arg Arg Leu Glu Glu Ala His Arg
    1580                1585                1590

Ala Gln Gln Tyr Tyr Phe Asp Ala Ala Glu Ala Glu Ala Trp Met
    1595                1600                1605

Ser Glu Gln Glu Leu Tyr Met Met Ser Glu Glu Lys Ala Lys Asp
    1610                1615                1620

Glu Gln Ser Ala Val Ser Met Leu Lys Lys His Gln Ile Leu Glu
    1625                1630                1635

Gln Ala Val Glu Asp Tyr Ala Glu Thr Val His Gln Leu Ser Lys
    1640                1645                1650

Thr Ser Arg Ala Leu Val Ala Asp Ser His Pro Glu Ser Glu Arg
    1655                1660                1665

Ile Ser Met Arg Gln Ser Lys Val Asp Lys Leu Tyr Ala Gly Leu
    1670                1675                1680

Lys Asp Leu Ala Glu Glu Arg Arg Gly Lys Leu Asp Glu Arg His
    1685                1690                1695

Arg Leu Phe Gln Leu Asn Arg Glu Val Asp Asp Leu Glu Gln Trp
    1700                1705                1710

Ile Ala Glu Arg Glu Val Val Ala Gly Ser His Glu Leu Gly Gln
    1715                1720                1725

Asp Tyr Glu His Val Thr Met Leu Gln Glu Arg Phe Arg Glu Phe
    1730                1735                1740

Ala Arg Asp Thr Gly Asn Ile Gly Gln Glu Arg Val Asp Thr Val
    1745                1750                1755

Asn His Leu Ala Asp Glu Leu Ile Asn Ser Gly His Ser Asp Ala
    1760                1765                1770

Ala Thr Ile Ala Glu Trp Lys Asp Gly Leu Asn Glu Ala Trp Ala
    1775                1780                1785

Asp Leu Leu Glu Leu Ile Asp Thr Arg Thr Gln Ile Leu Ala Ala
    1790                1795                1800

Ser Tyr Glu Leu His Lys Phe Tyr His Asp Ala Lys Glu Ile Phe
    1805                1810                1815

Gly Arg Ile Gln Asp Lys His Lys Lys Leu Pro Glu Glu Leu Gly
    1820                1825                1830

Arg Asp Gln Asn Thr Val Glu Thr Leu Gln Arg Met His Thr Thr
    1835                1840                1845

Phe Glu His Asp Ile Gln Ala Leu Gly Thr Gln Val Arg Gln Leu
    1850                1855                1860

Gln Glu Asp Ala Ala Arg Leu Gln Ala Ala Tyr Ala Gly Asp Lys
    1865                1870                1875

Ala Asp Asp Ile Gln Lys Arg Glu Asn Glu Val Leu Glu Ala Trp
    1880                1885                1890

Lys Ser Leu Leu Asp Ala Cys Glu Ser Arg Arg Val Arg Leu Val
    1895                1900                1905

Asp Thr Gly Asp Lys Phe Arg Phe Phe Ser Met Val Arg Asp Leu
    1910                1915                1920

Met Leu Trp Met Glu Asp Val Ile Arg Gln Ile Glu Ala Gln Glu
    1925                1930                1935

Lys Pro Arg Asp Val Ser Ser Val Glu Leu Leu Met Asn Asn His
    1940                1945                1950

Gln Gly Ile Lys Ala Glu Ile Asp Ala Arg Asn Asp Ser Phe Thr
    1955                1960                1965

Thr Cys Ile Glu Leu Gly Lys Ser Leu Leu Ala Arg Lys His Tyr

1970                1975                1980

Ala Ser Glu Glu Ile Lys Glu Lys Leu Leu Gln Leu Thr Glu Lys
    1985                1990                1995

Arg Lys Glu Met Ile Asp Lys Trp Glu Asp Arg Trp Glu Trp Leu
    2000                2005                2010

Arg Leu Ile Leu Glu Val His Gln Phe Ser Arg Asp Ala Ser Val
    2015                2020                2025

Ala Glu Ala Trp Leu Leu Gly Gln Glu Pro Tyr Leu Ser Ser Arg
    2030                2035                2040

Glu Ile Gly Gln Ser Val Asp Glu Val Glu Lys Leu Ile Lys Arg
    2045                2050                2055

His Glu Ala Phe Glu Lys Ser Ala Ala Thr Trp Asp Glu Arg Phe
    2060                2065                2070

Ser Ala Leu Glu Arg Leu Thr Thr Leu Glu Leu Leu Glu Val Arg
    2075                2080                2085

Arg Gln Gln Glu Glu Glu Glu Arg Lys Arg Arg Pro Pro Ser Pro
    2090                2095                2100

Glu Pro Ser Thr Lys Val Ser Glu Glu Ala Glu Ser Gln Gln Gln
    2105                2110                2115

Trp Asp Thr Ser Lys Gly Glu Gln Val Ser Gln Asn Gly Leu Pro
    2120                2125                2130

Ala Glu Gln Gly Ser Pro Arg Met Ala Glu Thr Val Asp Thr Ser
    2135                2140                2145

Glu Met Val Asn Gly Ala Thr Glu Gln Arg Thr Ser Ser Lys Glu
    2150                2155                2160

Ser Ser Pro Ile Pro Ser Pro Thr Ser Asp Arg Lys Ala Lys Thr
    2165                2170                2175

Ala Leu Pro Ala Gln Ser Ala Ala Thr Leu Pro Ala Arg Thr Gln
    2180                2185                2190

Glu Thr Pro Ser Ala Gln Met Glu Gly Phe Leu Asn Arg Lys His
    2195                2200                2205

Glu Trp Glu Ala His Asn Lys Lys Ala Ser Ser Arg Ser Trp His
    2210                2215                2220

Asn Val Tyr Cys Val Ile Asn Asn Gln Glu Met Gly Phe Tyr Lys
    2225                2230                2235

Asp Ala Lys Thr Ala Ala Ser Gly Ile Pro Tyr His Ser Glu Val
    2240                2245                2250

Pro Val Ser Leu Lys Glu Ala Val Cys Glu Val Ala Leu Asp Tyr
    2255                2260                2265

Lys Lys Lys Lys His Val Phe Lys Leu Arg Leu Asn Asp Gly Asn
    2270                2275                2280

Glu Tyr Leu Phe Gln Ala Lys Asp Asp Glu Glu Met Asn Thr Trp
    2285                2290                2295

Ile Gln Ala Ile Ser Ser Ala Ile Ser Ser Asp Lys His Glu Val
    2300                2305                2310

Ser Ala Ser Thr Gln Ser Thr Pro Ala Ser Ser Arg Ala Gln Thr
    2315                2320                2325

Leu Pro Thr Ser Val Val Thr Ile Thr Ser Glu Ser Ser Pro Gly
    2330                2335                2340

Lys Arg Glu Lys Asp Lys Glu Lys Asp Lys Glu Lys Arg Phe Ser
    2345                2350                2355

Leu Phe Gly Lys Lys Lys
    2360

<210>  70
<211>  1148
<212>  PRT
<213>  Homo sapiens

<400>  70

Met Ala Ser Ala Pro Ala Glu Ala Glu Thr Arg Gln Arg Leu Leu Arg
1               5                   10                  15

Thr Val Lys Lys Glu Val Lys Gln Ile Met Glu Glu Ala Val Thr Arg
            20                  25                  30

Lys Phe Val His Glu Asp Ser Ser His Ile Ile Ser Phe Cys Ala Ala
        35                  40                  45

Val Glu Ala Cys Val Leu His Gly Leu Arg Arg Arg Ala Ala Gly Phe
    50                  55                  60

Leu Arg Ser Asn Lys Ile Ala Ala Leu Phe Met Lys Val Gly Lys Asn
65                  70                  75                  80

Phe Pro Pro Ala Glu Asp Leu Ser Arg Lys Val Gln Asp Leu Glu Gln
                85                  90                  95

Leu Ile Glu Ser Ala Arg Asn Gln Ile Gln Gly Leu Gln Glu Asn Val
            100                 105                 110

Arg Lys Leu Pro Lys Leu Pro Asn Leu Ser Pro Leu Ala Ile Lys His
        115                 120                 125

Leu Trp Ile Arg Thr Ala Leu Phe Glu Lys Val Leu Asp Lys Ile Val
    130                 135                 140

His Tyr Leu Val Glu Asn Ser Ser Lys Tyr Tyr Glu Lys Glu Ala Leu
145                 150                 155                 160

Leu Met Asp Pro Val Asp Gly Pro Ile Leu Ala Ser Leu Leu Val Gly
                165                 170                 175

Pro Cys Ala Leu Glu Tyr Thr Lys Met Lys Thr Ala Asp His Phe Trp
            180                 185                 190

Thr Asp Pro Ser Ala Asp Glu Leu Val Gln Arg His Arg Ile His Ser
        195                 200                 205

Ser His Val Arg Gln Asp Ser Pro Thr Lys Arg Pro Ala Leu Cys Ile
    210                 215                 220

Gln Lys Arg His Ser Ser Gly Ser Met Asp Asp Arg Pro Ser Leu Ser
225                 230                 235                 240

Ala Arg Asp Tyr Val Glu Ser Leu His Gln Asn Ser Arg Ala Thr Leu
                245                 250                 255

Leu Tyr Gly Lys Asn Asn Val Leu Val Gln Pro Arg Asp Asp Met Glu
            260                 265                 270

Ala Val Pro Gly Tyr Leu Ser Leu His Gln Thr Ala Asp Val Met Thr
        275                 280                 285

Leu Lys Trp Thr Pro Asn Gln Leu Met Asn Gly Ser Val Gly Asp Leu
    290                 295                 300

Asp Tyr Glu Lys Ser Val Tyr Trp Asp Tyr Ala Met Thr Ile Arg Leu
305                 310                 315                 320

Glu Glu Ile Val Tyr Leu His Cys His Gln Gln Val Asp Ser Gly Gly
                325                 330                 335

Thr Val Val Leu Val Ser Gln Asp Gly Ile Gln Arg Pro Pro Phe Arg
            340                 345                 350

Phe Pro Lys Gly Gly His Leu Leu Gln Phe Leu Ser Cys Leu Glu Asn
        355                 360                 365

Gly Leu Leu Pro His Gly Gln Leu Asp Pro Pro Leu Trp Ser Gln Arg
    370                 375                 380

Gly Lys Gly Lys Val Phe Pro Lys Leu Arg Lys Arg Ser Pro Gln Gly
385                 390                 395                 400

Ser Ala Glu Ser Thr Ser Ser Asp Lys Asp Asp Asp Glu Ala Thr Asp
                405                 410                 415

Tyr Val Phe Arg Ile Ile Tyr Pro Gly Met Gln Ser Glu Phe Val Ala
            420                 425                 430

Pro Asp Phe Leu Gly Ser Thr Ser Ser Val Ser Val Gly Pro Ala Trp
        435                 440                 445

Met Met Val Pro Ala Gly Arg Ser Met Leu Val Val Ala Arg Gly Ser
    450                 455                 460

Gln Trp Glu Pro Ala Arg Trp Asp Thr Thr Leu Pro Thr Pro Ser Pro
465                 470                 475                 480

Lys Glu Gln Pro Pro Met Pro Gln Asp Leu Met Asp Val Ser Val Ser
                485                 490                 495

Asn Leu Pro Ser Leu Trp Gln Pro Ser Pro Arg Lys Ser Ser Cys Ser
            500                 505                 510

Ser Cys Ser Gln Ser Gly Ser Ala Asp Gly Ser Ser Thr Asn Gly Cys
        515                 520                 525

Asn His Glu Arg Ala Pro Leu Lys Leu Leu Cys Asp Asn Met Lys Tyr
    530                 535                 540

Gln Ile Leu Ser Arg Ala Phe Tyr Gly Trp Leu Ala Tyr Cys Arg His
545                 550                 555                 560

Leu Ser Thr Val Arg Thr His Leu Ser Ala Leu Val Asn His Met Ile
                565                 570                 575

Val Ser Pro Asp Leu Pro Cys Asp Ala Gly Gln Gly Leu Thr Ala Arg
            580                 585                 590

Ile Trp Glu Gln Tyr Leu His Asp Ser Thr Ser Tyr Glu Glu Gln Glu
        595                 600                 605

Leu Leu Arg Leu Ile Tyr Tyr Gly Gly Ile Gln Pro Glu Ile Arg Lys
    610                 615                 620

Ala Val Trp Pro Phe Leu Leu Gly His Tyr Gln Phe Gly Met Thr Glu
625                 630                 635                 640

Thr Glu Arg Lys Glu Val Asp Glu Gln Ile His Ala Cys Tyr Ala Gln
                645                 650                 655

Thr Met Ala Glu Trp Leu Gly Cys Glu Ala Ile Val Arg Gln Arg Glu
            660                 665                 670

Arg Glu Ser His Ala Ala Ala Leu Ala Lys Cys Ser Ser Gly Ala Ser
        675                 680                 685

Leu Asp Ser His Leu His Arg Met Leu His Arg Asp Ser Thr Ile Ser
    690                 695                 700

Asn Glu Ser Ser Gln Ser Cys Ser Ser Gly Arg Gln Asn Ile Arg Leu
705                 710                 715                 720

His Ser Asp Ser Ser Ser Ser Thr Gln Val Phe Glu Ser Val Asp Glu
                725                 730                 735

Val Glu Gln Val Glu Ala Glu Gly Arg Leu Glu Glu Lys Gln Pro Lys
            740                 745                 750

Ile Pro Asn Gly Asn Leu Val Asn Gly Thr Cys Ser Pro Asp Ser Gly
        755                 760                 765

His Pro Ser Ser His Asn Phe Ser Ser Gly Leu Ser Glu His Ser Glu
    770                 775                 780

Pro Ser Leu Ser Thr Glu Asp Ser Val Leu Asp Ala Gln Arg Asn Thr
785                 790                 795                 800

Pro Thr Val Leu Arg Pro Arg Asp Gly Ser Val Asp Asp Arg Gln Ser

                805                 810                 815

Ser Glu Ala Thr Thr Ser Gln Asp Glu Ala Pro Arg Glu Glu Leu Ala
            820                 825                 830

Val Gln Asp Ser Leu Glu Ser Asp Leu Leu Ala Asn Glu Ser Met Asp
        835                 840                 845

Glu Phe Met Ser Ile Thr Gly Ser Leu Asp Met Ala Leu Pro Glu Lys
    850                 855                 860

Asp Asp Val Val Met Glu Gly Trp Arg Ser Ser Glu Thr Glu Lys His
865                 870                 875                 880

Gly Gln Ala Asp Ser Glu Asp Asn Leu Ser Glu Glu Pro Glu Met Glu
                885                 890                 895

Ser Leu Phe Pro Ala Leu Ala Ser Leu Ala Val Thr Thr Ser Ala Asn
            900                 905                 910

Glu Val Ser Pro Val Ser Ser Ser Gly Val Thr Tyr Ser Pro Glu Leu
        915                 920                 925

Leu Asp Leu Tyr Thr Val Asn Leu His Arg Ile Glu Lys Asp Val Gln
    930                 935                 940

Arg Cys Asp Arg Asn Tyr Trp Tyr Phe Thr Pro Ala Asn Leu Glu Lys
945                 950                 955                 960

Leu Arg Asn Ile Met Cys Ser Tyr Ile Trp Gln His Ile Glu Ile Gly
                965                 970                 975

Tyr Val Gln Gly Met Cys Asp Leu Leu Ala Pro Leu Leu Val Ile Leu
            980                 985                 990

Asp Asp Glu Ala Leu Ala Phe Ser Cys Phe Thr Glu Leu Met Lys Arg
        995                 1000                1005

Met Asn Gln Asn Phe Pro His Gly Gly Ala Met Asp Thr His Phe
    1010                1015                1020

Ala Asn Met Arg Ser Leu Ile Gln Ile Leu Asp Ser Glu Leu Phe
    1025                1030                1035

Glu Leu Met His Gln Asn Gly Asp Tyr Thr His Phe Tyr Phe Cys
    1040                1045                1050

Tyr Arg Trp Phe Leu Leu Asp Phe Lys Arg Glu Leu Val Tyr Asp
    1055                1060                1065

Asp Val Phe Leu Val Trp Glu Thr Ile Trp Ala Ala Lys His Val
    1070                1075                1080

Ser Ser Ala His Tyr Val Leu Phe Ile Ala Leu Ala Leu Val Glu
    1085                1090                1095

Val Tyr Arg Asp Ile Ile Leu Glu Asn Asn Met Asp Phe Thr Asp
    1100                1105                1110

Ile Ile Lys Phe Phe Asn Glu Met Ala Glu Arg His Asn Thr Lys
    1115                1120                1125

Gln Val Leu Lys Leu Ala Arg Asp Leu Val Tyr Lys Val Gln Thr
    1130                1135                1140

Leu Ile Glu Asn Lys
    1145

## Description

### Field of the invention

The present invention relates to methods of diagnosing heart muscle tissue damage, determining the extent of heart muscle tissue damage and differentiating between atrial heart muscle damage and ventricular heart muscle damage in a patient. Furthermore, the invention relates to a kit and a marker panel for use in these methods.

### Background

Heart diseases, especially coronary artery diseases (CAD), are the leading causes of morbidity and mortality in developed countries. Based on population statistics it can be calculated that approximately 310 heart attacks per hundred-thousand people older than 18 years-old and approximately 150 deaths per hundred-thousand adults can be expected each year in developed nations. In the cardiovascular medicine, enzymes and other proteins that are released from the dying heart have been identified as biomarker. Particularly, they are measurable for example after a heart attack in high concentration in blood. The first biomarkers for heart muscle damage have been described in the 1950'. Among those Aspartate-Aminotransferase (AST) and Lactate-Dehydrogenase (LDH) were described. In the 1970' and 1980', further biomarkers were discovered, e.g. Creatine Kinase (CK), its isoenzyme Creatine Kinase-Myocardial band (CK-MB), myoglobin and troponin (troponin T and troponin I). The troponins and CKMB are still in clinical use for the identification of heart muscle tissue damages. The measurement of the concentration of these biomarkers in the blood is usually periodically repeated since an increase and/or a decrease in the concentration of these biomarkers in the blood allow drawing conclusions about the occurrence or the beginning of a heart attack, the extent of the heart attack and the success of a therapy against heart attack.

All biomarkers previously cited have in common that they do not allow differentiation between atrial heart muscle damage (in the heart atrium) and ventricular heart muscle damage (in the ventricle). No specific biomarker for atrial heart muscle damage or for ventricular heart muscle damage has been discovered up to now. Such a specific biomarker would be highly relevant, particularly in the field of heart surgery in the context of postoperative care.

In patients with atrial fibrillation, a cardiac arrhythmia, an ablation of the heart atrium to treat the atrial fibrillation, is often performed during cardiac surgery. Ablation lines are set in the heart atrium following a specific scheme to prevent the transmission of electrical impulses that cause the atria to fibrillate rather than contract in a regular pattern. This ablation can be performed as endocardial cryoablation after opening of the right and left atrium or as epicardial radiofrequency ablation of the pulmonary veins as a concomitant procedure. Both, the endocardial cryoablation and the epicardial radiofrequency ablation cause damage to the heart muscle cells. Thus, the treatment of atrial fibrillation by ablation of a part of heart muscle tissue includes the destruction of heart muscle cells of the atrium and thereby causes a postoperative increase of unspecific biomarkers (CK-MB and Troponin) in the blood. Since the biomarkers used so far do not differentiate between atrial heart muscle damage and ventricular heart muscle damage, the postoperative evaluation to determine if the increase of the biomarkers is due to the damage occasioned by the ablation lines in the atrium or if it is due to an additional heart muscle damage of the ventricle, is not possible. For these reasons, patients often need to undergo further invasive diagnosis, e.g. coronary angiography by means of a heart catheter, in order, for example, to exclude a perioperative heart attack. Beside this indication, a biomarker reflecting an atrial damage could be of high interest as a parameter to monitor the success of an ablation therapy, for instance after interventional, catheter-based ablation therapy.

Accordingly, there is demand for the implementation of an atrial biomarker for the specific detection of atrial damage. Such an atrial specific biomarker will avoid unnecessary invasive medical investigation in patients, especially in patients who have undergone a treatment by ablation.

### Summary of the Invention

The present invention relates to a method of diagnosing heart muscle tissue damage, preferably atrial heart muscle tissue damage, in a patient, wherein the method comprises determining the level of a biomarker for atrial heart muscle tissue damage selected from the group consisting of MYBPH (Uniprot Database entry: Q13203, protein sequence SEQ ID NO:7), MYBPHL (Uniprot Database entry: A2RUH7, protein sequence SEQ ID NO:3), MYOT (Uniprot Database entry: Q9UBF9, protein sequence SEQ ID NO:9), ASNSD1 (Uniprot Database entry: Q9NWL6, protein sequence SEQ ID NO:10), CHFR (Uniprot Database entry: Q96EP1, protein sequence SEQ ID NO:11), NTN1 (Uniprot Database entry: 095631, protein sequence SEQ ID NO:12), RFC5 (Uniprot Database entry: P38251, protein sequence SEQ ID NO:13), POLI (Uniprot Database entry: Q9UNA4, protein sequence SEQ ID NO:14), COMP (Uniprot Database entry: P49747, protein sequence SEQ ID NO:15), POF1B (Uniprot Database entry: Q8WVV4, protein sequence SEQ ID NO:16), PLA2G2A (Uniprot Database entry: P14555, protein sequence SEQ ID NO:17), HDAC10 (Uniprot Database entry: Q969S8, protein sequence SEQ ID NO:18), ASPG (Uniprot Database entry: Q86U10, protein sequence SEQ ID NO:19), FMOD (Uniprot Database entry: Q06828, protein sequence SEQ ID NO:20), CA13 (Uniprot Database entry: Q8N1Q1, protein sequence SEQ ID NO:21), CACNA2D2 (Uniprot Database entry: Q9NY47, protein sequence SEQ ID NO:22), GNL3L (Uniprot Database entry: Q9NY47, protein sequence SEQ ID NO:23), COL2A1 (Uniprot Database entry: P02458, protein sequence SEQ ID NO:24), PDLIM4 (Uniprot Database entry: P50479, protein sequence SEQ ID NO:25), LEPREL1 (Uniprot Database entry: Q8IVL5, protein sequence SEQ ID NO:26), OMD (Uniprot Database entry: Q99983, protein sequence SEQ ID NO:27), DGKZ (Uniprot Database entry: Q99983, protein sequence SEQ ID NO:28), NT5DC2 (Uniprot Database entry: Q9H857, protein sequence SEQ ID NO:29), ITLN1 (Uniprot Database entry: Q8WWA0, protein sequence SEQ ID NO:30), NTM (Uniprot Database entry: Q9P121, protein sequence SEQ ID NO:31), PRKG2 (Uniprot Database entry: Q13237, protein sequence SEQ ID NO:32), CHGB (Uniprot Database entry: P05060, protein sequence SEQ ID NO:33), FAM179A (Uniprot Database entry: Q6ZUX3, protein sequence SEQ ID NO:34), CKMT1A (Uniprot Database entry: P12532, protein sequence SEQ ID NO:35), CKMT1B (Uniprot Database entry: F8WCN3, protein sequence SEQ ID NO:36), LTBP3 (Uniprot Database entry: Q9NS15, protein sequence SEQ ID NO:37), METTL7B (Uniprot Database entry: Q6UX53, protein sequence SEQ ID NO:38), LTBP2 (Uniprot Database entry: Q14767, protein sequence SEQ ID NO:39), OGDHL (Uniprot Database entry: Q9ULD0, protein sequence SEQ ID NO:40), PAM (Uniprot Database entry: P19021, protein sequence SEQ ID NO:41), SBK3 (Uniprot Database entry: P0C264, protein sequence SEQ ID N0:42), SFRP1 (Uniprot Database entry: Q8N474, protein sequence SEQ ID NO:43), SGSm1 (Uniprot Database entry: Q9NS15, protein sequence SEQ ID NO:70) and/or (different) isoforms thereof in a sample obtained from said patient and comparing the level of the biomarker for atrial heart muscle tissue damage determined in said patient with the level of the same biomarker for atrial heart muscle tissue damage observed in a control individual, wherein if the level of the biomarker for atrial heart muscle tissue damage determined in said patient is increased compared to the level of the biomarker for atrial heart muscle tissue damage in the control individual, said patient is diagnosed with a heart muscle tissue damage.

In various embodiments of the method described herein, the isoforms of MYBPHL have protein sequences which have at least 70 % sequence identity with the protein sequence of MYBPHL, namely, at least 70 %, 75 %, 80 %, 85 %, 90 %, 92 %, 93 %, 94 %, 95 %, 96 %, 97 %, 98 %, 99% or 99.5 % sequence identity. For example, the isoforms of MYBPHL are selected from the group consisting of MYBPHL isoform1 (DNA sequence: SEQ ID NO:1, coding DNA sequence: SEQ ID NO:2, protein sequence: SEQ ID NO:3) and MYBPHL isoform 2 (DNA sequence: SEQ ID NO:4, coding DNA sequence: SEQ ID NO:5, protein sequence: SEQ ID NO:6). Generally, "isoform", as used herein, refers to a functionally similar protein that shares at least 70 % sequence identity, preferably at least 75 %, 80 %, 85 %, 90 %, 92 %, 93 %, 94 %, 95 %, 96 %, 97 %, 98 %, 99 % or 99.5 % sequence identity with the respective reference protein.

In certain embodiments of the method described herein, the method comprises evaluating the extent of the atrial heart muscle tissue damage in said patient by determining the ratio between the level of the biomarker for atrial heart muscle tissue damage in the sample obtained from said patient and the level of the biomarker for atrial heart muscle tissue damage in the control individual.

In various embodiments of the method described herein, the method further comprises determining the level of a further biomarker for heart muscle tissue damage, wherein the method is suitable to differentiate between atrial muscle tissue damage and ventricular muscle tissue damage. The further biomarker for heart muscle tissue damage is a biomarker different from the biomarkers for atrial heart muscle tissue damage mentioned above and can be a general biomarker for heart muscle tissue damage selected from the group consisting of CK-MB or Troponin. Alternatively or additionally, the further biomarker for heart muscle tissue damage is for example a biomarker specific for ventricular heart muscle tissue damage, for example FHL-2 (Uniprot Database entry: Q14192, protein sequence SEQ ID NO:44), SMYD2 (Uniprot Database entry: Q9NRG4, protein sequence SEQ ID NO:45), FASTKD1 (Uniprot Database entry: Q53R41, protein sequence SEQ ID NO:46), PRR33 (Uniprot Database entry: A8MZF0, protein sequence SEQ ID NO:47), SORBS2 (Uniprot Database entry: O94875, protein sequence SEQ ID NO:48), CRISPLD1 (Uniprot Database entry: Q9H336, protein sequence SEQ ID NO:49), NPHP4 (Uniprot Database entry: 075161, protein sequence SEQ ID NO:50), ANKRD2 (Uniprot Database entry: Q9GZV1, protein sequence SEQ ID NO:51), TMEM159 (Uniprot Database entry: Q96B96, protein sequence SEQ ID NO:52), ATP7A (Uniprot Database entry: Q04656, protein sequence SEQ ID NO:53), C12ORF73 (Uniprot Database entry: Q69YU5, protein sequence SEQ ID NO:54), NAV1 (Uniprot Database entry: Q8NEY1, protein sequence SEQ ID NO:8), ATP5B (Uniprot Database entry: P06576, protein sequence SEQ ID NO:55), HSPB7 (Uniprot Database entry: Q9UBY9, protein sequence SEQ ID NO:56), TMEM88 (Uniprot Database entry: Q6PEY1, protein sequence SEQ ID NO:57), WDR62 (Uniprot Database entry: O43379, protein sequence SEQ ID NO:58), ACTN3 (Uniprot Database entry: Q08043, protein sequence SEQ ID NO:59), TRIM72 (Uniprot Database entry: Q6ZMU5, protein sequence SEQ ID NO:60), EGFLAM (Uniprot Database entry: Q63HQ2, protein sequence SEQ ID NO:61), LRRC39 (Uniprot Database entry: Q96DD0, protein sequence SEQ ID NO:62), DPYSL4 (Uniprot Database entry: 014531, protein sequence SEQ ID NO:63), PFKFB2 (Uniprot Database entry: O60825, protein sequence SEQ ID NO:64), ABCB6 (Uniprot Database entry: Q9NP58, protein sequence SEQ ID NO:65), METTL2B (Uniprot Database entry: Q6P1Q9, protein sequence SEQ ID NO:66), METTL2A (Uniprot Database entry: Q96IZ6, protein sequence SEQ ID NO:67), CARNS1 (Uniprot Database entry: A5YM72, protein sequence SEQ ID NO:68), SPTBN1 (Uniprot Database entry: Q01082, protein sequence SEQ ID NO:69) or isoforms thereof.

In some embodiments of the method described herein, the sample is a body fluid, preferably blood serum or blood plasma.

In certain embodiments of the method described herein, the patient is a human.

In various embodiments of the method described herein, determining the level of the biomarker for atrial heart muscle tissue damage and/or determining the level of the further biomarker comprises determining the protein concentration of the biomarker for atrial heart muscle tissue damage and/or determining the protein concentration of the further biomarker.

In various embodiments of the method described herein, determining the level of the biomarker comprises determining the protein concentration of the biomarker, wherein the protein concentration is determined by an immunoassay, ELISA, mass spectrometry, chromatography, Western Blot, or gel electrophoresis, such as SDS-PAGE. Alternatively or additionally, circulating nucleic acids, in particular RNA, such as mRNA, may be determined. For nucleic acid detection, various nucleic acid capture and amplification methods may be used. Detection may for example be achieved by using specific detection probes that are detectably labeled. Such techniques are widely known in the art and can be chosen by those skilled in art based on their general knowledge.

In another aspect, the present invention concerns a kit for use in the method of the invention, wherein the kit comprises reagents for determining the level of the biomarker for atrial heart muscle tissue damage and reagents for determining the level of the further biomarker for heart muscle damage in a sample. In certain embodiments, the kit comprises reagents for an ELISA assay, such as a capture and a detection antibody specific for the biomarker of choice.

Moreover, the present invention relates to a marker panel comprising at least one biomarker for atrial heart muscle tissue damage and at least one further biomarker for heart muscle damage for use in the method of the invention. Also encompassed are kits for the detection of such a marker panel comprising at least one detection reagent specific for the at least one biomarker for atrial heart muscle tissue damage and at least one detection reagent for the at least one further biomarker for heart muscle damage.

The term "antibody" is used in the broadest sense and specifically covers, for example, monoclonal antibodies, polyclonal antibodies, antibodies with polyepitopic specificity, single chain antibodies, multi-specific antibodies and fragments of antibodies. Such antibodies can be chimeric, humanized, human and synthetic.

"Percent (%) sequence identity" or "Percent (%)"amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

### Brief Description of the Drawings

**Figure 1** shows the results of a gene expression analysis of the biomarker MYBPHL (Figure 1A) and the biomarker FHL2 (Figure 1B) in tissues taken from left atrium (LA), right atrium (RA), and left ventricle (LV) by means of a biopsy in the patients.
**Figure 2** shows the results of a gene expression analysis of the biomarkers MYBPHL, FHL2, MYBPC3 and MYBPH as well as the MYBPHL isoforms MYBPHL isoform 1 and MYBPHL isoform 2 in tissues taken from 9 left ventricle muscle tissues and 9 left atrium muscle tissues obtained by means of a biopsy in patients, wherein the gene expression analysis has been performed by qPCR.
**Figure 3** shows the separation of the amplification products of the biomarker MYBPHL isoform 2 in tissues taken from left atriums and from left ventricles, wherein the separation has been performed by gel electrophoresis.
**Figure 4** shows the separation of the amplification products of the biomarker MYBPHL isoform 2 in non-cardiac tissues, wherein the separation has been performed by gel electrophoresis.
**Figure 5** shows the results of the measurement of MYBPHL protein concentration in a blood plasma sample taken at periodical measurement time points over a defined period of time, wherein the plasma sample is taken from patients suffering from atrial fibrillation and undergoing an ablation.
**Figure 6** shows the results of the measurement of MYBPHL protein concentration in a blood plasma sample taken at periodical measurement time points over a defined period of time, wherein the plasma sample is taken from patients suffering from atrial fibrillation and undergoing an endocardial ablation (A) or an epicardial ablation (B).
**Figure 7** shows the fold-change in the MYBPHL protein concentration in a blood plasma according to Figure 6a.
**Figure 8** shows the results of the measurement of MYBPHL protein concentration in a blood plasma sample taken from a group of patients suffering from ST-elevation myocardial infarction, e.g. patients with a definitive damage in the ventricle. The blood samples were taken at two measurement time points, one before the surgical intervention and one 72 hours after the surgical intervention, wherein the surgical intervention was performed by means of a heart catheter.
**Figure 9A** shows the results of the measurement of CK-MB protein concentration in the blood samples obtained from the same patient group as in example 2. **Figure 9B** shows the fold-change in the CK-MB protein concentration in the blood samples according to Figure 9A.
**Figure 10** shows a correlation between the MYBPHL concentration values in a blood plasma sample of 12 patients who underwent an ablation and the CK-MB concentration values in the blood plasma sample of the same patients.
**Figure 11** shows a representation of the ratio between the fold-change of MYBPHL and the fold-change of FHL-2. On the two left columns of the representation, the results for patients suffering from ST-elevation myocardial infarction are shown, while on the six left columns of the representation, the results for patients suffering from atrial fibrillation and undergoing an ablation are shown.

### Detailed Description

The invention is based on the inventors' surprising finding that biomarkers specific for heart muscle tissue damage, preferably atrial heart muscle tissue damage, selected from the group consisting of MYBPH, MYBPHL, MYOT, ASNSD1, CHFR, NTN1, RFC5, POLI, COMP, POF1B, PLA2G2A, HDAC10, ASPG, FMOD, CA13, CACNA2D2, GNL3L, COL2A1, PDLIM4, LEPREL1, OMD, DGKZ, NT5DC2, ITLN1, NTM, PRKG2, CHGB, FAM179A, CKMT1A, CKMT1B, LTBP3, SGSM1, METTL7B, LTBP2, OGDHL, PAM, SBK3, SFRP1 and/or isoforms thereof are detectable in blood samples of patients with heart muscle tissue damage, whereas those specific biomarkers are not detected in healthy and benign control tissue and thus can serve as markers for heart muscle tissue damage, preferably for atrial heart muscle tissue damage. Isoforms of a biomarker have protein sequences which may have at least 70 % sequence identity with the protein sequence of the biomarker, namely, 70 %, 75 %, 80 %, 85 %, 90 %, 92 %, 93 %, 94 %, 95 %, 96 %, 97 %, 98 % or 99 % sequence identity and which typically share certain aspects of functionality or even have identical functionality. It is further understood that when reference is made herein to protein database entries, said reference is intended to cover all isoforms listed under said entry. Similarly, when reference is made herein to a specific amino acid sequence, it is understood that said reference is by way of example only, and it is intended that different isoforms of said protein that may differ in their amino acid sequence, and may, for example, be listed under the database entries referenced herein, are also encompassed in the scope of the invention.

The present invention thus relates to a method (a) for diagnosing heart muscle tissue damage, preferably atrial heart muscle tissue damage, (b) for determining the extent of heart muscle tissue damage and/or (c) for differentiating between atrial heart muscle damage and ventricular heart muscle damage in a patient. In some embodiments, the respective methods can also cover the medical treatment that follows the diagnosis step. For example, if a patient is diagnosed with heart muscle tissue damage, said patient may be treated by administration of a drug, further medical or even surgical intervention.

The markers disclosed herein can be detected in body fluid samples, e.g., in a blood sample, and thus provide for an alternative or improved method for the diagnosis of heart muscle tissue damages. As such a method does not require expensive equipment, the costs for the diagnosis of heart muscle tissue damage, preferably atrial heart muscle tissue damage, can be reduced. Furthermore, the new method can be carried out by any trained medical personnel and therefore does not require the patient to travel to specific screening centers. This allows more frequent medical examinations. As mentioned in the introductory part, a further advantage of this method is that patients, for example, who have undergone treatment of atrial fibrillation by ablation, do not need to undergo the currently used invasive diagnosis in order, for example, to exclude a perioperative heart attack, which is a significant advantage of the methods of the present invention.

In a first aspect, the present invention relates to a method of diagnosing heart muscle tissue damage, preferably atrial heart muscle tissue damage, in a patient, wherein the method comprises determining the level of a biomarker for atrial heart muscle tissue damage selected from the group consisting of MYBPH (Uniprot Database entry: Q13203), MYBPHL (Myosin-binding protein H-like; Uniprot Database entry: A2RUH7), MYOT (Uniprot Database entry: Q9UBF9), ASNSD1 (Uniprot Database entry: Q9NWL6), CHFR (Uniprot Database entry: Q96EP1), NTN1 (Uniprot Database entry: 095631), RFC5 (Uniprot Database entry: P40937), POLI (Uniprot Database entry: Q9UNA4), COMP (Uniprot Database entry: P49747), POF1B (Uniprot Database entry: Q8WVV4), PLA2G2A (Uniprot Database entry: P14555), HDAC10 (Uniprot Database entry: Q969S8), ASPG (Uniprot Database entry: Q86U10), FMOD (Uniprot Database entry: Q06828), CA13 (Uniprot Database entry: Q8N1Q1), CACNA2D2 (Uniprot Database entry: Q9NY47), GNL3L (Uniprot Database entry: Q9NVN8), COL2A1 (Uniprot Database entry: P02458), PDLIM4 (Uniprot Database entry: P50479), LEPREL1 (Uniprot Database entry: Q8IVL5), OMD (Uniprot Database entry: Q99983), DGKZ (Uniprot Database entry: Q13574), NT5DC2 (Uniprot Database entry: Q9H857), ITLN1 (Uniprot Database entry: Q8WWA0), NTM (Uniprot Database entry: Q9P121), PRKG2 (Uniprot Database entry: Q13237), CHGB (Uniprot Database entry: P05060), FAM179A (Uniprot Database entry: Q6ZUX3), CKMT1A / CKMT1B (Uniprot Database entry: P12532), LTBP3 (Uniprot Database entry: Q9NS15), SGSM1 (Uniprot Database entry: Q2NKQ1), METTL7B (Uniprot Database entry: Q6UX53), LTBP2 (Uniprot Database entry: Q14767), OGDHL (Uniprot Database entry: Q9ULD0), PAM (Uniprot Database entry: P19021), SBK3 (Uniprot Database entry: P0C264), SFRP1 (Uniprot Database entry: Q8N474) and/or isoforms thereof, in particular the isoforms that are listed under the given database entries with the ones listed herein by reference to their amino acid sequence being particular embodiments, in a sample obtained from the patient and comparing the level of the biomarker for atrial heart muscle tissue damage determined in the patient with the level of the biomarker for atrial heart muscle tissue damage observed in a control individual, wherein if the level of the biomarker for atrial heart muscle tissue damage determined in the patient is increased compared to the level of the biomarker for atrial heart muscle tissue damage in the control individual, said patient is diagnosed with a heart muscle tissue damage, for example atrial heart muscle tissue damage.

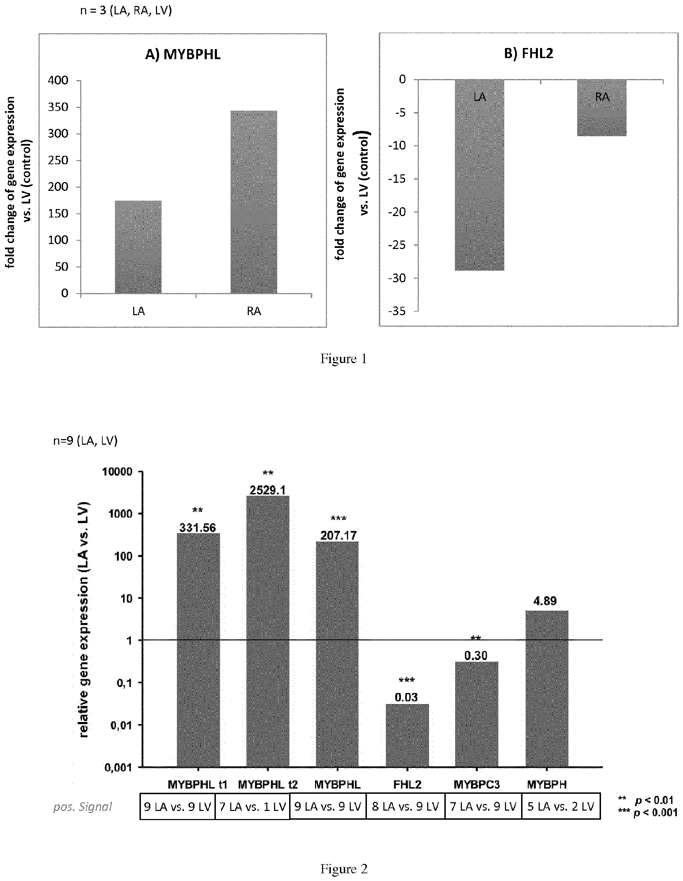

The inventors have identified Myosin-binding protein H-like (MBPHL= MYBPHL) as a biomarker for heart muscle tissue damage, for example as a specific biomarker for atrial heart muscle tissue damages (see example 1 and Figure 1). As shown in Figure 1 and Figure 2, MYBPHL gene is expressed in both the heart atrium and in the heart ventricle. However, the gene expression of MYBPHL in the atrium is clearly higher as in the ventricle, namely 150-fold higher in the left atrium and a 350-fold higher in the right atrium compared to the left ventricle. This means that, although an increase of the protein level of MYBPHL is detected, for example in the blood plasma of a patient, in the occurrence of a heart muscle tissue damage, the protein level of MYBPHL increases significantly more in the case the patient suffers from atrial heart muscle tissue damage as when the patient suffers from ventricular heart muscle tissue damage. Thus, MYBPHL can be used as specific biomarker for atrial heart muscle tissue damage.

In various embodiments, MYBPHL or an isoform thereof is used as a first biomarker for atrial heart muscle tissue damage. Said marker can be combined with any one or more of the other biomarkers for atrial heart muscle tissue damage, as disclosed above. In various embodiments, it can be combined with MYBPH or an isoform thereof. Accordingly, in various embodiments of the invention, one or more, such as 1, 2, 3, 4, 5, 6, 7, 8, 9, 10 or more, such as 20, 30 or all 37 of the above-listed biomarkers for atrial heart muscle tissue damage can be used. The can be combined with any one of more of the further biomarkers for general heart muscle damage, disclosed above.

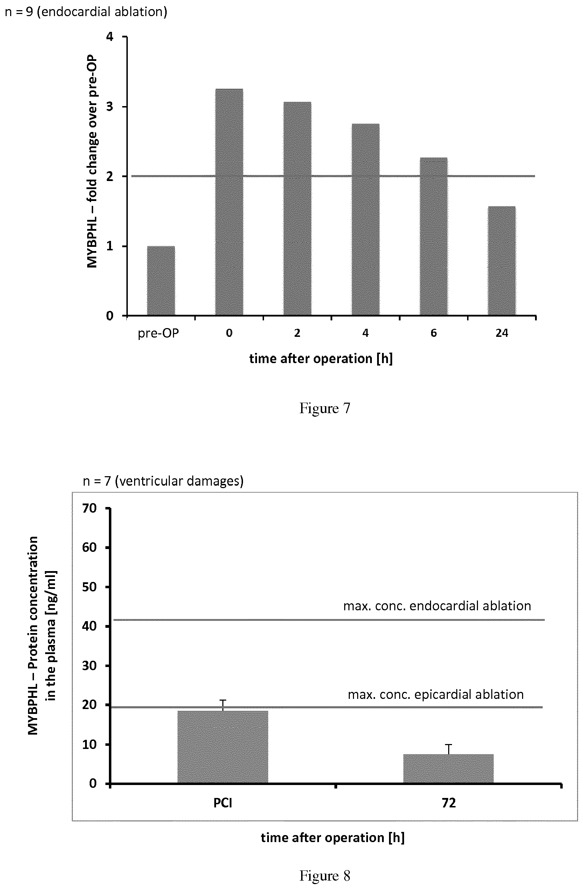

Figure 8 shows the results of the measurement of MYBPHL protein concentration in a blood plasma sample taken from a group of patients suffering from ST-elevation myocardial infarction, e.g. patients with a definitive damage in the ventricle, before and after a surgical intervention to treat the ST-elevation myocardial infarction. Large ST elevated myocardial infarctions leads to combined damages in the ventricle and the atrium. Figure 8 shows that the MYBPHL protein concentration, before the surgical intervention, is increased due to the ventricular damage and the atrial damage. 72 hours after the intervention to treat the ST-elevation myocardial infarction, the MYBPHL protein concentration is reduced. Thus, the results of the experiment showed in Figure 8 confirm the suitability of the biomarker MYBPHL as biomarker for heart muscle tissue damage.

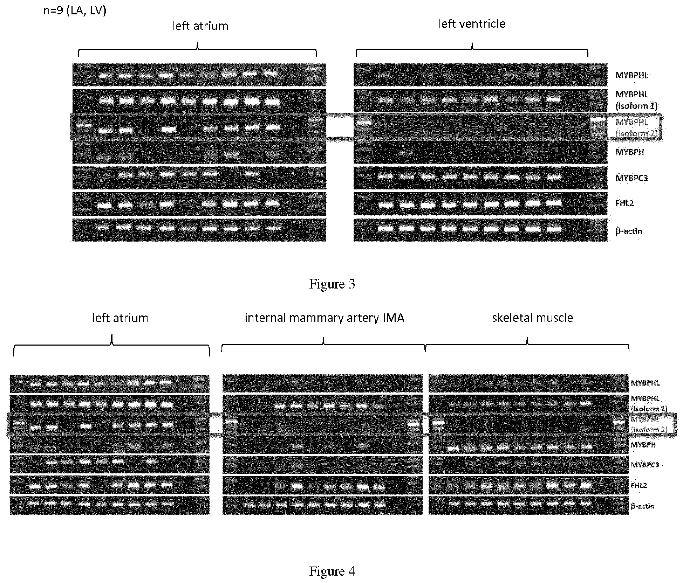

In addition, the inventors have identified Myosin-binding protein H (MBPH =MYBPH), and isoforms of MYBPHL, e.g. MYBPHL isoform 1 and MYBPHL isoform 2, as additional suitable biomarkers for the diagnosis of atrial heart muscle tissue damages. Figure 2 shows that, although MYBPHL isoform 1 gene is expressed in the 9 left studied atria as well as in the 9 left studied ventricles, MYBPHL isoform 1 gene expression is around 210-fold higher in the atrium as in the ventricle. MYBPH gene and MYBPHL isoform 2 gene are mostly expressed in the left atria (5 left atria versus 2 left ventricles for MYBPH and 7 left atria versus 1 left ventricle for MYBPHL isoform 2). Figure 3, which shows the separation of the amplification products of the biomarker MYBPHL isoform 2 from left atria and from left ventricles, confirms this observation. Hence the biomarkers MYBPH and MYBPHL isoform 2 are highly specific biomarkers for atrial heart muscle tissue damages. Figure 4 shows the separation of the amplification products of the biomarker MYBPHL isoform 2 in non-cardiac tissues. In Figure 4, MYBPHL isoform 2 was not observed in non-cardiac tissues like arterial vessels containing smooth muscle cells and skeletal muscle samples, confirming the specificity of MYBPLH isoform 2 for heart muscle, specifically for the left atrium, is shown. In contrast MYBPH showed an enriched expression in skeletal muscle.

The level of the biomarker for atrial heart muscle tissue damage observed in a control individual is the level of the biomarker determined in a normal healthy individual or in a control patient. A normal healthy individual is a person for whom no or insignificant heart problems have been observed. A control patient may be a patient suffering from a heart condition, for example about to undergo a surgery like a coronary artery bypass grafting or a valve surgery, and on which a MAZE procedure consisting of an ablation of the atrium, i.e. atrial heart muscle tissue damage, will be performed during the surgery. A control patient may also be a patient suffering from a heart defect and who had undergone an isolated aortic valve replacement by means of a heart-lung machine, since such a patient usually do not show alteration in the atrium. Furthermore, the control patient may be a patient who had undergone a catheter-based implantation of a heart valve without the use of a heart-lung machine.

An increased level of the biomarker in the patient means that its concentration is increased relative to the control individual. This term includes that, in the normal healthy individual, the biomarker is detectable at a concentration that remains stable or nearly stable over time while the biomarker concentration in a patient having heart muscle tissue damage varies and is higher than the biomarker concentration measured in the normal healthy individual. It is also possible to define a ratio, also called fold-change, between the biomarker level in the patient and the biomarker level in the control individual, wherein, when the ratio is in a range from 1.5 to 40, e.g. at least 2, 3, 4, 5, 6, 10, 12, 15, 20, 30, the level of the biomarker in the patient is defined as increased compared to the level in the control individual. In various embodiments, an "increased level" refers to an at least 1.5-fold increase, preferably an at least 2-fold increase, relative to the level observed in a healthy individual.

In certain embodiments of the method described herein, the method comprises evaluating the extent of heart muscle tissue damage in the patient by determining the ratio (also called fold-change) between the level of the biomarker for atrial heart muscle tissue damage in the sample obtained from the patient and the level of the biomarker for atrial heart muscle tissue damage in a healthy individual.

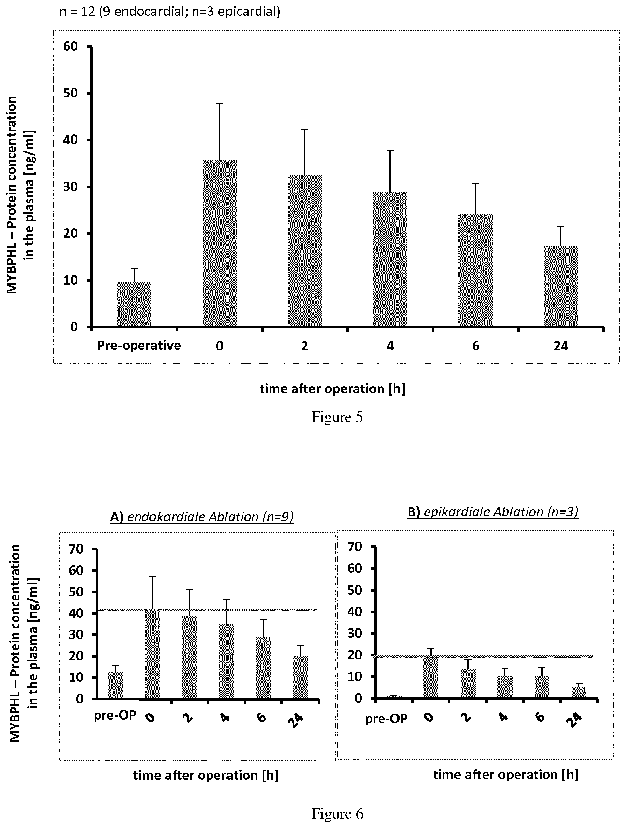

Figure 5 shows the evolution of the MYBPHL protein concentration in blood samples of patients undergoing MAZE procedure, an ablation of the atrium, i.e. atrial heart muscle tissue damage. As shown in Figure 5, the MYBPHL protein concentration is the highest MYBPHL protein concentration after the atrium ablation, namely at the time point of the arrival of the patient at the intensive care unit. The MYBPHL protein concentration decreases over time, for example within 24 hours after the ablation, as no further atrial heart muscle tissue damage is occurring. Also Figure 6 shows the evolution of the MYBPHL protein concentration in blood samples of patients undergoing endocardial ablation or epicardial ablation. Endocardial ablation is a more aggressive form of ablation compared to epicardial ablation. As shown in Figure 6, the MYBPHL protein concentration is higher in patients with endocardial ablation as in patients with epicardial ablation. Hence, as shown in the experiments illustrated in Figure 5 and Figure 6, the MYBPHL protein concentration and therefore its fold-change is proportional to the extent of the atrial heart muscle tissue damage. In other words, the bigger the heart muscle tissue damage is, the higher the MYBPHL level is. Furthermore, the biomarker MYBPHL has the advantage to be a sensitive biomarker, since the MYBPHL level is still detectable 24 hours after atrial ablation, whereas the known biomarker Myoglobin is not detected anymore in a sample of the patient after 24 hours.

In various embodiments of the method described herein, the method further comprises, in the sample of the patient, determining the level of a further biomarker for heart muscle tissue damage, wherein the method is suitable to distinguish between atrial muscle tissue damage and ventricular muscle tissue damage.

The differentiation between atrial muscle tissue damage and ventricular muscle tissue damage can be achieved by determining the level of a further biomarker for heart muscle tissue damage in the sample of the patient. The method may further comprise comparing the level of the further biomarker with the level of the biomarker specific for atrial heart muscle tissue damage, and/or comparing the level of the further biomarker in the sample of the patient with the level of the further biomarker in a sample obtained from a control individual to obtain the fold-change. The further biomarker for heart muscle tissue damage can be a general biomarker for heart muscle tissue damage selected from the group consisting of CK-MB or Troponin. Also, the further biomarker for heart muscle tissue damage can be a biomarker specific for ventricular heart muscle tissue damage. As shown in Figure 2, FHL-2 is a biomarker specific for ventricular heart muscle tissue damage, since expression levels of the FHL-2 gene are almost 50 times higher in the left ventricle compared to the left atrium.

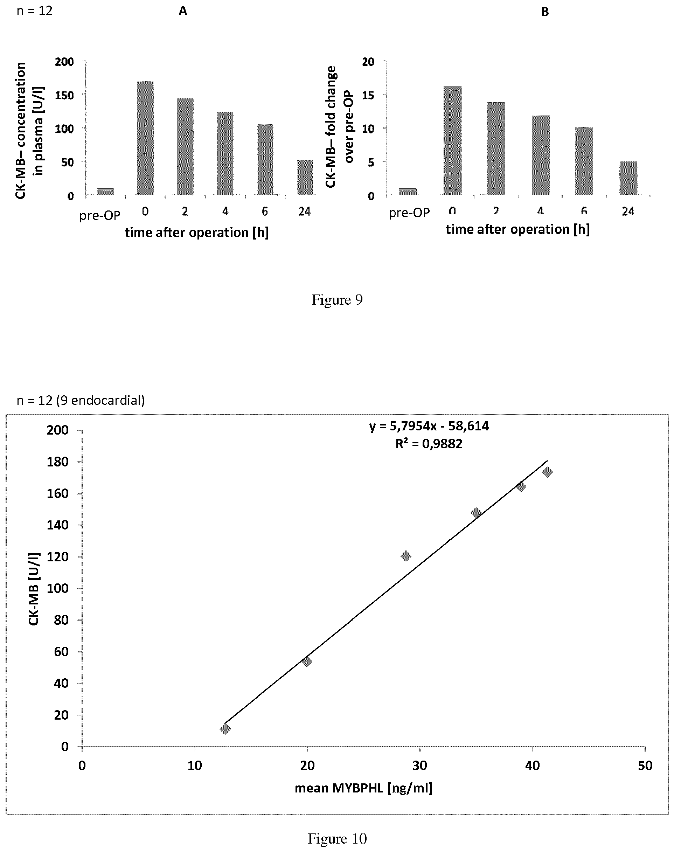

When the further biomarker for heart muscle tissue damage is a general biomarker for heart muscle tissue damage, preferably selected from the group consisting of CK-MB or Troponin, the ratio between the protein concentration of the general biomarker in the sample of the patient and the protein concentration of the biomarker for atrial heart muscle tissue damage may be calculated. Alternatively or additionally, the level of the biomarker for atrial heart muscle tissue damage and the level of the general biomarker for heart muscle tissue damage can be determined at periodical measurement time points over a defined period of time, for example every two hours, for example over 24 hours or 72 hours. If the level of the biomarker for atrial heart muscle tissue damage and the level of the general biomarker for heart muscle tissue damage both decrease over the defined period of time, for example proportionally to each other, the patient is diagnosed with an atrial heart muscle tissue damage (see example 5, Figure 9 and Figure 10). If the level of the biomarker for atrial heart muscle tissue damage decreases when the level of the general biomarker for heart muscle tissue damage remains stable or increases, the patient is diagnosed with ventricular muscle tissue damage.

When the further biomarker for a heart muscle tissue damage is a biomarker specific for ventricular heart muscle tissue damage, preferably FHL-2, the ratio between the fold-change of MYBPHL and the fold-change of FHL-2 is calculated, for example at periodical measurement time points over a defined period of time, for example every two hours, for example over 24 hours or 72 hours. When the ratio between the fold-change of MYBPHL and the fold-change of FHL-2 is above 1,5, for example in a range from 1,5 to 4, the patient is diagnosed with atrial heart muscle tissue damage (see example 6, Figure 10).

Also, the differentiation between atrial muscle tissue damage and ventricular muscle tissue damage can be achieved by measuring the level of the biomarkers very specific for atrial heart muscle tissue damages selected from the group consisting of MYBPH or MYBPHLt2 in a sample of the patient and comparing these levels with the level of these very specific biomarkers in a control individual. When the ratio between the level of the very specific biomarkers in the sample of the patient and the level of the very specific biomarkers in the sample of a control individual is in the range from 1,5 to 40, e.g. 2, 3, 4, 5, 6, 10, 12, 15, 20, 30, the patient is diagnosed with atrial heart muscle tissue damage.

In various embodiments of the invention, the sample is a biological sample, for example a body fluid, cell or tissue sample. Body fluids comprise, but are not limited to blood, blood plasma, blood serum, breast milk, cerebrospinal fluid, cerumen (earwax), endolymph and perilymph, gastric juice, mucus (including nasal drainage and phlegm), peritoneal fluid, pleural fluid, saliva, sebum (skin oil), semen, sweat, tears, vaginal secretion, nipple aspirate fluid, vomit and urine. In certain embodiments of the methods detailed above, the body fluid is selected from the group consisting of blood, serum, plasma, urine, and saliva. The tissue sample may be heart tissue and the cell sample may comprise cells from heart tissue.

In some embodiments the patient is a mammal, preferably a human.

Generally, the term "mammal", as used herein, comprises human, monkeys, pigs, cows, cats, dogs, guinea pigs, rabbits, mice, sheep, goats and horses.

For the detection of the markers of the present invention specific binding partners may be employed. In some embodiments, the specific binding partners are useful to detect the presence of a marker in a sample, wherein the marker is a protein or RNA. The marker and its binding partner represent a binding pair of molecules, which interact with each other through any of a variety of molecular forces including, for example, ionic, covalent, hydrophobic, van der Waals, and hydrogen bonding. Preferably, this binding is specific. "Specific binding" means that the members of a binding pair bind preferentially to each other, i.e. usually with a significant higher affinity than to non-specific binding partners. The binding affinity for specific binding partners is thus usually at least 10-fold, preferably at least 100-fold higher than that for non-specific binding partners.

Exemplary binding partners for the markers of the invention are selected from the group consisting of antibodies, antibody fragments and variants, molecules with antibody-like properties, such as lipocalin muteins or Spiegelmers or aptamers. Antibody fragments and variants include Fv fragments, linear single chain antibodies and the like all of which are known to those skilled in the art. Particularly preferred are antibodies.

In various embodiments of the methods detailed above, determining the level of a biomarker specific for atrial heart muscle tissue damage comprises determining the amount of protein of the biomarker present in a sample. In other embodiments, determining the level of a marker includes determining the expression level of a biomarker for atrial heart muscle tissue damage, for example on the RNA level. Generally, determining the level of a biomarker comprises determining the mRNA level and/or protein level of a biomarker.

Accordingly, in some embodiments the level of at least one or more biomarkers is determined on mRNA level. In further embodiments the level of at least one or more biomarkers is determined on protein level. In various embodiments, at least one or more markers are determined at mRNA level and at least one or more markers are determined at protein level.

If a marker is determined on mRNA level, the mRNA may be the mRNA transcript, a 5'- and/or 3'-truncated mRNA or spliced mRNA forms.

If a marker is determined on protein level, the protein may be the full length protein or a fragment thereof. The protein fragment may be a truncated protein, i.e. lack one or more amino acids, for example, at the N-terminus or C-terminus or both. This may be due to post-translational processing or due to the action of proteases present in the cell or the sample. The markers determined in the methods of the invention thus also include naturally occurring fragments, preferably immunogenic fragments. Also, the protein may be posttranslationally modified, e.g., phosphorylated, hydroxylated, glycosylated, N-glycosylated, O-glycosylated, ubiquitinylated, acetyated, methylated, prenylated or sulphated.

In certain embodiments of the methods of the invention, the levels of at least 2, 3, 4, 5, 6, 7, 8, 9, 10, 20, 50 or 100 or more biomarkers are determined.

In various embodiments of the method of the present invention, determining the level of a biomarker for atrial heart muscle tissue damage comprises determining the protein level of the biomarker for atrial heart muscle tissue damage in a sample.

In some of these embodiments, the level of the one or more biomarkers is determined at protein and/or mRNA level. Accordingly, in some embodiments the methods involve the determination of the level of the biomarkers on protein level only.

The methods detailed above, wherein the level of at least one or more markers is determined on protein level comprise in some embodiments the determination of the protein level by an immunoassay, mass spectrometry, chromatography, Western Blot, or gel electrophoresis.

In some embodiments, the immunoassay may be, but is not limited to an Enzyme-linked Immunosorbent Assay (ELISA), Western blot, agglutination test, biotin/avidin type assay, radioimmunoassay, immunoelectrophoresis and immunoprecipitation. The reactions generally include revealing labels such as fluorescent, chemiluminescent, radioactive, enzymatic labels or dye molecules, or other methods for detecting the formation of a complex between the antigen and the antibody or antibodies reacted therewith. These and further immunoassays are well known in the art (David Wild (Ed.): The Immunoassay Handbook. 3rd ed. Elsevier Science Publishing Company, Amsterdam 2005).

The aforementioned assays may involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

In certain embodiments of the above detailed methods, if the determination is via mass spectrometry, the mass spectrometry may be selected from the group comprising MS measurements using EI, CI, ESI, APLI, APPI and APCI.

The biomarker determination on protein level employing chromatography may be selected from the group comprising liquid chromatography, HPLC, FPLC, Smart chromatography, gel chromatography, size exclusion chromatography, reverse phase chromatography and ion-exchange chromatography (Introduction to Modern Liquid Chromatography, Lloyd R. Snyder, 5 Wiley, 2009).

In various embodiments, if the biomarker is detected via gel electrophoresis, the gel electrophoresis may be selected from the group, but not limited to agarose gel electrophoresis, sodium dodecyl sulfate poly acryl amide gel electrophoresis (SDS-PAGE), 2D-gel electrophoresis, native gel electrophoresis and quantitative preparative native continuous polyacrylamide gel 10 electrophoresis (QPNC-PAGE).

Of course, in certain embodiments of the methods of the present invention at least two determination methods may be coupled to each other in a subsequent manner. In a variant, gel electrophoresis may be followed by mass spectroscopic analysis. Alternatively, gel electrophoresis may be followed by Western Blot, chromatography may be followed by 15 mass spectroscopic analysis, chromatography may be followed by an immunoassay, e.g. ELISA.

In further embodiments of the methods of the present invention, determining the level of the biomarker for atrial heart muscle tissue damage comprises determining the mRNA level of the biomarker for atrial heart muscle tissue damage in a sample. In alternative variants of these embodiments, the level of one or more further biomarkers is determined. In some of these embodiments, the level of the one or more biomarkers is determined at protein and/or mRNA level. Accordingly, in some embodiments the methods involve the determination of the level of the biomarkers on RNA level only.

Where in the methods detailed above a biomarker is determined on mRNA level, the RNA level may be determined by PCR, gel electrophoresis and/or Northern Blot.

In case the biomarker level is determined on the RNA level, the detection reagent may be a nucleic acid molecule, such as an oligonucleotide. The oligonucleotide may be a nucleic acid probe that may be labeled to allow detection or may be an oligonucleotide primer that allows amplification of the target molecule.

In a further aspect, the present invention relates to a kit for use in a method as detailed above, wherein the kit comprises reagents for determining the level of one or more biomarker for atrial heart muscle tissue damage and reagents for determining the level of the further biomarker for heart muscle damage in a sample. In certain embodiments, the reagents for determining the level of a biomarker for atrial heart muscle tissue damage and of the further biomarker for heart muscle damage in a sample are antibodies and/or oligonucleotides. In some of the above embodiments, the kit comprises reagents for determining the level of a biomarkers for atrial heart muscle tissue damage and reagents for determining the level of the further biomarker for heart muscle damage in a sample. In one embodiment, the kit comprises further reagent for the detection of at least 1, 2, 3, 4, 5, 6, 7, 8, 9, 10, 20, 30, 40, 50 or 100 further biomarkers.

In addition, the present invention provides a biomarker panel comprising one or more biomarkers for atrial heart muscle tissue damage, as disclosed herein, and one or more further biomarkers for heart muscle damage, as disclosed herein, for use in the method of the invention. In certain embodiments, the panel comprises at least one or more further biomarkers. In one embodiment, the biomarker panel comprises at least 1, 2, 3, 4, 5, 6, 7, 8, 9, 10, 20, 30, 40, 50 or 100 further markers. These may be selected from those disclosed herein. Also encompassed are kits that comprise detection reagents for the markers included in the biomarker panel. Said detection reagents may, for example, be antibodies specific for the respective marker.

### Examples

### Example 1: Increased MYBPHL, MYBPH, MYBPHL isoform 1 and MYBPHL isoform 2 gene expression in atrial heart muscle tissues

A study of the human heart proteome was performed, for example according to "Region and cell-type resolved quantitative proteomic map of the human heart" Doll S, DreBen M, Geyer PE, Itzhak DN, Braun C, Doppler SA, Meier F, Deutsch MA, Lahm H, Lange R, Krane M, Mann M. Nat Commun. 2017 Nov 13;8(1):1469. doi: 10.1038/s41467-017-01747-2. PMID: 29133944. The expression of various biomarker in heart tissue samples obtained from patients who underwent heart surgery by means of biopsy has been validated by qPCR. In the study tissue samples were taken out from the following areas (wherein the number of tissue sample was n=3): left atrium (LA), right atrium (RA), left ventricle (LV) and right ventricle (RV).

Figure 1 shows the results of gene expression analysis of the biomarker MYBPHL and the biomarker FHL2, wherein the gene expression analysis has been performed by qPCR. Figure 1A shows a highly significant differential in the expression of MYBPHL in the left atrium and in the right atrium compared to the left ventricle. Thereby the gene expression of MYBPHL in the right atrium is 300 times higher than in the left ventricle. Figure 1B shows a significant differential in the expression of FHL2 in the left ventricle compared to the left atrium and the right atrium, wherein the gene expression of FHL 2 in the left ventricle is 28 times higher than in the left atrium.

Figure 2 shows the results of gene expression analysis of the biomarker MYBPHL, FHL-2, MYBPC3 and MYBPH as well as the MYBPHL isoforms MYBPHL isoform 1 (MYBPHL t1) and MYBPHL isoform 2 (MYBPHL t2) in tissues taken from 9 left ventricle muscle tissues and 9 left atrium muscle tissues obtained by means of a biopsy in a patient, wherein gene expression analysis has been performed by qPCR. Thereby gene expressions of MYBPHL, MYBPH and the two MYBPHL isoforms MYBPHL isoform 1 and MYBPHL isoform 2 are higher in the left atrium, wherein the differential expression of MYBPH is clearly lower than the one of MYBPHL (almost 42 times lower). FHL-2 is a gene, which is expressed at a higher level in the left ventricle. Furthermore MYBPHL isoform 2 shows a high specificity for the left atrium since MYBPHL isoform 2 is expressed only in 1 left ventricle sample out of 9 left ventricle samples, whereas MYBPHL isoform 2 is expressed in 7 left atrium samples out of 9 left atrium samples.

Figure 3 shows the results of gene expression analysis of the biomarker MYBPHL isoform 2, wherein gene expression analysis has been shown by gel electrophoresis. Thereby the high specificity of MYBPHL isoform 2 for the left atrium is confirmed, wherein MYBPHL isoform 2 has been detected in none of left ventricle samples, whereas MYBPHL isoform 2 has been detected in 7 left atrium samples out of 9 left atrium samples.

Figure 4 shows the expression level of MYBPHL isoform 2 in the left atrium as well as in non-cardiac tissues. Figure 4 shows that MYBPHL isoform 2 has been detected in none of the arterial vessels containing smooth muscle cells and skeletal muscle samples, whereas MYBPHL isoform 2 has been detected in the left atrium, confirming thereby the high cardiac specificity of MYBPHL isoform 2.

### Example 2: Detection of the biomarker MYBPHL in blood plasma

A study was performed to determine if the biomarker MYBPHL could be detected in blood plasma. A patient group who has undergone an ablation (modified MAZE procedure) for the treatment of atrial fibrillation besides heart surgery like coronary artery bypass grafting or valve surgery has been studied. In the MAZE procedure, focused and intended specific heart muscle damage has been performed either in endocardial heart muscle tissue or in epicardial heart muscle tissue of the patient. Over a time period of 24 hours at the following measurement time points, a blood sample has been taken out from the patient:

| | | |
|---|---|---|
| 1. | Pre-OP | before the beginning of the heart surgery |
| 2. | 0h | arrival of the patient in the intensive care unit |
| 3. | 2h | 2 hours after arrival of the patient in the intensive care unit |
| 4. | 4h | 4 hours after arrival of the patient in the intensive care unit |
| 5. | 6h | 6 hours after arrival of the patient in the intensive care unit |
| 6. | 24h | 24 hours after arrival of the patient in the intensive care unit |

Figure 5 shows the results of the measurement of MYBPHL protein concentration in the blood plasma samples obtained from the patient group of 12 patients (n = 12), who has undergone endocardial and epicardial ablation. The concentration of MYBPHL in the plasma sample increased clearly and significantly already at the measurement time point 2. At the measurement time point 2, the maximum of MYBPHL plasma concentration is reached. Over the time, the MYBPHL plasma concentration decreased gradually, wherein the MYBPHL plasma concentration at measurement time point 6, namely 24 hours after the arrival of the patient in the intensive care unit, still not have reached the preoperative concentration level. The data show that MYBPHL compared to Myoglobin, an unspecific biomarker know in the art, allows the detection in blood plasma of the occurrence of heart muscle damage even after more than 24 hours after its occurrence.

### Example 3: damage extent dependence with the concentration of the biomarker MYBPHL in blood plasma

Figure 6 shows the results of the measurement of MYBPHL protein concentration in a blood plasma sample over a defined period of time and at measurement time points as described in Example 2 in patients having undergone either endocardial ablation (A) or epicardial ablation (B). In this study a group of 9 patients (n = 9) underwent endocardial ablation (A) and a group of 3 patients (n = 3) underwent epicardial ablation (B). Endocardial ablation is a more aggressive form of ablation as epicardial one and leads to stronger heart muscle damage. The postoperative values show for both groups of patients with endocardial ablation and epicardial ablation a significant increase of MYBPHL protein concentration in the blood plasma, which decreases over time. However, for patients who received epicardial ablation, the MYBPHL protein concentration in the blood plasma never reached a higher level than the MYBPHL protein concentration at all postoperative measurement times in the blood plasma of patients who received endocardial ablation. This shows the sensitivity and the correlation between the MYBPHL protein concentration and the present atrial heart muscle damage, since the MYBPHL protein concentration is higher by patients having received the more aggressive endocardial ablation.

Figure 7 shows the fold-change in the MYBPHL protein concentration in a blood plasma sample according to Figure 6. For the case of endocardial ablation, the preoperative MYBPHL plasma concentration has been arbitrary set at 1. In the case of endocardial ablation, a maximum of 3-fold MYBPHL concentration increase has been measured. The maximum corresponds to the measurement time point of the patient's arrival in the intensive care unit. On the other hand, in the case of epicardial ablation, a maximum of 20 fold MYBPHL concentration increase has been measured, wherein the intensity of the atrial heart muscle damage is lower, as described in Fig.5.

### Example 4: MYBPHL plasma concentration in patients suffering from an acute ST-elevation myocardial infarction

The MYBPHL protein concentration was measured in a patient group that presented acute ST-elevation myocardial infarction. Figure 8 shows the results of the measurement of MYBPHL protein concentration in blood plasma samples. Blood samples were taken at two measurement time points, one before intervention and one 72 hours after the intervention. The intervention was performed by means of a heart catheter. Due to the pathology of acute ST-elevation myocardial infarction, the patients of this group suffered from a definitive ventricle damage along with a atrial cardiac damage as shown in Figure 8, where the highest MYBPHL plasma concentration is found at the measurement time point 1, e.g. before the intervention. This MYBPHL plasma concentration is similar to the MYBPHL plasma concentration found in patients treated with an epicardial MAZE. 72 hours after the intervention, the MYBPHL plasma concentration decreased to values similar to the ones obtained in example 3 before patients were treated with MAZE (figure 5 and figure 6a) or in healthy subjects. The data of this study show that the MYBPHL plasma concentration highly increases in the case of an acute heart muscle damage and decreases over time to reach normal values when the pathology is not anymore present.

### Example 5: proportionality between the protein concentration of the specific biomarker MYBPHL and the protein concentration of the known unspecific biomarker CK-MB in the case of atrial heart muscle tissue damage

Figure 9A shows the results of the measurement of CK-MB protein concentration in blood serum samples obtained from the same patient group as in example 2. Figure 9B shows the fold-change in the CK-MB protein concentration in the blood serum sample according to Figure 9A. Compared to the MYBPHL, the CK-MB protein concentration reached its maximum value also at the measurement time point 2. At this measurement time point 2, the CK-MB protein concentration is 15 times higher as the CK-MB protein concentration before the ablation.

Figure 10 shows a correlation between the MYBPHL concentration values in a blood plasma sample of 12 patients who underwent an ablation and the CK-MB concentration values in a blood serum sample of the same patients at the same measurement times. CK-MB, although unspecific for atrial muscle damage or for ventricular muscle damage, is an established biomarker for the detection of heart muscle damage, wherein its concentration is known as being correlated with the extent of the heart muscle damage. As shown in Figure 10 a 1-to-1 correlation could be proved between MYBPHL concentration values and CK-MB concentration values.

### Example 6: differentiation between atrial heart muscle tissue damage and ventricular heart muscle tissue damage using the fold-change of the biomarker MYBPHL and the fold-change of the biomarker FHL-2

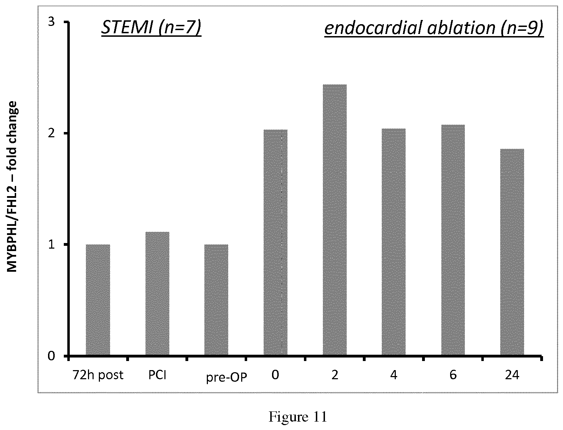

Figure 11 shows a representation of the ratio between the fold-change of MYBPHL and the fold-change of FHL-2. On the two left columns of the representation, the results for patients suffering from ST-elevation myocardial infarction are shown, while on the six right columns of the representation, the results for patients suffering from atrial fibrillation and undergoing an ablation are shown. The ratio between the fold-change of MYBPHL and the fold-change of FHL-2 increases up to 2 for patients having undergone atrial ablation. On the contrary the ratio between the fold-change of MYBPHL and the fold-change of FHL-2 remains stable at about 1 before and after the intervention for patients suffering from ST-elevation myocardial infarction, e.g. ventricular heart muscle tissue damage.

All documents cited herein, are hereby incorporated by reference in their entirety.

The inventions illustratively described herein may suitably be practiced in the absence of any element or elements, limitation or limitations, not specifically disclosed herein. Thus, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

## Claims

1. A method of diagnosing heart muscle tissue damage, preferably atrial heart muscle tissue damage, in a patient, wherein the method comprises:
(a) determining the level of a biomarker for atrial heart muscle tissue damage selected from the group consisting of MYBPH, MYBPHL, MYOT, ASNSD1, CHFR, NTN1, RFC5, POLI, COMP, POF1B, PLA2G2A, HDAC10, ASPG, FMOD, CA13, CACNA2D2, GNL3L, COL2A1, PDLIM4, LEPREL1, OMD, DGKZ, NT5DC2, ITLN1, NTM, PRKG2, CHGB, FAM179A, CKMT1A, CKMT1B, LTBP3, SGSM1, METTL7B, LTBP2, OGDHL, PAM, SBK3, SFRP1 and/or isoforms thereof in a sample obtained from the patient; and
(b) comparing the level determined in step a) with the level of the biomarker for atrial heart muscle tissue damage observed in a control individual, wherein if the level determined in step a) is increased compared to the level of the biomarker for atrial heart muscle tissue damage in the control individual, said patient is diagnosed with a heart muscle tissue damage.

2. The method of claim 1, wherein the isoforms of MYBPHL are selected from the group consisting of MYBPHL isoform 1 and MYBPHL isoform 2.

3. The method of claim 1 or 2, wherein the extent of the heart muscle tissue damage in said patient is evaluated by determining the ratio between the level of the biomarker for atrial heart muscle tissue damage in the sample obtained from said patient and the level of the biomarker for atrial heart muscle tissue damage in a control individual.

4. The method of any one of claims 1 to 3, wherein the method further comprises:
(c) in the sample of said patient, determining the level of a further biomarker for a heart muscle tissue damage,
wherein the method is suitable to differentiate between atrial muscle tissue damage and ventricular muscle tissue damage.

5. The method of claim 4, wherein the further biomarker is a biomarker specific for ventricular heart muscle tissue damage, preferably FHL-2, SMYD2, FASTKD1, PRR33, SORBS2, CRISPLD1, NPHP4, ANKRD2, TMEM159, ATP7A, C12ORF73, NAV1, ATP5B, HSPB7, TMEM88, WDR62, ACTN3, TRIM72, EGFLAM, LRRC39, DPYSL4, PFKFB2, ABCB6, METTL2B, METTL2A, CARNS1, SPTBN1 or isoforms thereof.

6. The method of any one of claims 1 to 4, wherein the further biomarker is a general biomarker for heart muscle tissue damage, preferably selected from the group consisting of CK-MB or Troponin,

7. The method of any one of claims 1 to 6, wherein the sample is a blood serum or a blood plasma.

8. The method of any one of claims 1 to 7, wherein the patient is a human.

9. The method of any one of claims 1 to 8, wherein determining the level of the biomarker comprises determining the protein concentration of the biomarker.

10. The method of claim 9, wherein the protein concentration is determined by an immunoassay, ELISA, mass spectrometry, chromatography, Western Blot, or gel electrophoresis.

11. A kit for use in a method according to any one of claims 4-10, wherein the kit comprises reagents for determining the level of the biomarker for atrial heart muscle tissue damage and reagents for determining the level of the further biomarker for heart muscle damage in a sample.

12. A marker panel comprising a biomarker for atrial heart muscle tissue damage and a further biomarker for heart muscle damage for use in a method according to any one of claims 4-10.
